# EUROPEAN PATENT APPLICATION

(11) **EP 1 233 076 A2**
(43) Date of publication of application: **21.08.2002**
(21) Application number: 02447026.2
(22) Date of filing: 15.02.2002
(51) Int. Cl.: C12Q 1/68

(54) **Differential diagnosis for mycobacterial and pseudomonas species using species-specific upstream p34 gene region probes**

(30) Priority: 19.02.2001 EP 01870030; 21.02.2001 US 269848; 23.05.2001 US 292509
(71) Applicant: UNIVERSITE CATHOLIQUE DE LOUVAIN, 1348 Louvain la Neuve (BE)
(72) Inventor: Gala, Jean-Luc, 1932 St.-Stevens-Woluwe (BE); Vannuffel, Pascal, 7133 Buvrinnes (BE)
(74) Representative: De Clercq, Ann

(57) **Abstract**

The present invention relates to methods and devices for detecting and differentiating between Mycobacterium strains in a sample based upon species-specific upstream p34 gene region (us-p34) sequences. New us-p34 sequences and probes and primers derived therefrom are provided as well as methods and diagnostic kits based on the same. The invention also relates to methods and devices for detecting and differentiating between Pseudomonas strains in a sample based upon species-specific rRNA opreon (*rrn*) sequences. New *rrn* sequences and probes and primers derived therefrom are provided as well as methods and diagnostic kits based on the same.

## Description

### FIELD OF THE INVENTION.

The present invention refers to new genetic sequences, diagnostic and/or quantification methods and devices using said sequences for the identification of various types of Mycobacterium and Pseudomonas strains

### BACKGROUND TO THE INVENTION.

### Classification

The mycobacterial diseases may be divided in three categories: tuberculosis which is caused either by *M. tuberculosis* or *M. bovis,* both belonging to the *M. tuberculosis*-complex (TUB), leprosy caused by *M. leprae,* and diseases caused by nontuberculous mycobacteria (NTM), which cause all mycobacterial diseases other than tuberculosis and leprosy. Historically, tuberculosis and leprosy are the two preponderant human mycobacterial diseases. In recent years however, NTM have become more frequent in developed countries, partly because of the development of Acquired Immunodeficiency Syndrome (AIDS) (1). Tuberculosis occurs only in humans and animals, no other reservoir has been found to date. Conversely, the reservoir of mycobacteria responsible for NTM disease is mainly environmental and the majority of these mycobacteria are naturally resistant to known antimycobacterial drugs, making eradication unfeasible. Identification of mycobacteria species by rapid and specific methods is now mandatory. In particular, there is a need for rapid differentiation of strict human and animal pathogenic mycobacteria (such as, for example, *M. tuberculosis-complex, M. paratuberculosis, M. leprae)* from potentially pathogenic mycobacteria (such as, for example, *M. avium-complex; M. chelonae, M. kansasii, M. xenopi, M. simiae, M. malmoense)* and from normally saprophytic species (such as, for example, *M. gordonae; M. terrae; M. nonchromogenicum; M. flavescens; M. gastri; M. smegmatis).* Except for the strict pathogens, the majority of mycobacteria can indeed be found everywhere in the nature (soil, fresh and seawater) and can colonise temporary or permanently the human respiratory or digestive tract after ingestion or inhalation.
a) Members of the tuberculosis-complex (TUB):
   - This group comprises *M. tuberculosis, M. bovis, M. africanum, M. microti.*
b) Non tuberculous mycobacteria (NTM) :
   - Members of the M. avium-complex (MAC-complex) includes several environmental species that resemble M. avium and M. intracellulare or that have intermediate characteristics common to these *species* (*M. avium; M. intracellulare; M. paratuberculosis, M. scrofulaceum).*
   - Mycobacteria other than TUB (also named MOTT) and not belonging to the MAC-complex (such as, for example, *M. malmoense, M. sulzgai, M. kansasii, M. xenopi, M. chelonae, M. simiae, M. marinum, M. gordonae, M. fortuitum*).

### Epidemiology

Tuberculosis infects one-third of the world's population and kills more than 3 million of people each year. Tuberculosis, caused by mycobacteria of the M. tuberculosis group (TUB), is still highly endemic in large parts of the world and also the potential for introduction into and further transmission within countries where it has become rare (2). Tuberculosis has indeed re-emerged in many industrialised nations in recent years as a results of AIDS and changing population dynamics characterised by increased numbers of migrant workers, immigrants, and homeless people (3). Of particular concern is the spread of drug-resistant strains (4). Cases of tuberculosis are more and more often difficult to treat because of the increasing prevalence of TUB strains that are resistant to all front-line antituberculous drugs. In HIV-1 infected people, TUB is the most common opportunistic bacterial infections. In advanced stages of AIDS, mycobacterial infections due to members of the M. avium-intracellulare complex (MAC) are the most common systemic bacterial opportunistic infection (1). Moreover, reduced and compromised immune function as found in new-borns, infants, and immune-suppressed individuals allows opportunistic infections caused by mycobacteria other than M. tuberculosis (NTM) including *M. avium-intracellulare, M. chelonae, M. fortuitum, M. kansasii, M. xenopi, M. marinum, M. ulcerans, M. scrofulaceum,* and *M. szulgai.*

The increasing number of mycobacterial infections has made it clinically important to quickly identify mycobacteria at the species level. The diagnosis of a pathogenic versus a non-pathogenic species not only has epidemiological implications but is also relevant to the demands of patient management. Individuals with highly contagious infections may be isolated to prevent the spread of the disease. As a matter of fact, antibiotic treatments may vary according to the species encountered. Today, the number of non-tuberculous infections is difficult to assess because there is no system for notification as it exists *for M. tuberculosis.* The currently reported frequency of these species is likely to be an underestimation due to the lack of additional testing in cases of minimal disease or misidentification as *M. tuberculosis.* Despite the likely underestimation of NTM diseases, a growing number of NTM isolates are submitted to laboratories for identification. This may reflect an increase in the prevalence of opportunistic mycobacterial disease (notably in the AIDS context), or it may also reflect an increase in the number and nature of specimens submitted for culture brought about by a greater awareness of tuberculosis.

The ability to detect and identify these mycobacterial diseases in an early stage would potentially decrease morbidity and mortality and lessen its socio-economic impact. The «gold standard» for definite diagnosis has remained culturing the mycobacterium combined with phenotypic tests (for example, the analysis of fatty acid and mycolic acid contents). However, traditional culture methods for human specimens can require up to 8 weeks (this varies according to the mycobacterial species). For instances, primary culture of *M. ulcerans* under optimal conditions may take several months (5).

Other methods based on molecular biology technologies have allowed the development of genetic tests for the identification of some mycobacterial species. In the last decade, the use of restriction fragment length polymorphism (RFLP) analysis, oligotyping, and nucleic acid probes have been proposed as a mean for detecting and definitively identifying infectious agents (6). These tests are highly specific and rapid when compared to conventional culture method and have been applied to a wide variety of pathogens, especially to fastidious micro-organisms like mycobacteria. To date, the design of molecular tests has speeded up the diagnosis of this fastidious genus but still suffers some drawbacks. For species identification, a highly polymorphic region of the 16S rRNA gene has been shown to contain species-specific polymorphisms. This region is currently used in several commercially available assays, applicable either directly on a specimen (Accu-Probe; Gen-Probe) or after enzymatic amplification of the target for improved sensitivity (AMTD; Gen-Probe; Amplicor MTB, Roche). However, these commercial kits are mostly designed for the diagnosis of the most common disease-causing mycobacterial species, i.e. *M. tuberculosis* and MAC strains, and each kit has been designed to identify only one mycobacterial species per test which makes it a very expensive and limitative way of identifying mycobacterial species.

Coetsier et al. (2000) described a method for differentiating between groups of mycobacteria strains based on amplification of the us-p34 region using universal probes and analyzing the length of the amplified products which allowed discrimination between tuberculosis or Non-tuberculosis mycobacteria. Further species-specific discrimination was based upon species-specific f25 genomic sequences.

### SUMMARY OF THE INVENTION

The newly identified and characterised sequences described in the present invention enable the design of differential diagnosis strategies. These diagnosis strategies are able to identify, in one single assay, a wide range of mycobacterial species that include TUB and NTM. In addition, within the NTM group, it is even possible to differentiate between the members of the MAC-complex. Moreover, these molecular targets open new perspectives for the quantification of mycobacteria strains in clinical samples.

The present invention aims to provide new genetic sequences, methods, kits and devices for the improvement of the identification and/or the quantification of various types of mycobacteria species through their upstream-p34 (us-p34) determinants, which allow by a rapid molecular screening, their epidemiological study as well as their rapid characterisation in clinical human, animal and/or environmental samples.

Another aim of the invention is to identify similar genetic sequences which may exist in known and yet unknown Mycobacteria species.

The present invention also aims to provide new genetic sequences, methods, kits and devices for the improvement of the identification and/or the quantification of various types of Pseudomonas species through their rRNA operon (*rrn*) determinants, which allow by a rapid molecular screening and characterisation in clinical human, animal and/or environmental samples.

The nucleic acids of the invention can be used as primers for amplification or as probes for hybridization or for producing biochips or micro-arrays.

### DETAILED DESCRIPTION OF THE INVENTION

The present inventors for the first time recognised, by sequencing the upstream part of the p34 gene, species-specific differences in the sequences between various mycobacterial strains, allowing determination of the presence and identification of specific Mycobacterium strains or species in various samples.

Therefore, according to a first embodiment the present invention relates to a method for detecting at least one non-tuberulosis Mycobacterium (NTM) strain in a sample, comprising:
(i) providing at least one non-tuberulosis Mycobacterium species-specific upstream p34 gene region (us-p34) nucleotide probe,
(ii) reacting said us-p34 nucleotide probe with said sample under conditions that allow for the selective formation of nucleotide duplexes between said us-p34 nucleotide probe and a corresponding non-tuberulosis Mycobacterium nucleic acid target present in said sample, and,
(iii) detecting any nucleotide duplexes containing said us-p34 nucleotide probe.

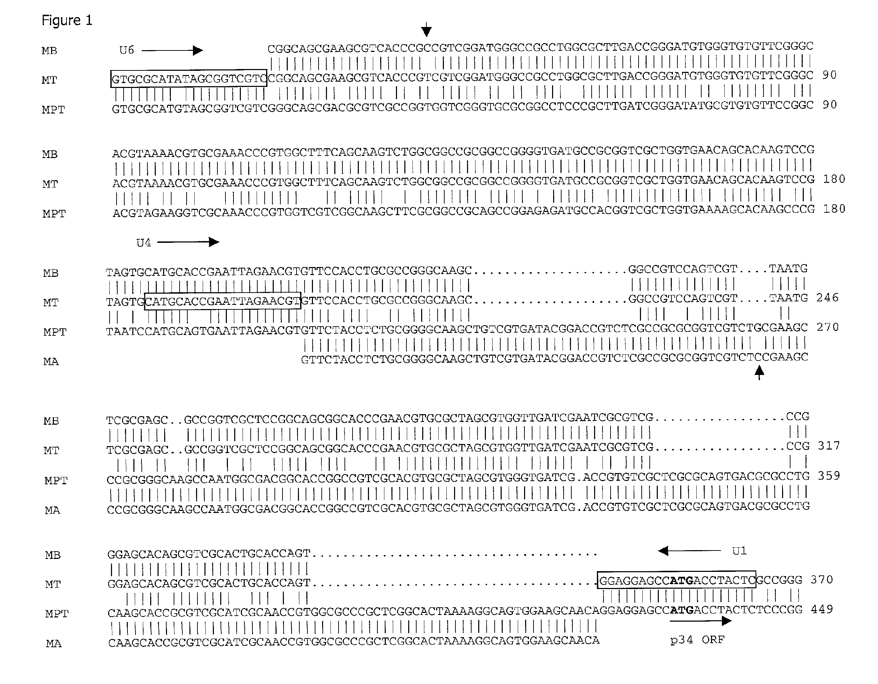

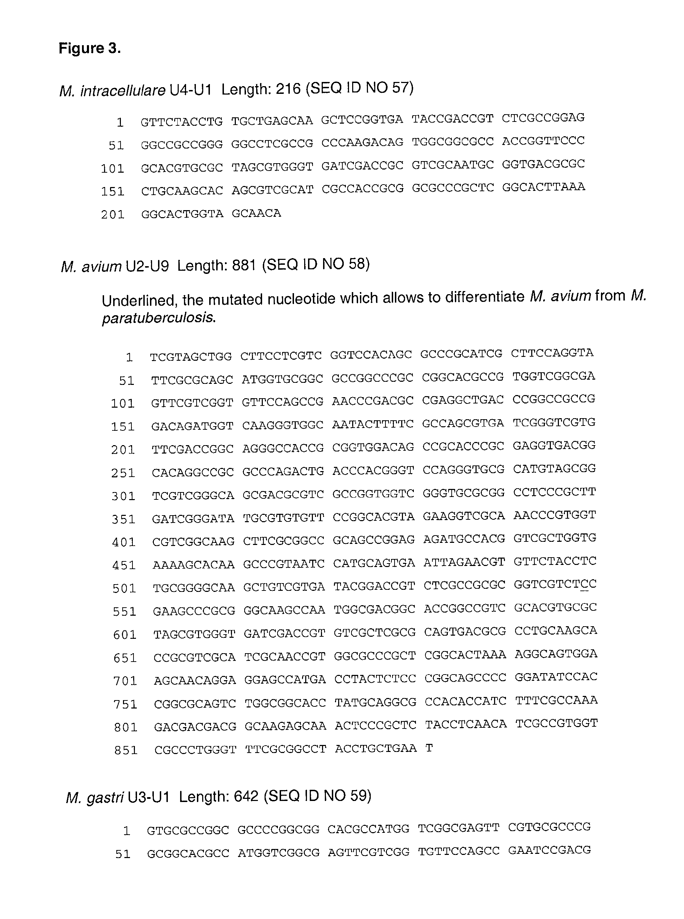

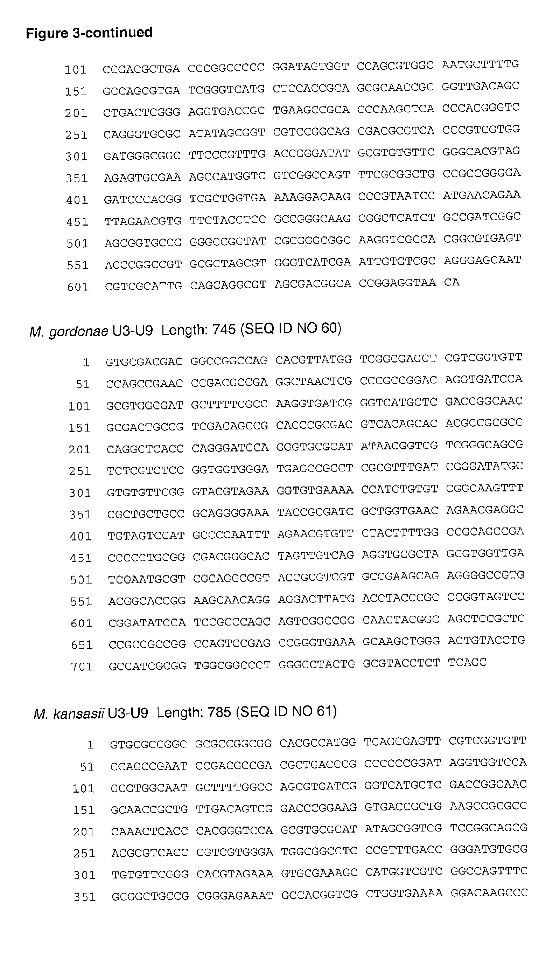

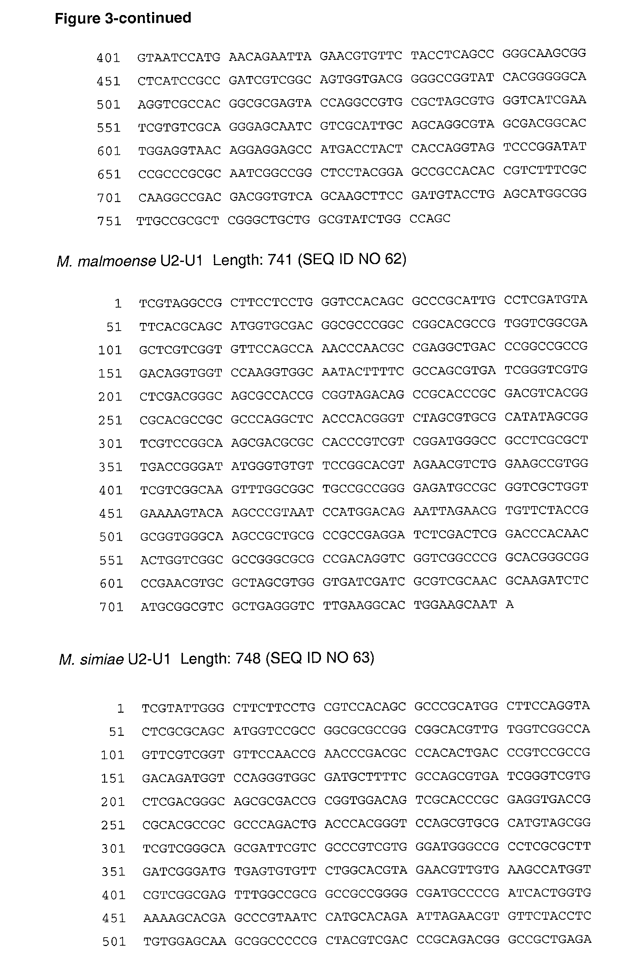

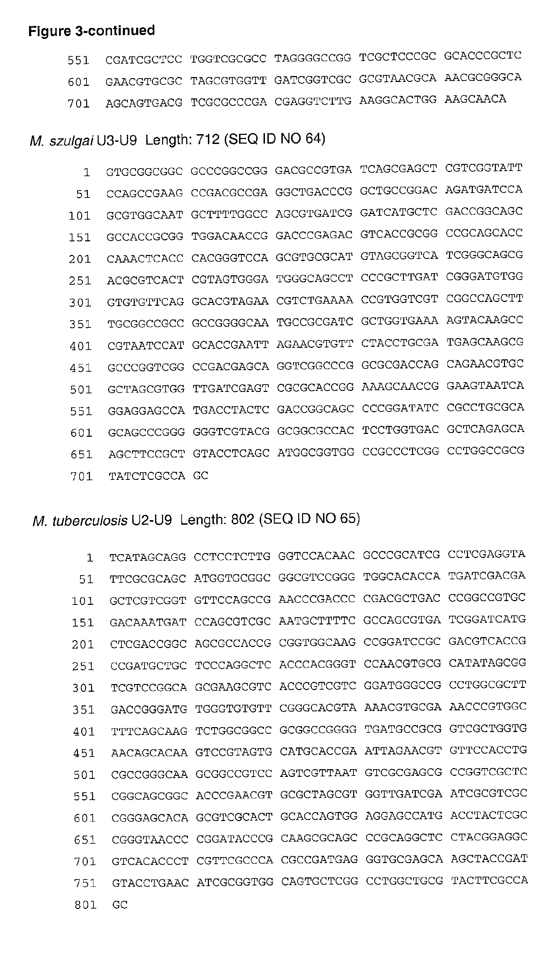

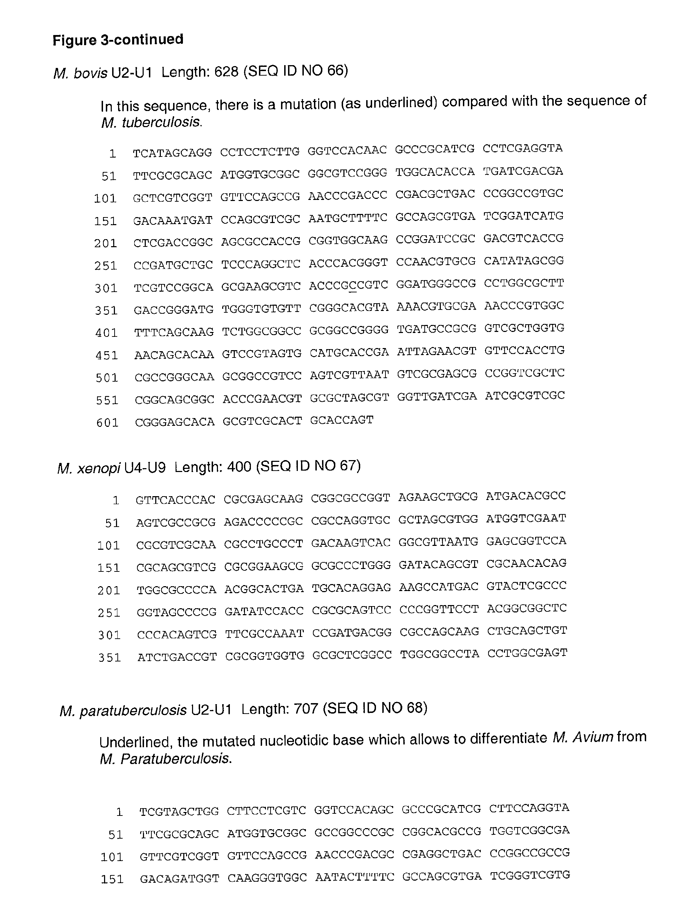

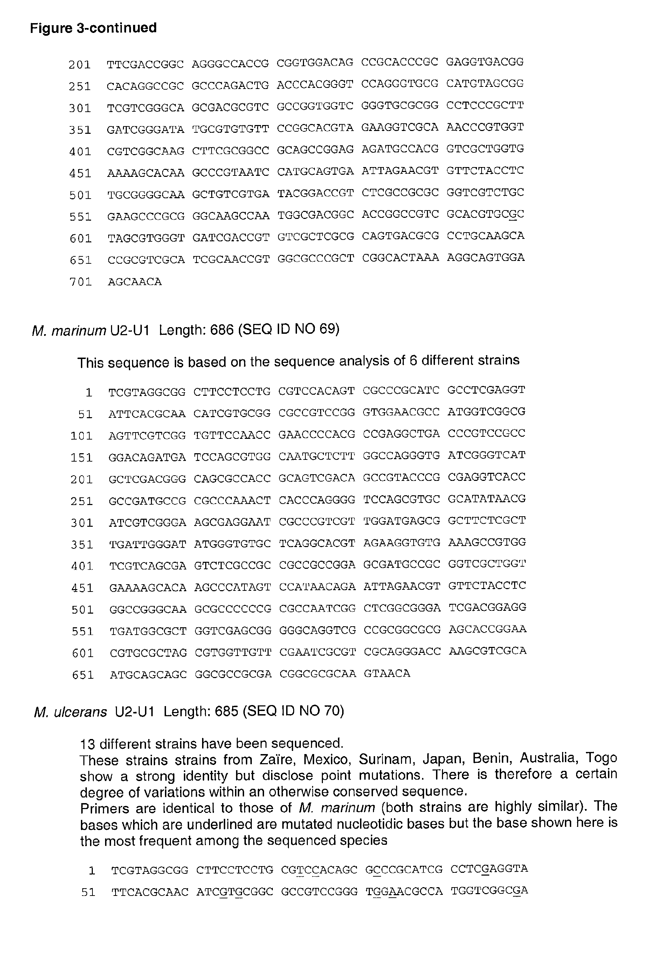

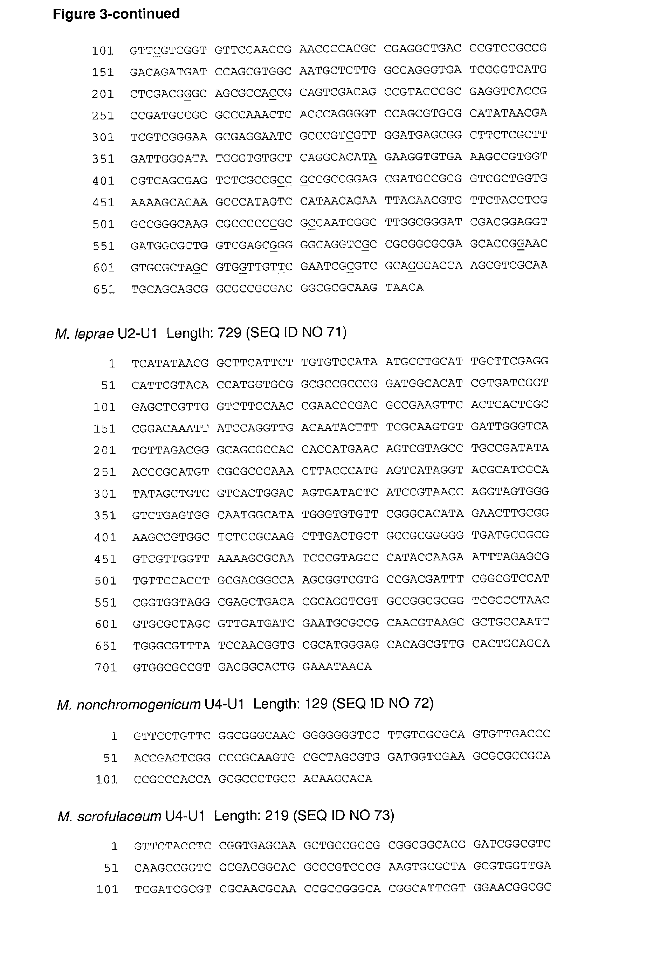

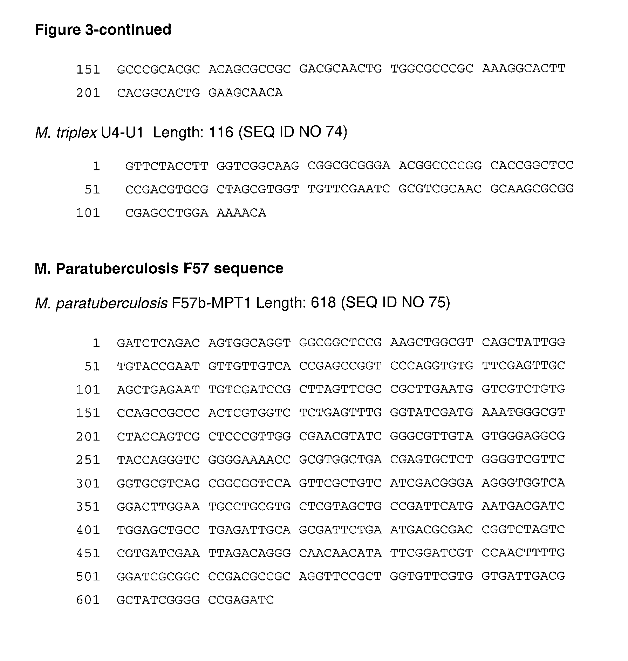

Said probes may comprise the whole or only parts of species-specific us-p34 gene region sequences, some of which are disclosed in Figure 3 and some of which the skilled man has aligned in Figures 1 and Figures 8 to 10. On the basis of such an alignment, it is within the skilled man's knowledge to actually design suitable probes to be used in said method.

It should be clear that in all of the methods of the invention, not only one species-specific probe can be used but also at least two, at least three or at least four or five or more species-specific probes can be used, for instance depending on the type of assay or method.

In this method more than one Mycobacterium strain may be detected, provided that the species-specific probes can be distinguished from each other. Numerous well-known methods are described in the art to come to this result. As an example, the species-specific probes can be labelled with different markers which can be detected by various methods or devices after hybridisation with the target as to distinguish between the nucleotide duplexes formed.

It should be undestood that the above described methode is not limited to the us-p34 sequences actually provided in Figure 3. The us-p34 sequences envisaged in the invention encompass the complete us-p34 sequences and also all the corresponding sequences of the various Mycobacerium species which are shown to contain species-specific differences.

Said differences not only comprise point mutations but also include deletion and/or insertion of several nucleotide bases. These deletions and/or insertions may result in different lengths of the us-p34 upstream region between various species. Accordingly, also probes can be designed overlapping said deletions or insertions, or alternatively spanning the nucleotides of the deletion or the insertion. The skilled man is already guided how to design such species-specific probes by the alignments provided in the figures.

According to a preferred embodiment the present invention more particularly relates to a method as defined above, wherein said Mycobacterium species-specific us-p34 nucleotide probe specifically hybridizes with at least part of a sequence selected from the group of sequences represented by SEQ ID NOs 57, 59 to 64, 67, 69 to 74 (Figure 3), or the complement thereof, or the corresponding sequences wherein T has been replaced by U.

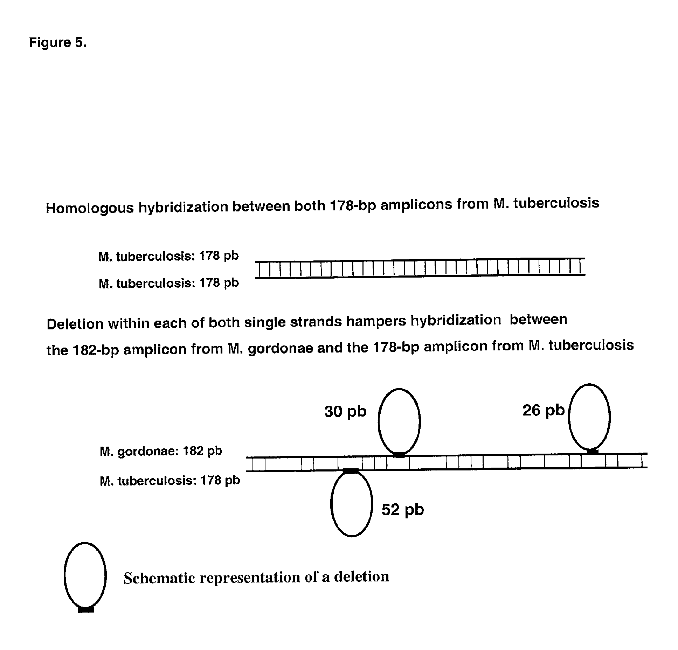

The specific or non-specific hybridization of the species-specific us-p34 probes is further illustrated in Figure 5.

In Tables 3 (a) to (c), nucleotide sequences are represented for use as probe or primer in various methods for detection, identification, hybridisation to, capture, amplification of Mycobacterium species-specific nucleic acids.

The present invention more particularly relates to a method as defined above, wherein said non-tuberculosis Mycobacterium species-specific us-p34 nucleotide probe is selected from the group of sequences represented by SEQ ID NOs 8 to 24, 26 to 47, 49 to 51, 53 and 54 (Tables 3 (a) to (c)) and SEQ ID NOs 57, 59 to 64, 67, 69 to 74 (Figure 3), or the corresponding sequences wherein T has been replaced by U.

The present invention also relates to a method for detecting at least one tuberulosis Mycobacterium (TUB) strain in a sample, comprising:
(i) providing at least one tuberulosis Mycobacterium species-specific upstream p34 gene region (us-p34) nucleotide probe comprising a sequence selected from the group of sequences represented by SEQ ID NOs 25, 48 and 52,
(ii) reacting said us-p34 nucleotide probe with said sample under conditions that allow for the selective formation of nucleotide duplexes between said us-p34 nucleotide probe and a corresponding tuberulosis Mycobacterium nucleic acid target present in said sample, and,
(iii) detecting any nucleotide duplexes containing said us-p34 nucleotide probe.

According to further embodiments of the invention whenever reference is made to sequences represented by a specific SEQ ID NO, it should be clear that the terms "comprising" and "having a sequence" also includes the term "consisting of" relating to the specific sequence "as such". The expressions "comprising a sequence" and "having a sequence" are interchangeable according to the invention.

According to yet another embodiment, the invention relates to a method for the differential detection of Mycobacteria in a sample, comprising:
(i) providing at least two distinct non-tuberculosis Mycobacterium species-specific us-p34 nucleotide probes,
(ii) reacting said us-p34 nucleotide probes with said sample under conditions that allow for the selective formation of nucleotide duplexes between said us-p34 specific nucleotide probe and a non-tuberculosis Mycobacterium nucleic acid present in said sample,
(iii) detecting any nucleotide duplexes containing said us-p34 nucleotide probe, and,
(iv) inferring from the nucleotide duplex formed, the presence and the identification of a specific Mycobacterium strain.

In a preferred embodiment, the sequences of said non-tuberculosis Mycobacterium species-specif us-p34 probes comprise or are selected from the group of sequences represented by any of SEQ ID NOs 8 to 24, 26 to 47, 49 to 51, 53 and 54 (Tables 3 (a) to (c)) and SEQ ID NOs 57, 59 to 64, 67, 69 to 74 (Figure 3).

According to yet an alternative embodiment, the invention relates to a method for the differential detection of Mycobacteria in a sample, comprising:
(i) providing at least two distinct Mycobacterium species-specific us-p34 nucleotide probes, wherein at least one of said species-specific us-p34 nucleotide probes has a sequence selected from the group of sequences represented by any of SEQ ID NOs 58, 65, 66 and 68 and at least one other species-specific us-p34 nucleotide probe has a sequence selected from the sequences represented by any of SEQ ID NOs 8 to 54, 57, 59 to 64, 67, 69 to 74,
(ii) reacting said us-p34 nucleotide probes with said sample under conditions that allow for the selective formation of nucleotide duplexes between said us-p34 specific nucleotide probe and a Mycobacterium nucleic acid present in said sample,
(iii) detecting any nucleotide duplexes containing said us-p34 nucleotide probe, and,
(iv) inferring from the nucleotide duplex formed, the presence and the identification of a specific Mycobacterium strain.

The present invention also relates to a method for detecting at least one Mycobacteria strain in a sample, comprising:
(i) providing at least one suitable us-p34 primer pair comprising a sense or anti-sense Mycobacterium species-specific us-p34 primer,
(ii) reacting said us-p34 primer pair with said sample under conditions that allow for the selective amplification of us-p34 sequences of at least one Mycobacterium nucleic acid present in said sample,
(iii) detecting the amplified product of step (ii), and,
(iv) inferring from the amplified product formed, the presence and the identification of at least one (specific) Mycobacterium strain or species.

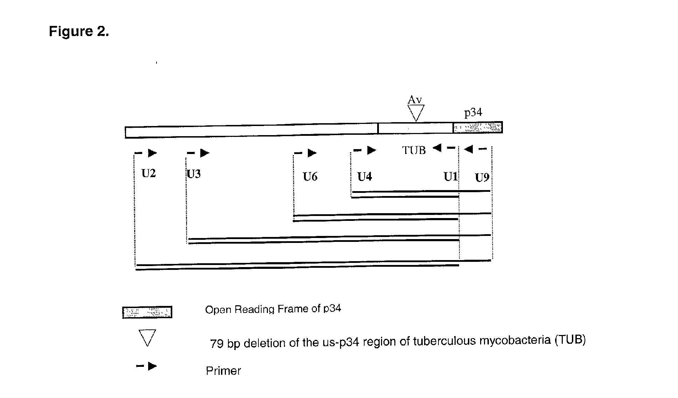

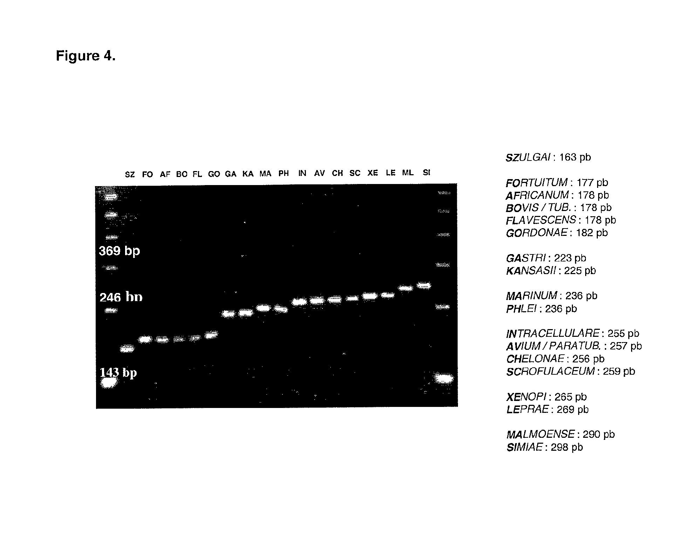

It should be clear to the one skilled in the art and from the information provided in the Figures, especially Figure 4, that the above described method does not specifically require the use of a Mycobacterium species-specific us-p34 primers to be able to identify specific Mycobacterium strans or species from the amplification product formed. It has been found by the present inventors that the differences in the us-p34 region of various species results in different lengths of the amplification products when amplified for instance with the universal primers provided in Tables 1 and 2 (as represented in SEQ ID NOs 1 to 7). The detection of Mycobacterium species in a sample is therefore possible through means of non-specific or specific amplification of the us-p34 regions. Said universal primers are especially designed by the present inventors to amplify us-p34 sequences of most if not all Mycobacterium species. For instance, based on the difference in length of products amplified with the universal probes, a distinction can be made between members of the tuberculosis mycobacteria and the Non-tuberculosis mycobacteria of the avium complex of mycobacteria (Figure 2). In Tables 1 and 2 the skilled man for instance will find several universal sense and antisense primers which can be used according to the sheme represented in Figure 2 to form a suitable primer pair for amplifying the desired us-p34 region in the above method.

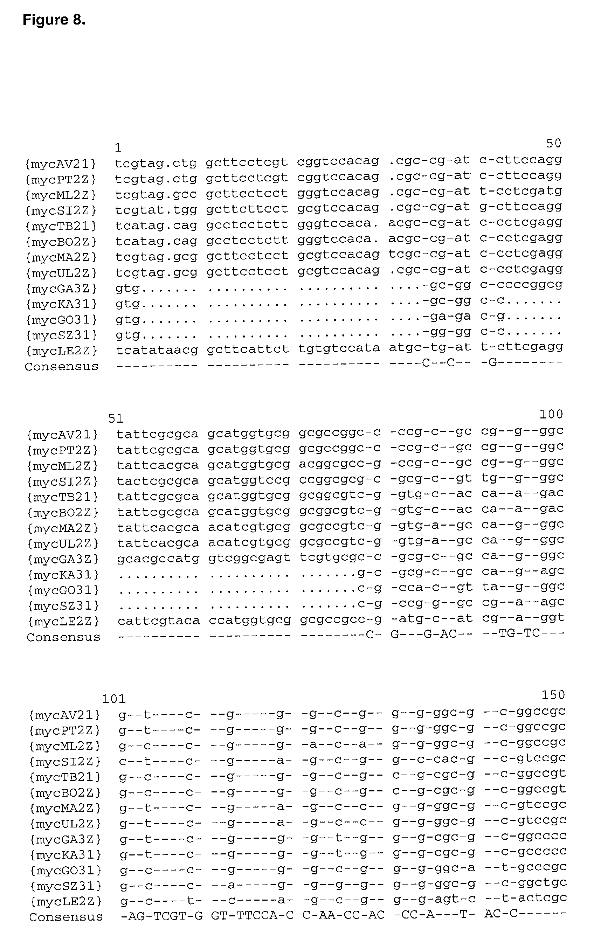

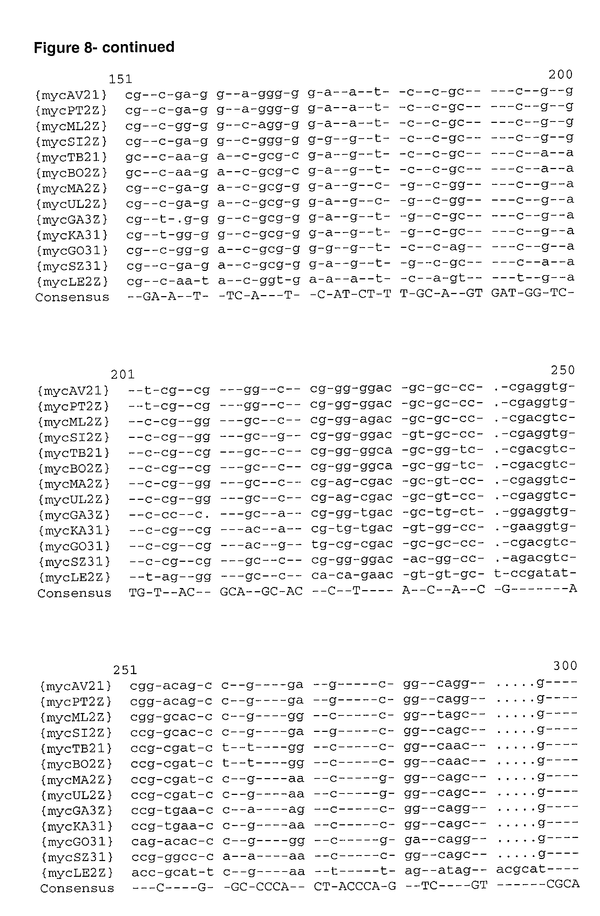

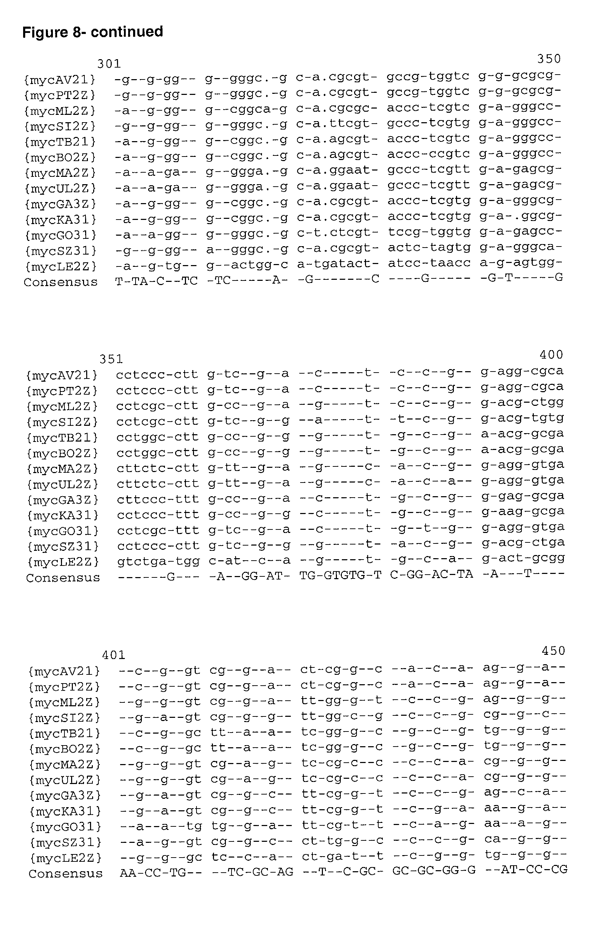

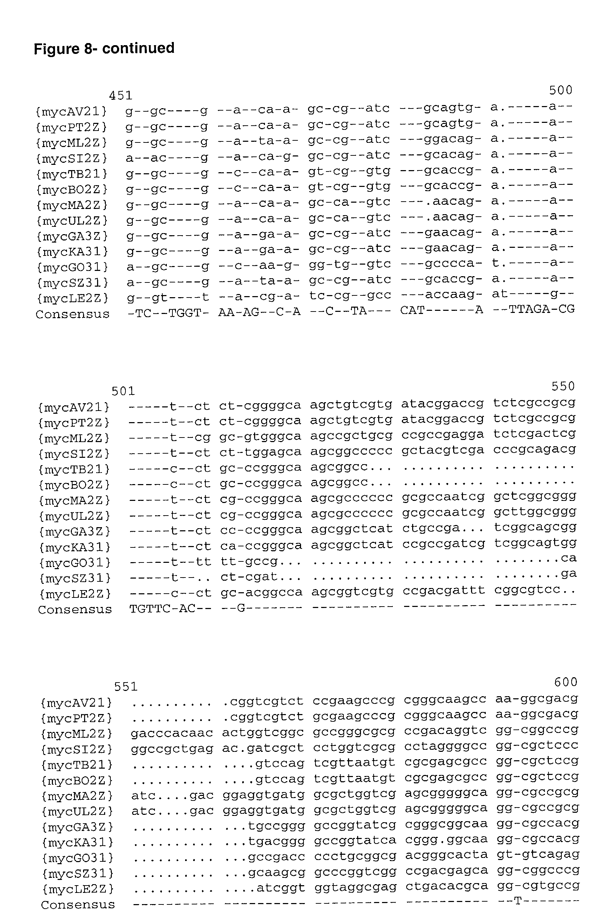

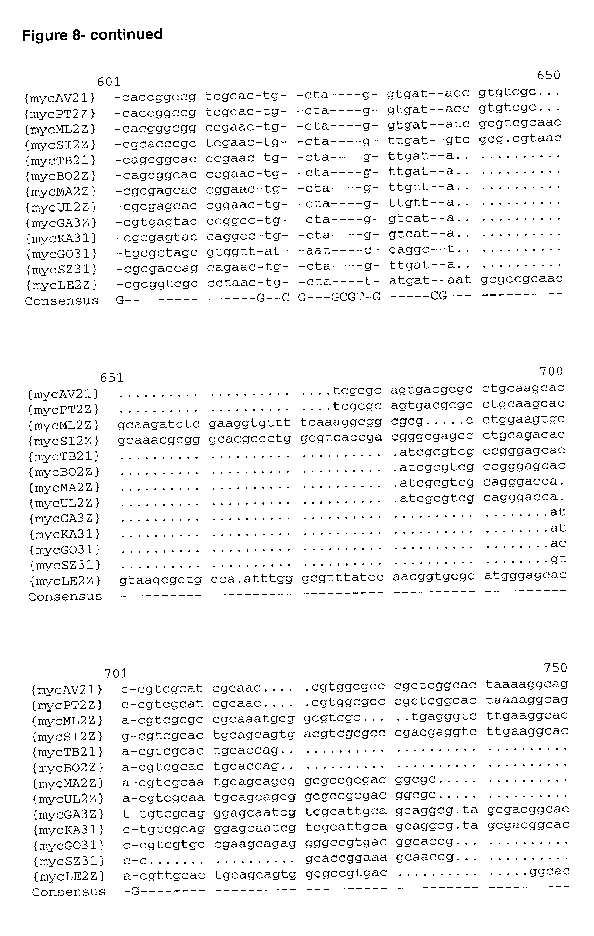

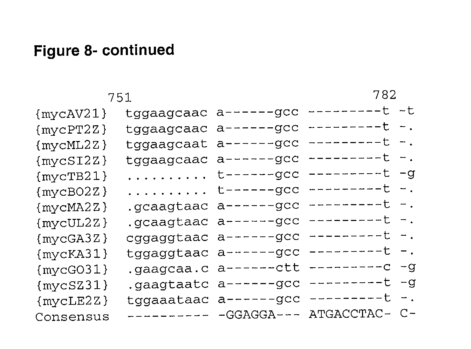

Alternatively also species-specific primers can be used in such a method, which may lead to the species-specific identification of the Mycobacterium present in the sample. Species-specific us-p34 primers are derived from us-p34 sequences (parts of the sequences as given in Figure 3) which are present in one specific species but not in the others. On the basis of the alignment of the sequences as given in Figure 3 (see Figures 8 to 10), it is within the knowledge of the skilled man to design actual primer sequences which will selectively amplify one species (or one type or one strain) of Mycobacterium. Nucleotides having a sequence as respresented in any of SEQ ID NOs 8 to 54 (Table 3 (a) to (c)) can be used as a species-specific primer in a such a method. Also primers can be designed overlapping deletions or insertions which are apperent from the alignments provided in the figures, or alternatively primers spanning the nucleotides of the deletion or the insertion. The skilled man is already guided how to design such species-specific primers by studying the figures of the application.

The invention thus also relates to a method for detecting at least one Mycobacterium strain in a sample, comprising:
(i) providing at least one suitable primer pair comprising a sense or antisense Mycobacterium species-specific us-p34 primer selected from the group of sequences represented by SEQ ID NOs 8 to 54,
(ii) reacting said us-p34 primer pair with said sample under conditions that allow for the selective amplification of an us-p34 sequence in a Mycobacterium nucleic acid present in said sample,
(iii) detecting the amplified product of step (ii), and,
(iv) inferring from the amplification product the presence and the identification of at least one specific Mycobacterium strain.

It should be obvious for the skilled in the art that al least one primer constituting said primer pair can be chosen from the sequences provided in Table 3 (a) to (c) where sequences of a sense and antisense primer are provided for a specific Mycobacterium species. Alternatively, the skilled in the art can choose a second primer from the universal primers presented in Tables 1 and 2 and represented by SEQ ID NOs 1 to 7. As such, the amplification products resulting from one universal and one species-specific primer are still species-specific.

The invention also relates to a method for the differential detection of mycobacteria in a sample, comprising:
(i) providing at least one suitable us-p34 primer pair comprising a sense and anti-sense Mycobacterium species-specific us-p34 primer,
(ii) reacting said us-p34 primer pair with said sample under conditions that allow for the selective amplification of us-p34 sequences of at least one Mycobacterium nucleic acid present in said sample,
(iii) detecting the amplified product of step (ii), and,
(iv) inferring from the amplified product formed, the presence and the identification of at least one (specific) Mycobacterium.

The sense and anti-sense primers for use in the above method can be selected from the group of sequences represented by SEQ ID NOs 4 to 54.

The invention further relates to a method for the differential detection of mycobacteria in a sample, comprising:
(i) providing at least one suitable primer pair conmprising a a first sense or anti-sense Mycobacterium us-p34 primer selected from the group of sequences represented by SEQ ID NOs 1 to 3 (Table 1) and a second sense or anti-sense Mycobacterium us-p34 primer selected from the group of sequences represented by SEQ ID NOs 8 to 54,
(ii) reacting said us-p34 primer pair with said sample under conditions that allow for the selective amplification of us-p34 sequences of at least one Mycobacterium nucleic acid present in said sample,
(ii) detecting the amplified product of step (ii), and,
(iii) inferring from the amplified product formed, the presence and the identification of at least one specific Mycobacterium.

As described above, already the length of the amplification products may be indicative for the presence of a specific Mycobacterium species, since the amplification products resulting from one universal and one species-specific primer are still species-specific. Therefore according to a further embodiment, the primers to be used in the above method are selected from the sequences represented in SEQ ID NOs 1 to 7 and presented as universal primers in Tables 1 and 2. In addition, also species-specific us-p34 primers can be used in the above method. The invention thus also relates to a method as described above wherein a first primer constituting the primer pair is selected from the universal primers represented by SEQ ID NOs 1 to 7 and a second primer is selected from the species-specific primers represented by SEQ ID NOs 8 to 54.

According to a preferred embodiment the invention relates to a method for the detection of a Mycobacterium species in strain, said Mycobacterium species belonging to the MAC complex, and said method comprising:
(i) providing at least one us-p34 probe having a sequence selected from the group of sequences represented in any of SEQ ID NOs 8, 14, 15, 22, 27, 28, 29, 34, 35, 50, 51, 57, 58 , 68 and 73,
(ii) reacting said us-p34 probe with said sample under conditions that allow for the selective formation of nucleotide duplexes between said us-p34 nucleotide probe and a MAC complex Mycobacterium nucleic acid target in said sample, and,
(iii) detecting any nucleotide duplexes containing said us-p34 nucleotide probe.

Members belonging to the MAC comples Mycobacterium species are presented in Table 4 and species-specific us-p34 primers are presened in Table 3 (a) to (c). In an alternative method also embodied by the present invention, step (iii) of the above-described method can be replaced by the step of determining the us-p34 sequence of said MAC Mycobacterium nucleic acid in said sample wherein the with the expression "determining the us-p34 sequence" is meant, for instance, direct sequencing to confirm the presence of said specific MAC complex us-p34 sequence. Other methods can be by mass spectrometry, capillary electrophoresis or HPLC.

According to a preferred embodiment the invention relates to a method for the detection of MOTT Mycobacterium species in a sample, comprising:
(i) providing at least one us-p34 probe having a sequence selected from the group of sequences as represented in any of SEQ ID NOs 9 to 13, 16 to 21, 23, 24, 26, 30 to 33, 36 to 47, 49, 53, 54, 59 to 64, 67, 69 to 72 and 74,
(ii) reacting said us-p34 probe with said sample under conditions that allow for the selective formation of nucleotide duplexes between said us-p34 nucleotide probe and a MOTT Mycobacterium nucleic acid target in said sample, and,
(iii) detecting any nucleotide duplexes containing said us-p34 nucleotide probe.

Members belonging to the MOTT comples Mycobacterium species are presented in Table 4 and species-specific us-p34 primers are presened in Table 3 (a) to (c). In an alternative method also embodied by the present invention, step (iii) of the above-described method can be replaced by the step of determining the us-p34 sequence of said MOTT Mycobacterium nucleic acid in said sample wherein the with the expression "determining the us-p34 sequence" is meant, for instance, direct sequencing to confirm the presence of said specific MOTT complex us-p34 sequence. Other methods can be by mass spectrometry, capillary electrophoresis or HPLC.

The invention further relates to a method for detecting new us-p34 sequences in a sample, comprising:
(i) providing at least one suitable primer pair comprising a sense and anti-sense us-p34 primer having a sequence selected from the sequences as represented in any of SEQ ID NOs 1 to 7,
(ii) reacting said us-p34 primer pair with said sample under conditions that allow for the amplification of an us-p34 sequence in a Mycobacterium nucleic acid target in said sample, and,
(iii) determining the sequence of the amplification product obtained in (ii).

The invention also relates to a method for detecting new us-p34 sequences in a sample, comprising:
(i) providing at least one suitable primer pair comprising a sense or anti-sense us-p34 primer having a sequence selected from the sequences as represented in any of SEQ ID NOs 1 to 3 and a sense or anti-sense us-p34 primere selected from the sequences represented in SEQ ID NOs 4 to 7,
(ii) reacting said us-p34 primer pair with said sample under conditions that allow for the amplification of an us-p34 sequence in a Mycobacterium nucleic acid target in said sample, and,
(iii) determining the sequence of the amplification product obtained in (ii).

The expression "new us-p34 sequences" refers to new (not yet identified) mycobacterium species sequences. Some of these new sequences are disclosed in Figure 3.

The present invention further relates to a method for the differential detection of mycobacteria in a sample, comprising:
(i) providing at least one suitable primer pair comprising a sense and anti-sense us-p34 primer sequence selected from the sequences as represented in any of SEQ ID NOs 1 to 7,
(ii) reacting said us-p34 primer pair with said sample under conditions that allow for the amplification of an us-p34 sequence in a Mycobacterium nucleic acid target in said sample,
(iii) selectively hybridizing the amplification products obtained in (ii) with at least one Mycobacterium species-specific us-p34 nucleotide probe having a sequence selected from the group of sequences represented in any of SEQ ID NOs 8 to 74,
(iv) detecting any nucleotide duplexes containing said Mycobacterium species-specific us-p34 nucleotide probe, and,
(v) inferring from the nucleotide duplex formed the presence of a specific Mycobacterium species.

Different examples of how universal primers and species-specific and probes are used in the above method are shown in Table 3.

In some interesting methods according to the invention it is possible to differentiate between two Mycobacterium species.

Therefore in one embodiment, the invention relates to a method for differentiating between Mycobacterium bovis and Mycobacterium tubercolusosis in a sample, comprising:
(i) providing at least one us-p34 probe selective for Mycobacterium bovis or Mycobacterium tuberculosis wherein said probe comprises the sequence as represented in SEQ ID NO 66 for Mycobacterium bovis or SEQ ID NO 65 for Mycobacterium tuberculosis,
(ii) reacting said us-p34 nucleotide probe with said sample under conditions that allow for the selective formation of nucleotide duplexes between said us-p34 nucleotide probe and a corresponding Mycobacterium bovis or tuberculosis nucleic acid target present in said sample, and,
(iii) detecting any nucleotide duplexes containing said Mycobacterium bovis or tuberculosis specifc us-p34 nucleotide probe.

The invention also relates to a method for differentiating between Mycobacterium bovis and Mycobacterium tubercolusosis in a sample, comprising:
(i) providing at least one suitable primer pair comprising at least one sense or antisense us-p34 primer selective for Mycobacterium bovis or Mycobacterium tuberculosis wherein said primer comprises the sequence as represented in SEQ ID NO 66 for Mycobacterium bovis or SEQ ID NO 65 for Mycobacterium tuberculosis,
(ii) reacting said us-p34 primer pair with said sample under conditions that allow for the selective amplification of Mycobacterium bovis and/or Mycobacterium tuberculosis nucleic acid target present in said sample, and,
(iii) inferring from the reaction product(s) the presence of Mycobacterium bovis and/or Mycobacterium tuberculosis in said sample.

The invention further relates to a method for differentiating between Mycobacterium avium and Mycobacterium avium subspecies paratuberculosis in a sample, comprising:
(i) providing at least one us-p34 probe selective for Mycobacterium avium and Mycobacterium avium subspecies paratuberculosis wherein said probe has a sequence selected from the sequences represented in any of SEQ ID NOs 8, 27 to 29, 50 or 58 for Mycobacterium avium or SEQ ID NO 68 for Mycobacterium avium subspecies paratuberculosis ;
(ii) reacting said us-p34 nucleotide probe with said sample under conditions that allow for the selective formation of nucleotide duplexes between said us-p34 nucleotide probe and a corresponding Mycobacterium avium and Mycobacterium avium subspecies paratuberculosis nucleic acid target present in said sample, and,
(iii) detecting any nucleotide duplexes containing said Mycobacterium avium and Mycobacterium avium subspecies paratuberculosis specifc us-p34 nucleotide probe

The invention also relates to a method for differentiating between Mycobacterium avium and Mycobacterium avium subspecies paratuberculosis in a sample, comprising:
(i) providing at least one suitable primer pair comprising at least one sense or antisense us-p34 primer selective for Mycobacterium avium and Mycobacterium avium subspecies paratuberculosis wherein said primer has a sequence selected from the sequences as represented in any of SEQ ID NOs 8, 27 to 29, 50 or 58 for Mycobacterium avium or SEQ ID NO 68 for Mycobacterium avium subspecies paratuberculosis,
(ii) reacting said us-p34 primer pair with said sample under conditions that allow for the selective amplification of Mycobacterium avium and Mycobacterium avium subspecies paratuberculosis nucleic acid target present in said sample, and,
(iii) inferring from the reaction product(s) the presence of Mycobacterium avium and Mycobacterium avium subspecies paratuberculosis in said sample.

The present invention also relates to a Mycobacterium species-specific us-p34 nucleotide probe or primer comprising at least 8 contiguous nucleotides from one of the nucleic acid sequences as represented in any of SEQ ID NOs 57 to 74, or the complement thereof, or the corresponding sequences wherein T has been replaced by U.

The present invention also relates to the Mycobacterium species-specific us-p34 nucleotide probe or primer having a sequence as represented in any of SEQ ID NOs 8 to 54.

The invention also relates to the Mycobacterium us-p34 universal primers having a sequence as represeted in any of SEQ ID NOs 1 to 7.

The present invention also relates to the non-tuberculosis Mycobacteria species-specific us-p34 nucleotide probe or primer having a sequence selected from the group of sequences as represented by SEQ ID NOs 8 to 74 an as shown in Table 3 and Figure 3.

The inenvention also relates to a nucleic acid comprising a sequence selected from the group of sequences as represented in any of SEQ ID Nos 8 to 74.

The present invention also relates to a composition comprising at least one nucleotide probe, primer or nucleic acid as defined above. Said composition preferably contains two, three or more of said components.

The present invention also relates to a diagnostic kit comprising a probe, primer or nucleic acid as defined above or a composition as defined above.

The invention further relates to a method for producing a biochip or a micro-array comprising attaching at least one Mycobacterium species-specific probe as describe above to a solid phase.

The invention also relates to a diagnostic kit for detecting at least one, or at least two or more nucleic acids chosen from the group of nucleic acids as represented in any of SEQ ID NOs 57 to 74.

The present invention also relates to a solid support for the detection of (specific) mycobacteria comprising fixed to said support at least two capture probes selected from Table 3 (SEQ ID NOs 8 to 54) or having a sequence selected from the group of sequences as represented by any of SEQ ID NOs 57 to 74.

The invention also relates to a solid support for the detection of mycobacteria comprising fixed to said support at least one nucleic acid having a sequence as represented in any of SEQ ID NOs 1 to 7.

The invention further relates to a micro-array for performing any of the methods of the invention, comprising a solid support, said solid support having immobilized thereon a set of different capture probes, said set of capture probes being optionally sub-divided in sub-sets of capture probes, wherein optionally each of said capture probes or each of said sub-set of capture probes is immobilized within a predefined region on said solid support. The methods wherein such a micro-array are used preferably comprise a hybridisation step.

The invention further relates to a micro-array comprising at least one Mycobacterium species-specific capture probe or nucleic acid as described earlier.

The invention also relates to a micro-array which allows detection of one or more nucleic acids chosen from the group of nucleic acids represented in any of SEQ ID NOs 57 to 74. The present invention also relates to a solid support as defined above for use in a method as defined above.

Different techniques can be applied to perform the methods of the present invention. These techniques may comprise immobilizing the target polynucleic acids, after amplification, on a solid support and performing hybridization with labelled oligonucleotide probes. Alternatively, the probes of the invention may be immobilized on a solid support (covalently or non-covalently) and hybrdization may be performed with labelled target polynucleic acids, possibly after amplification. This technique is called reverse hybridization. Said techniques are well-known to the man skilled in the art.

It is to be understood that also any other technique for detection of the above-mentioned amplified target sequences are also covered by the present invention. Such a technique can involve sequencing or other micro-array methods known in the art.

Micro-array methods exploit the preferential binding of complementary single-stranded nucleic acid sequences. A microarray is for instance a glass, silica (or other non-porous or porous material) slide or bead (or other geometric format), onto which nucleic acid molecules are attached at fixed locations (spots). There may be up to tens of thousands of spots on an array, each containing a number of identical nucleic acid molecules (or fragments or identical molecules), of lengths from ten to hundreds of nucleotides. The spots are either printed on the microarray by a robot, or synthesized by photo-litography (similarly as in computer chips productions) or by ink-jet printing or any other method known to the man skilled in the art. There exist several names for these technologies and these devices, like micro-array, DNA array, nucleic acid array, DNA chip, biochip, gene chip, or others. These names are all synonyms according to the invention.

The methods and assays described herein are not only applicable to Mycobacterium species, but also to detect and differentiate between Pseudomonas species, based on species-specific probes originating from the rRNA operon *(rrn),*

The Pseudomonas family belongs to the family of non-fermenting bacteria (7, 8). They are divided into 5 groups based on the homology level of their ribosomal RNA (Table 5).

From a clinical point of view, the three species most frequently found are: *Pseudomonas aeruginosa, Burkholderia cepacia, Stenotrophomonas maltophilia.*

The present inventors developed a method of differential identification allowing the discrimination of at least three species belonging to the same family of Pseudomonas: *Pseudomonas aeruginosa, Burkholderia cepacia, Stenotrophomonas maltophilia.* The identification method is based on the rRNA operon *(rrn),* more particularly on the variable region (spacer) which separates the different genes coding for 16S and 23S rRNA and the Isoleucine and Alanine tRNA.

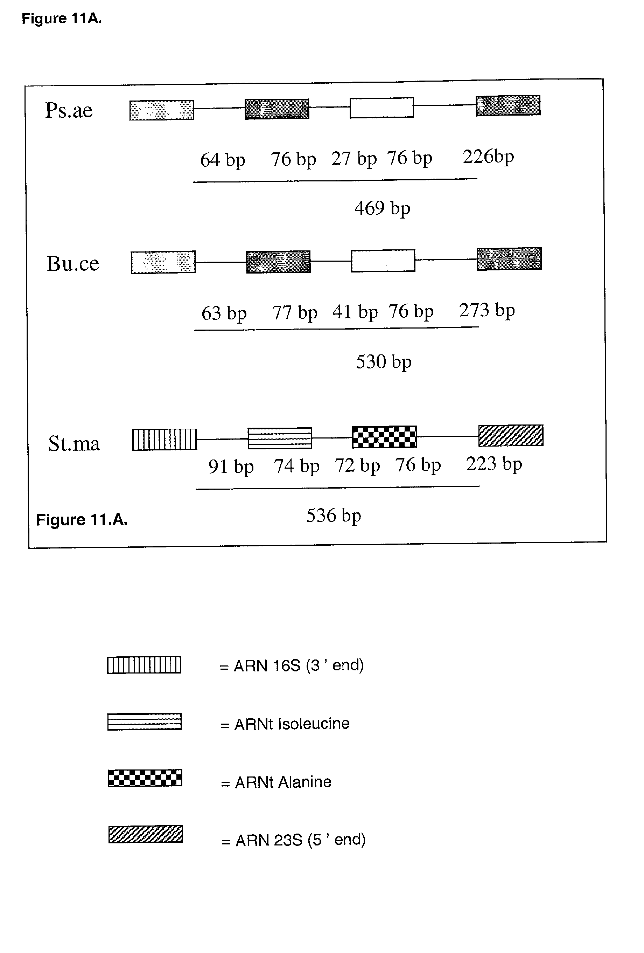

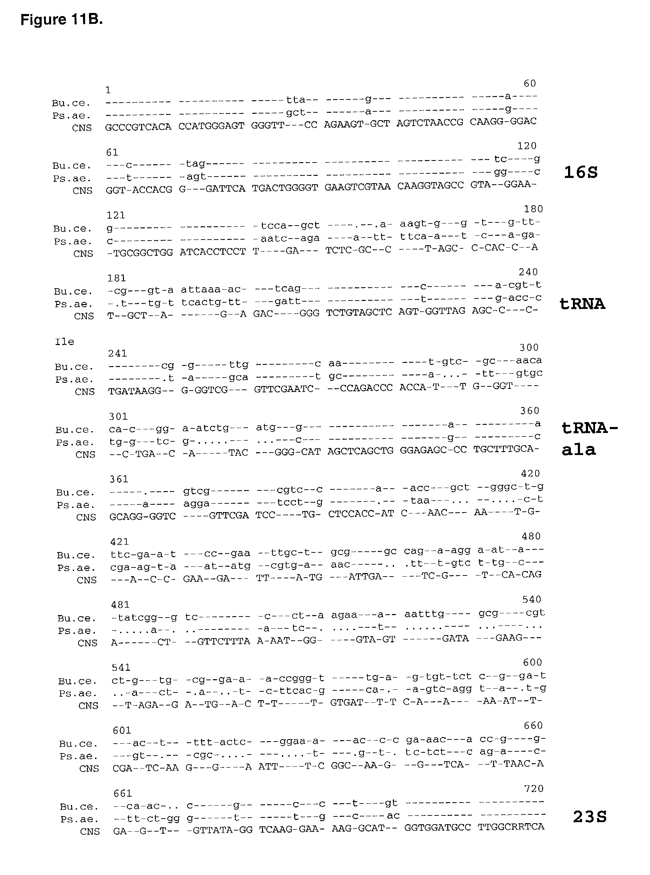

The bacteria belonging to the "Pseudomonas" group have an operon coding for the three ribosomal RNA, 16S, 23S and 5S. The organization of this operon is relatively conserved (Figure 11A). Between the sequences encoding 16S and 23S rRNA, one finds sequences coding for two tRNA (Isoleucine and Alanine tRNA) separated by non-coding regions of variable length and composition (Figure 11A and 11B). It is also noticed that these two tRNA have the following order in *P. aeruginosa* and *B. cepacia:* Isoleucine tRNA followed by Alanine tRNA and that this order is reversed in *S. maltophilia.*

In this *rrn* operon the present inventors identified species-specific probes and primers which could be used in the methods of the invention.

Therefore, the present invention relates to a method for detecting at least one Pseudomonas strain in a sample, comprising:
(i) providing at least one Pseudomonas species-specific rRNA operon *(rrn)* nucleotide probe,
(ii) reacting said *rrn* nucleotide probe with said sample under conditions that allow for the selective formation of nucleotide duplexes between said *rrn* nucleotide probe and a corresponding Pseudomonas nucleic acid target present in said sample, and,
(iii) detecting any nucleotide duplexes containing said *rrn* nucleotide probe.

In a further embodiment, said Pseudomonas species-specific *rrn* nucleotide probe in the above method, specifically hybridizes with at least part of a sequence selected from the sequences represented in any of SEQ ID NOs 76 to 82, or the complement thereof, or the corresponding sequences wherein T has been replaced by U.

In a further embodiment, in said method, said Pseudomonas species-specific *rrn* nucleotide probe is selected from the group of sequences represented in SEQ ID NOs 76 to 82 and 84 to 86, or the complement thereof, or the corresponding sequences wherein T has been replaced by U.

The invention also relates to a method for the differential detection of Pseudomonas in a sample, comprising:
(i) providing at least one Pseudomonas species-specific *rrn* nucleotide probe, for instance a Pseudomonas species-specific *rrn* nucleotide probe selected from the group of sequences represented in any of SEQ ID NOs 76 to 82 and 84 to 86, or the complement thereof, or the corresponding sequences wherein T has been replaced by U.
(ii) reacting said *rrn* nucleotide probe with said sample under conditions that allow for the selective formation of nucleotide duplexes between said *rrn* specific nucleotide probe and a Pseudomonas nucleic acid present in said sample,
(iii) detecting any nucleotide duplexes containing said *rrn* nucleotide probe, and,
(iv) inferring from the nucleotide duplex formed, the presence and the identification of a specific Pseudomonas strain.

A further molecular strategy developed by the inventors was an amplification by multiplex PCR allowing the production of fragments of different sizes according to the species from which DNA was amplified (Figure 12). The sensibility and the specificity of this strategy was validated on clinical strains (Figure 13 and 14).

Therefore, in one embodiment the invention relates to a method for the differential detection of Pseudomonas in a sample, comprising:
(i) providing at least one suitable *rrn* primer pair containing a sense or anti-sense *rrn* primer,
(ii) reacting said *rrn* primer pair with said sample under conditions that allow for the selective amplification of *rrn* sequences of at least one Pseudomonas nucleic acid present in said sample,
(iii) detecting the amplified product of step (ii), and,
(iv) inferring from the amplified product formed, the presence and the identification of at least one (specific) Pseudomonas.

The *rrn* primer pair which can be used in the above method can comprise a sequence selected from the group of sequences as represented in any of SEQ ID NOs 83 to 86. For instance, in the above method, SEQ ID NO 83 can be used as a sense primer in combination with any of the sequences represented in SEQ ID NOs 85 to 86 which represent species-specific *rrn* antisense primers. Amplification with these primer-pairs may lead to amplification products of different length, indicative for the presence of a specific Pseudomonas species. Therefore with "inferring from the amplified product" is meant for instance, analyzing the length of the amplified product, for instance on an agarose gel.

The methods of the invention can also be adapted for species-specific detection of members of the Pseudomonas familiy.

For instance, the invention also relates to a method for the detection of Pseudomonas aeruginosa in a sample, comprising:
(i) providing at least one *rrn* probe selected from the group of sequences represented in any of SEQ ID NOs 76, 80 or a fragment thereof comprising the sequence as represented in SEQ ID NO 85, or the complement thereof, or a the corresponding sequences wherein T has been replaced by U,
(ii) reacting said *rrn* probe with said sample under conditions that allow for the selective formation of nucleotide duplexes between said *rrn* nucleotide probe and a Pseudomonas aeruginosa nucleic acid target in said sample, and,
(iii) detecting any nucleotide duplexes containing said *rrn* nucleotide probe.

The invention also relates to a method for the detection of Burkholderia cepacia in a sample, comprising:
(i) providing at least one *rrn* probe selected from the group of sequences represented in any of SEQ ID NOs 77, 71, or a fragment thereof comprising the sequence as represented in SEQ ID NO 84, or the complement thereof, or a the corresponding sequences wherein T has been replaced by U,
(ii) reacting said *rrn* primer probe said sample under conditions that allow for the selective formation of nucleotide duplexes between said *rrn* nucleotide probe and a Burkholderia cepacia nucleic acid target in said sample, and,
(iii) detecting any nucleotide duplexes containing said *rrn* nucleotide probe.

The invention further relates to a method for the detection of Stenotrophomonas maltophilia in a sample, comprising:
(i) providing at least one *rrn* probe selected from the group of sequences as represented in SEQ ID NO 82, or a fragment thereof comprising the sequence as represented in SEQ ID NO 86, or the complement thereof, or a the corresponding sequences wherein T has been replaced by U,
(ii) reacting said *rrn* primer probe said sample under conditions that allow for the selective formation of nucleotide duplexes between said *rrn* nucleotide probe and a Stenotrophomonas maltophilia nucleic acid target in said sample, and,
(iii) detecting any nucleotide duplexes containing said *rrn* nucleotide probe

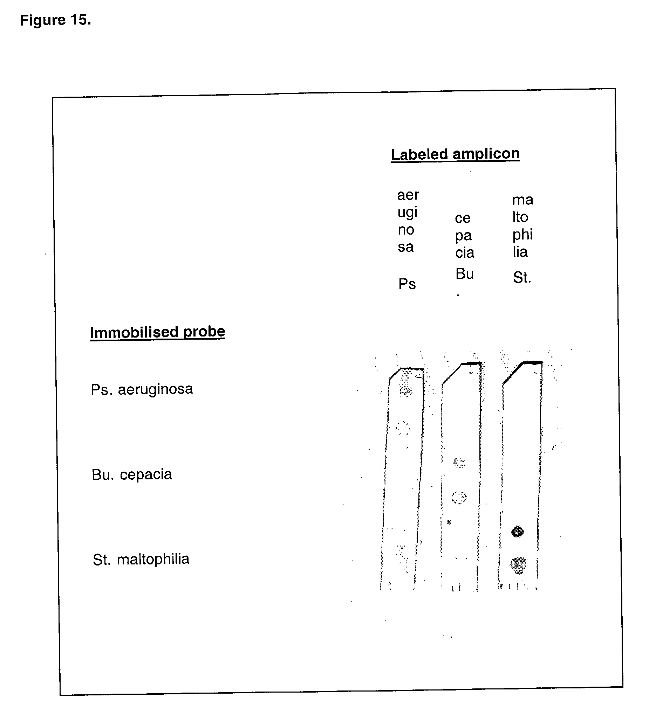

Yet another strategy comprises the development of a reverse hybridization system wherein the labeled and amplified DNA is specifically hybridized to probes corresponding to the different species (Figure 15). The invention thus also relates to multiplex PCR methods for identification of Pseudomonas species comprising the methods of the invention, as illustrated by the figures and the examples.

Therefore, the invention also relates to a method for the differential detection of Pseudomonas in a sample, comprising:
(i) providing a primer pair comprising a sense and anti-sense *rrn* sequence as represented in SEQ ID NOs 83 and 87,
(ii) reacting said *rrn* primer pair with said sample under conditions that allow for the amplification of an *rrn* sequence in a Pseudomonas nucleic acid target in said sample,
(iii) selectively hybridizing the amplification products obtained in (ii) with at least one Pseudomonas species-specific *rrn* nucleotide probe selected from the group of sequences represented in SEQ ID NOs 76 to 82 and 84 to 86,
(iv) detecting any nucleotide duplexes containing said Pseudomonas species-specific *rrn* nucleotide probe, and,
(v) inferring from the nucleotide duplex formed, the presence of a specific Pseudomonas species.

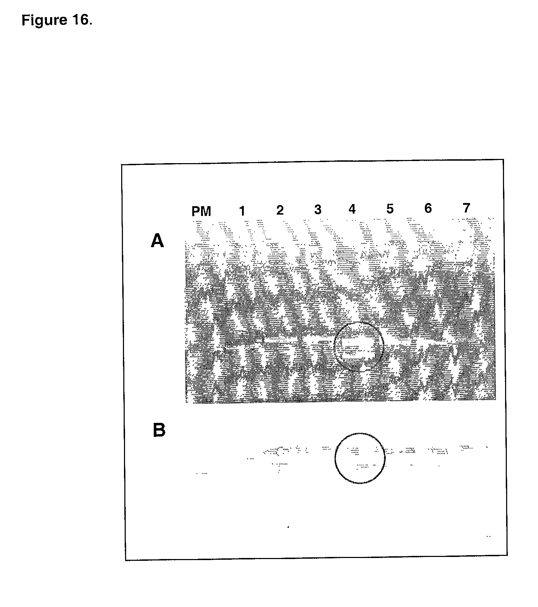

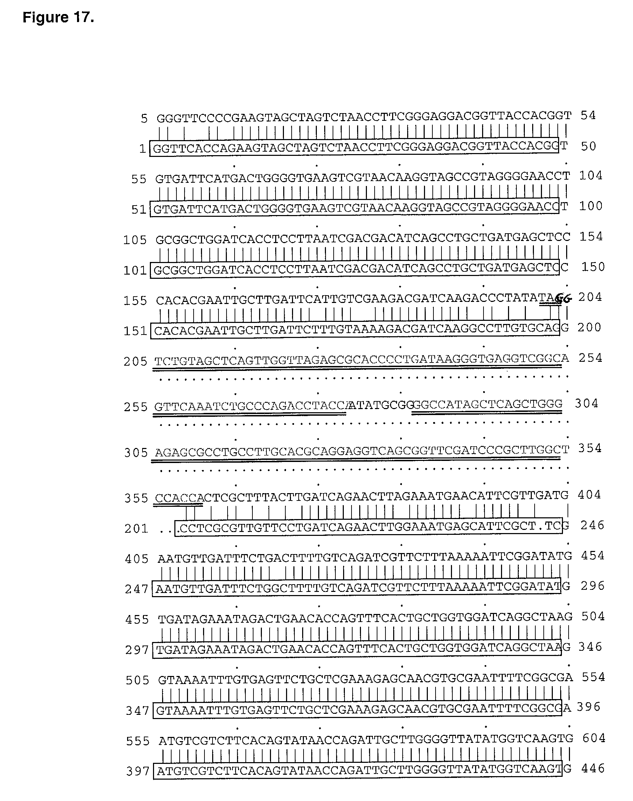

In order to extend the amplification strategies by multiplex PCR and reverse hybridization to other species from the *Ps. aeruginosa* group, an amplification of the 3' end of 16S tRNA to the 5' end of 23S tRNA was performed with DNA from control strains of *Ps. putida, Ps. fluorescens, Ps. alcaligenes* and *Ps. pseudoalcaligenes,* using PsSeq1 and PsSeq2 primers. Surprisingly the inventors noticed that for *Ps. putida* two bands of equal intensity but of different sizes were obtained (Figure 16A). The presence of this second band of smaller size was confirmed for two other isolates of *Ps. putida* (clinical samples form different origin). A hybridization was performed using a probe produced by DNA amplification of *Ps. aeruginosa* (primers PsSeq1 and PsSeq2). The colorimetric detection of the hybrids confirmed that this smaller band was specific of the *rrn* gene (Figure 16B). The two fragments were cloned and sequenced. The sequences confirmed that in *Ps. putida,* two *rrn* operons of different sizes were present, one of which has a deletion. The sequences are represented in SEQ ID NOs 78 and 79. This deletion covers the region coding for the two tRNA corresponding to Alanine and Isoleucine amino acids (Figure 17). Based on this knowledge, methods and primers for differential diagnosis are designed. Accordingly, in one embodiment, the invention also relates to a method for the detection of Pseudomonas putida in a sample, comprising:
(i) providing at least one *rrn* probe selected from the group of sequences represented in any of SEQ ID NOs 78 or 79, or a fragment thereof, or the complement thereof, or a the corresponding sequences wherein T has been replaced by U,
(ii) reacting said *rrn* primer probe said sample under conditions that allow for the selective formation of nucleotide duplexes between said *rrn* nucleotide probe and a Pseudomonas putida nucleic acid target in said sample, and,
(iii) detecting any nucleotide duplexes containing said *rrn* nucleotide probe.

For instance, a probe which is very useful for specifically detecting Pseudomonas putida can be designed from the alignment provided in Figure 17, an for instance is a probe having a sequence corresponding to at least part of SEQ ID NO 79, wherein said part overlaps with the deletion.

The invention also relates to a method for detecting new *rrn* sequences in a sample, for instance based on the universal probes of the invention, comprising:
(i) providing a primer pair comprising a sense and anti-sense *rrn* sequence as represented in SEQ ID NOs 83 and 87,
(ii) reacting said *rrn* primer pair with said sample under conditions that allow for the amplification of an *rrn* sequence in a Pseudomonas nucleic acid target in said sample, and,
(iii) determining the sequence of the amplification product obtained in (ii).

The invention further relates to a Pseudomonas species-specific *rrn* nucleotide probe or primer comprising at least 8 contiguous nucleotides from one of the nucleic acid sequences represented in SEQ ID NOs 76 to 82, or the complement thereof, or the corresponding sequences wherein T has been replaced by U.

The invention also relates to a Pseudomonas species-specific *rrn* nucleotide probe or primer selected from the sequences as represented in SEQ ID NOs 84 to 86.

The invention further relates to a a Pseudomonas *rrn* nucleotide primer selected from the sequences as represented in SEQ ID NOs 83 and 87.

Furhtermore, the invention relates to a nucleic acid comprising a sequence selected the group of sequences represented in any of SEQ ID NOs 76 to 86, to a composition comprising at least one nucleotide probe, primer or nucleic acid of the invention, and to a diagnostic kit comprising at least one nucleotide probe, primer, nucleic acid or composition of the invention.

The invention also relates to a method for producing a biochip or a micro-array comprising attaching at least one Pseudomonas species-specific probe as described herein.

The invention further relates to a diagnostic kit for detecting at least one, at least two or more nucleic acids chosen from the group of nucleic acids represented in any of SEQ ID NOs 76 to 82.

Furthermore, the invention relates to a micro-array for performing any of the methods of the invention wherein a hybridisation step is involved, comprising a solid support, said solid support having immobilized thereon a set of different capture probes, said set of capture probes being optionally sub-divided in sub-sets of capture probes, wherein optionally each of said capture probes or each of said sub-set of capture probes is immobilized within a predefined region on said solid support. For instance, said micro-array comprises at least one Pseudomonas species-specific capture probe or nucleic acid.

The invention further relates to a solid support for the detection of Pseudomonas comprising fixed to said support at least one, at least two, or at least more probes selected from any one of SEQ ID NOs 76 to 86 and to the use of said solid support or array in any of the methods described above. For instance, said solid support can be used as a biochip or as a microarray.

The following definitions and explanations will permit a better understanding of the present invention.

The target material in the samples to be analysed may either be DNA or RNA, e.g. genomic DNA, messenger RNA or amplified versions thereof. These molecules are in this application also termed "polynucleic acids" or "nucleic acids".

Well-known extraction and purification procedures are available for the isolation of RNA or DNA from a sample (e.g. in Sambrook *et al*., 1989).

The term "probe" according to the present invention refers to a single-stranded oligonucleotide which is designed to specifically hybridize to the target polynucleic acids. According to the invention those probes can be as long as the complete us-p34 sequences of the Mycobacterium p34 gene or the complete rRNA operon (*rrn*) of Pseudomonas. Some examples of such sequences are provided in the figures and in the nucleic acids having a sequence as represented in SEQ ID NOs 57 to 74 for Mycobacterium and as represented in SEQ ID NOs 76 to 82 for Pseudomonas. Also preferred according to the invention are probes having a sequence which is part of the us-p34 upstream sequences or part of the rRNA operon (*rrn*), and which are about 5 to 50 nucleotides long, more preferably at least 5, 10, 15, 20, 25, 30, 35, 40 or 45 nucleotides long. Particularly preferred lengths of probes include 10, 11, 12, 13, 14, 15, 16, 17, 18, 19, 20, 21, 22, 23, 24 or 25 nucleotides. The nucleotides as used in the present invention may be ribonucleotides, deoxyribonucleotides and modified nucleotides such as inosine or nucleotides containing modified groups which do not essentially alter their hybridization characteristics.

Probes may be labelled with a label of choice (e.g. biotin, fluoresceine, radioactive labels) according to methods well known in the art.

The term "primer" according to the present invention refers to a single stranded oligonucleotide sequence capable of acting as a point of initiation for synthesis of a primer extension product which is complementary to the nucleic acid strand to be copied. The length and the sequence of the primer must be such that they allow to prime the synthesis of the extension products. Specific length and sequence will depend on the complexity of the required DNA or RNA targets, as well as on the conditions at which the primer is used, such as temperature and ionic strength. Preferably the primer is about 5-50 nucleotides long. Also preferred according to the invention are primers having a sequence which is part of the us-p34 upstream sequences or part of the rRNA operon of about 5 to 50 nucleotides long, more preferably at least 5, 10, 15, 20, 25, 30, 35, 40 or 45 nucleotides long. Particularly preferred lengths of probes include 10, 11, 12, 13, 14, 15, 16, 17, 18, 19, 20, 21, 22, 23, 24 or 25 nucleotides. It is to be understood that the primers of the present invention may be used as probes and *vice versa,* provided that the experimental conditions are adapted.

The expression "suitable primer pair" in this invention refers to a pair of primers allowing specific amplification of a us-p34 or *rrn* target polynucleic acid fragment as defined above. Said amplification can be species-pecific or not, dependending on the use of universal or species-specific primers as indicated in the description.

The term "target region" of a probe or a primer according to the present invention is a sequence within the polynucleic acids to be detected to which the probe or the primer is completely complementary or partially complementary (i.e. with some degree of mismatch). It is to be understood that the complement of said target sequence is also a suitable target sequence in some cases.

"Specific hybridization" of a probe to a target region of a polynucleic acid means that said probe forms a duplex with part of this region or with the entire region under the experimental conditions used, and that under those conditions said probe does not form a duplex with other regions of the polynucleic acids present in the sample to be analysed.

"Specific hybridization" of a primer to a target region of a polynucleic acid means that, during the amplification step, said primer forms a duplex with part of this region or with the entire region under the experimental conditions used, and that under those conditions said primer does not form a duplex with other regions of the polynucleic acids present in the sample to be analysed. It is to be understood that "duplex" as used hereby, means a duplex that will lead to specific amplification.

The fact that amplification primers do not have to match exactly with the corresponding target sequence in the template to warrant proper amplification is amply documented in the literature. However, when the primers are not completely complementary to their target sequence, it should be taken into account that the amplified fragments will have the sequence of the primers and not of the target sequence. Primers may be labelled with a label of choice (e.g. biotin). The amplification method used can be either polymerase chain reaction (PCR), ligase chain reaction (LCR), nucleic acid sequence-based amplification (NASBA), transcription-based amplification system (TAS), strand displacement amplification (SDA) or amplification by means of Qβ replicase or any other suitable method to amplify nucleic acid molecules known in the art.

Probes and primer sequences are represented throughout the specification as single stranded DNA oligonucleotides from the 5' to the 3' end. It is obvious to the man skilled in the art that any of the below-specified probes can be used as such, or in their complementary form, or in their RNA form (wherein T is replaced by U).

The probes, primer and other nucleic acids according to the invention can be prepared by cloning of recombinant plasmids containing inserts including the corresponding nucleotide sequences, if need be by excision of the latter from the cloned plasmids by use of the adequate nucleases and recovering them, e.g. by fractionation according to molecular weight. The probes and primers according to the present invention can also be synthesized chemically, for instance by the conventional phospho-triester method.

The oligonucleotides used as primers or probes may also comprise nucleotide analogues such as phosphorothiates, alkylphosphorothiates or peptide nucleic acids or may contain intercalating agents. As most other variations or modifications introduced into the original DNA sequences of the invention these variations will necessitate adaptations with respect to the conditions under which the oligonucleotide should be used to obtain the required specificity and sensitivity. However the eventual results of hybridization will be essentially the same as those obtained with the unmodified oligonucleotides. The introduction of these modifications may be advantageous in order to positively influence characteristics such as hybridization kinetics, reversibility of the hybrid-formation, biological stability of the oligonucleotide molecules, etc.

The term "solid support" can refer to any substrate to which an oligonucleotide probe can be coupled, provided that it retains its hybridization characteristics and provided that the background level of hybridization remains low. Usually the solid supportwill be a microtiter plate, a membrane (e.g. nylon or nitrocellulose), a microsphere (bead), an array (micro-array) or a chip (biochip). The nature and geometry of the solid support will depend upon a variety of factors, including, among others, the type of array (e.g., one-dimensional, two-dimensional or three-dimensional) and the mode of attachment (e.g., covalent or noncovalent). Generally, the support according to the present invention may be composed of any material which will permit immobilization of the ligand, e.g. polynucleotide, and which will not melt or otherwise substantially degrade under the conditions used to hybridize and/or denature nucleic acids. Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin, haptens or proteins. In addition, where covalent immobilization is contemplated, the substrate should be polyfunctional or be capable of being polyfunctionalized or activated with reactive groups capable of forming a covalent bond with the ligand to be immobilized. Functional groups which may be present on the surface and used for linking may include carboxylic acids, aldehydes, amino groups, cyano groups, ethylenic groups, hydroxyl groups, inercapto groups and the like.

A number of materials suitable for use as supports in the instant invention have been described in the art. Exemplary suitable materials include, for example, acrylic, styrene-methyl methacrylate copolymers, ethylene/acrylic acid, acrylonitrile-butadienestyrene (ABS), ABS/polycarbonate, ABS/polysulfone, ABS/polyvinyl chloride, ethylene propylene, ethylene vinyl acetate (EVA), nitrocellulose, nylons (including nylon 6, nylon 6/6, nylon 6/6-6, nylon 6/9, nylon 6/10, nylon 6/12, nylon 11 and nylon 12), polycarylonitrile (PAN), polyacrylate, polycarbonate, polybutylene terephthalate (PBT), polyethylene terephthalate (PET), polyethylene (including low density, linear low density, high density, cross-linked and ultra-high molecular weight grades), polypropylene homopolymer, polypropylene copolymers, polystyrene (including general purpose and high impact grades), polytetrafluoroethylene (PTFE), fluorinated ethylene-propylene (FEP), ethylenetetrafluoroethylene (ETFE), perfluoroalkoxyethylene (PFA), polyvinyl fluoride (PVF), polyvinylidene fluoride (PVDF), polychlorotrifluoroethylene (PCTFE), polyethylenechlorotrifluoroethylene (ECTFE), polyvinyl alcohol (PVA), silicon styreneacrylonitrile (SAN), styrene maleic anhydride (SMA), and glass.

Other exemplary suitable materials for use as supports in the present invention include any type of porous "flow-through" supports known in the art.

The support may be in the form of beads, particles, sheets, or membranes and may be permeable or impermeable, depending on the type of array. For example, for linear or three-dimensional arrays the support may consist of bead or particles (such as conventional solid phase synthesis supports), fibers (such as glass wool or other glass or plastic fibers), glass or plastic capillary tubes, or metal oxide membranes. For two-dimensional arrays, the support may be in the form of plastic or glass sheets in which at least one surface is substantially flat, but it may be desirable to separate predefined regions physically for adherence of molecules, for example, wells, raised regions, etched trenches, or the like. The solid support may be planar or have simple or complex shape. The surface to which the molecule is adhered may be an external surface or an internal surface of the solid support. Particularly where the surface is porous, the molecule is likely to be attached to an internal surface. Where the solid surface is porous, various pore sizes may be employed depending upon the nature of the system.

A "predefined region" is a localized area on a support which is, was, or is intended to be used for the attachment, directly or by synthesis, of a ligand molecule. The predefined region may have any convenient shape, e.g., circular, rectangular, elliptical, wedge-shaped, etc. A predefined region and, therefore, the area upon which each distinct ligand molecule is attached, is preferably smaller than about 1 cm² or less than 1 mm². Usually, the regions have an area of less than 10.000 µm², or more usually less than 100 µm² and may be less than 10µm². Within these predefined regions, the ligand molecule therein attached is in a substantially pure form.

With "substantially pure" is meant exhibiting characteristics that distinguishes it from other predefined regions. Typically, purity may be measured in terms of biological activity or function as a result of uniform sequence. Such characteristics will typically be measured by way of binding with a selected receptor. Usually, the region is sufficiently pure such that the predominant species in the predefined region is the desired species.

"Spatially arranged to form distinct arrays" means that the predefined regions on the solid support, onto which the ligand molecules attach, are laid out in precise patterns, such as rows of dots, or rows of squares, or lines.

"Micro-array" as used in the present specification refers to a solid surface or support, with a matrix of ligand molecules arrayed at specific positions.

"Attached to" or "adhered to" a solid support denotes that said ligand molecules are directly or indirectly fixed to the solid substrate and that more than 95% of the ligand molecules remain associated with the solid support at least until all the manipulations are completed and the level of binding is assessed.

"Immobilized on the surface of a solid support" as used herein refers to the attachment or adherence of one or more molecules to the surface of a solid support including attachment or adherence to the inner surface of a porous solid support.

The term "label" as used in this specification refers to a molecule to aid in detection and quantification and denotes a molecule that can be detected either visually (e.g., because it has color, or generates a colored product, or emits fluorescence) or by use of a detector that detects properties of the reporter molecule (e.g., radioactivity, magnetic field, etc.). The nature of the label may be isotopic (³²P, ³⁵S, etc.) or non-isotopic (biotin, digoxigenin, etc.). Non-limiting examples of labels include radiolabels, fluorophores, chemiluminescence, biotin, streptavidin, digoxigenin, anti-digoxigenin, sugars, lectins, antigens, and enzyme conjugates. Labels may be proteins that may be used as direct or indirect labels, i.e., green fluorescent protein (GFP), blue fluorescent protein (BFP), yellow fluorescent protein (YFP), red fluorescent protein (RFP), luciferase, P-galactosidase, all commercially available, i.e.,Clontech, Inc. Molecules that change their fluorescence or activity upon a change in binding, may also be useful.

The term "labelled" also refers to the use of labelled nucleic acids. Labelling may be carried out by the use of labelled nucleotides incorporated during the polymerase step of the amplification or labelled primers, or by any other method known to the person skilled in the art.

The term "sample" refers any sample of biological or non-biological origin. The term "biological sample or sample" refers to for instance naso-pharyngeal aspirates, throat or nasopharyngeal swabs, nasopharyngeal washes or tracheal aspirates or other respiratory tract sample comprising DNA or RNA. Samples of non-biological origin can be from any organic or inorganic material which can be suspected to contain microbial material.

For designing probes with desired characteristics, the following useful guidelines known to the person skilled in the art can be applied.

Because the extent and specificity of hybridization reactions such as those described herein are affected by a number of factors, manipulation of one or more of those factors will determine the exact sensitivity and specificity of a particular probe, whether perfectly complementary to its target or not. The importance and effect of various assay conditions are explained further herein.

The stability of the [probe : target] nucleic acid hybrid should be chosen to be compatible with the assay conditions. This may be accomplished by avoiding long AT-rich sequences, by terminating the hybrids with G:C base pairs, and by designing the probe with an appropriate Tm. The beginning and end points of the probe should be chosen so that the length and %GC result in a Tm about 2-10°C higher than the temperature at which the final assay will be performed. The base composition of the probe is significant because G-C base pairs exhibit greater thermal stability as compared to A-T base pairs due to additional hydrogen bonding. Thus, hybridization involving complementary nucleic acids of higher G-C content will be more stable at higher temperatures.

Conditions such as ionic strength and incubation temperature under which a probe will be used should also be taken into account when designing a probe. It is known that the degree of hybridization will increase as the ionic strength of the reaction mixture increases, and that the thermal stability of the hybrids will increase with increasing ionic strength. On the other hand, chemical reagents, such as formamide, urea, DMSO and alcohols, which disrupt hydrogen bonds, will increase the stringency of hybridization. Destabilization of the hydrogen bonds by such reagents can greatly reduce the Tm. In general, optimal hybridization for synthetic oligonucleotide probes of about 10-50 bases in length occurs approximately 5°C below the melting temperature for a given duplex. Incubation at temperatures below the optimum may allow mismatched base sequences to hybridize and can therefore result in reduced specificity.

It is desirable to have probes which hybridize only under conditions of high stringency. Under high stringency conditions only highly complementary nucleic acid hybrids will form; hybrids without a sufficient degree of complementarity will not form. Accordingly, the stringency of the assay conditions determines the amount of complementarity needed between two nucleic acid strands forming a hybrid. The degree of stringency is chosen such as to maximize the difference in stability between the hybrid formed with the target and the non-target nucleic acid.

Regions in the target DNA or RNA which are known to form strong internal structures inhibitory to hybridization are less preferred. Likewise, probes with extensive self-complementarity should be avoided. As explained above, hybridization is the association of two single strands of complementary nucleic acids to form a hydrogen bonded double strand. It is implicit that if one of the two strands is wholly or partially involved in a hybrid that it will be less able to participate in formation of a new hybrid. There can be intramolecular and intermolecular hybrids formed within the molecules of one type of probe if there is sufficient self complementarity. Such structures can be avoided through careful probe design. By designing a probe so that a substantial portion of the sequence of interest is single stranded, the rate and extent of hybridization may be greatly increased. Computer programs are available to search for this type of interaction. However, in certain instances, it may not be possible to avoid this type of interaction.

Standard hybridization and wash conditions are disclosed in the Materials & Methods section of the Examples. Other conditions are for instance 3X SSC (Sodium Saline Citrate), 20% deionized FA (Formamide) at 50°C. Other solutions (SSPE (Sodium saline phosphate EDTA), TMAC (Tetramethyl ammonium Chloride), etc.) and temperatures can also be used provided that the specificity and sensitivity of the probes is maintained. When needed, slight modifications of the probes in length or in sequence have to be carried out to maintain the specificity and sensitivity required under the given circumstances.

The term "hybridization buffer" means a buffer allowing a hybridization reaction between the probes and the polynucleic acids present in the sample, or the amplified products, under the appropriate stringency conditions.

The term "wash solution" means a solution enabling washing of the hybrids formed under the appropriate stringency conditions.

The invention, now being generally described, will be more readily understood by reference to the following examples and figures, which are included merely for the purposes of illustration of certain aspects and embodiments of the present invention and are in no way to be construed as limiting the present invention.

### FIGURE LEGENDS

**Figure 1.** Multiple nucleotide sequences alignment of *M. bovis* (MB), *M. tuberculosis* (MT), *M. avium* subsp. *paratuberculosis* (MPT) and *M. avium* ss (MA) us-p34 genes. Gaps between sequences are indicated by dots (.). Vertical bar (I) indicates identity across sequences. The start codon (ATG) for the p34 ORF is in bold. Primer sequences are indicated by shaded boxes. The arrows indicate point mutations between *M. tuberculosis* and *M. bovis* and *M. avium* subsp. *paratuberculosis* and *M. avium* ss.

**Figure 2.** Amplifications of us-p34 regions with primers U1, U2, U3, U4 and U9.

**Figure 3.** New us-p34 sequences (5' to 3'). Primers used to obtain the sequence (either U2-U1 ; U3-U1 ; U4-U1 ; U2-U9 ; U3-U9 or U4-U9) and the amplicon size are as indicated. Additional internal primers (U1, U2, U3) have been shown within the sequences. Sequence variations (point mutations) found in the same species (for instance *M. ulcerans)* are also indicated when known.

**Figure 4.** U1-U4 consensus amplification of us-p34 regions of different mycobacterial species.

**Figure 5.** Specific and non-specific hybridization.

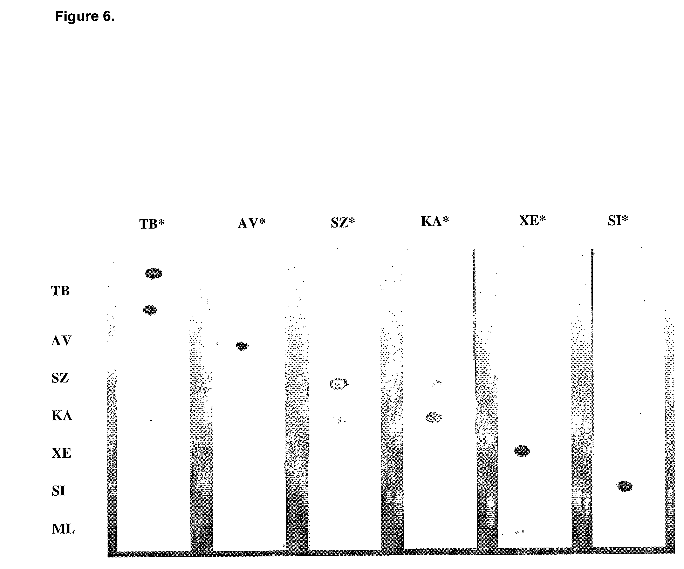

**Figure 6.** Differential reverse hybridization of mycobacteria target amplicons on a nylon membrane disclosing species-specific mycobacteria probes.
a) Unlabeled amplified DNA segments specific for various mycobacteria species were first transferred on nylon membrane (*M. tuberculosis* (TB), *M. avium* (AV), *M. szulgai* (SZ), *M. kansasii* (KA), *M. xenopi* (XE), *M. simiae* (SI) and *M. malmoense* (ML)).
b) Digoxigenin-labeled amplicons from *M. tuberculosis* (TB*), *M. avium* (AV*), *M. szulgai* (SZ*), *M. kansasii* (KA*), *M. xenopi* (XE*) and *M. simiae* (SI*) were hybridized on the nylon membrane. Specific differential hybridization is obtained.

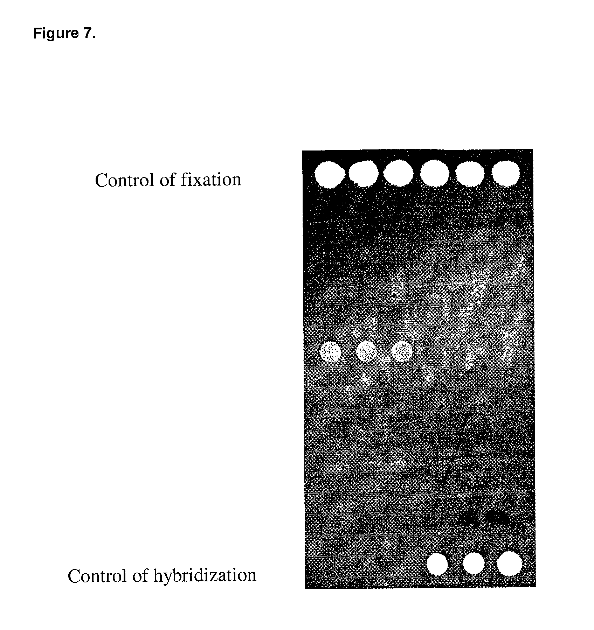

**Figure 7.** Example of biochips detecting specifically *M. gordonae*

**Figure 8.** Alignment of several Mycobacterial us-p34 sequences. Parameters used for sequence alignment: gap creation penalty = 5; gap extension penalty = 1.

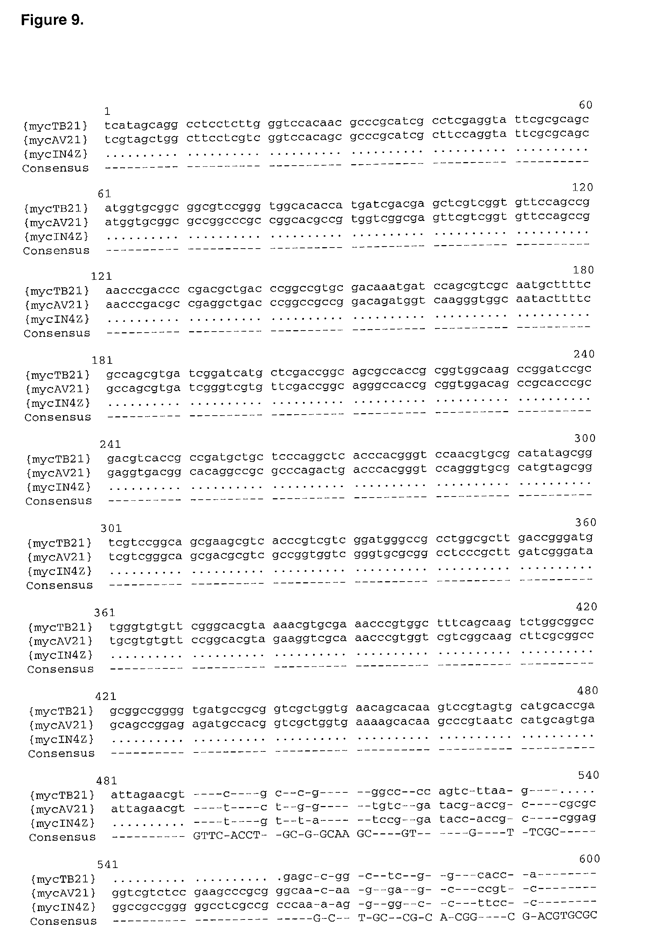

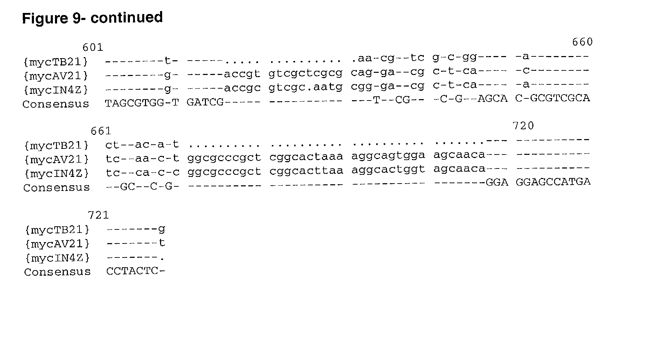

**Figure 9.** Alignment of three Mycobacterial us-p34 sequences (M. tuberculosis, M. avium and M. intracellulare). Parameters used for sequence alignment: gap creation penalty = 5; gap extension penalty = 1.

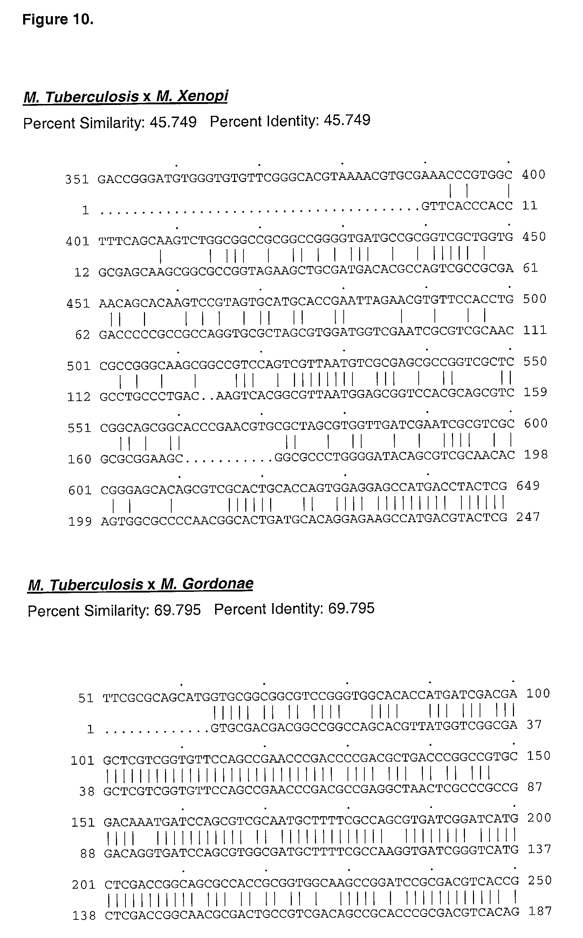

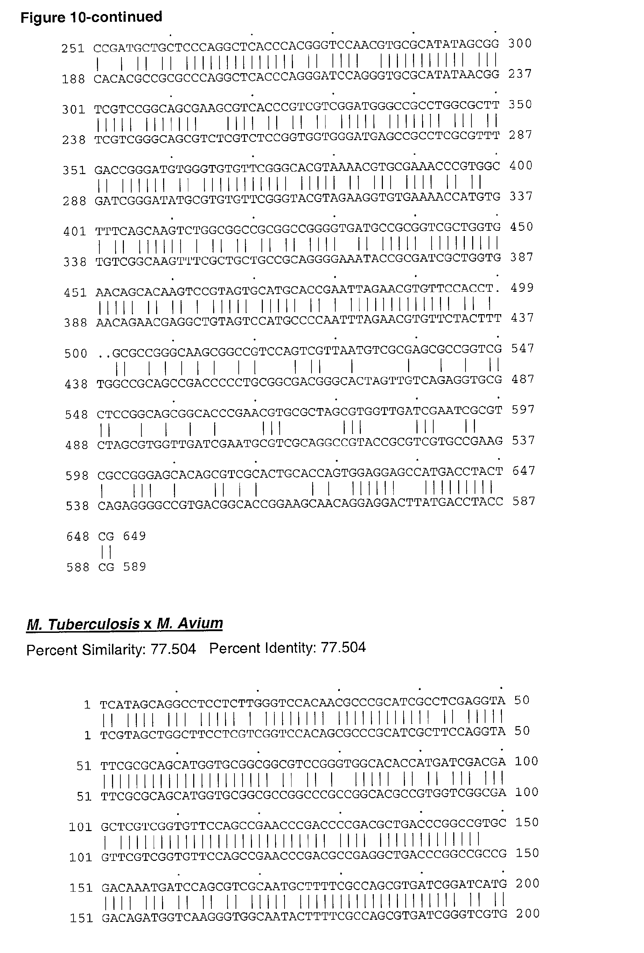

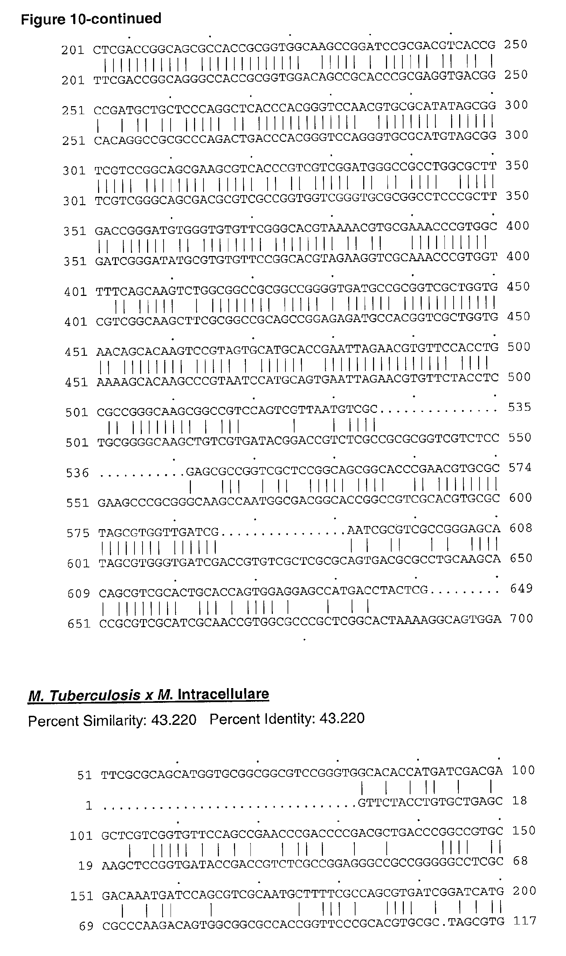

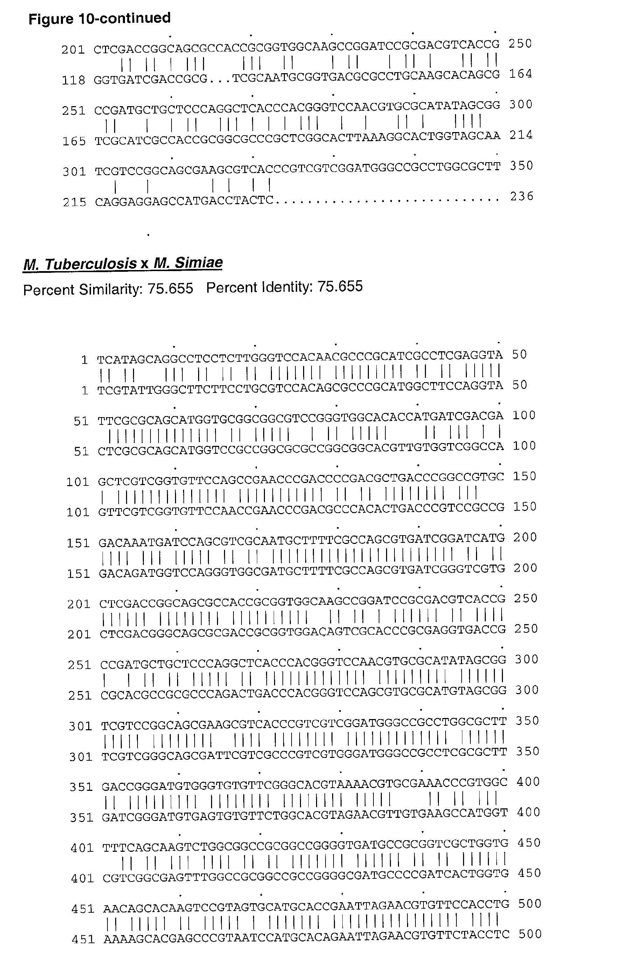

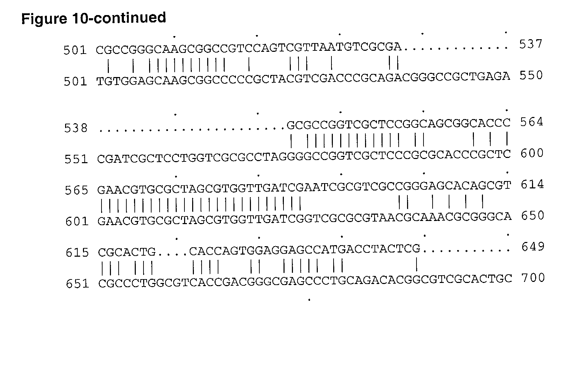

**Figure 10.** Alignment of several pairs of Mycobacterial us-p34 sequences. Parameters used for sequence alignment: gap creation penalty = 50; gap extension penalty = 3.

**Figure 11. A.** Organisation of the rrn operon of *Pseudomonas aeruginosa* (Ps.ae), *Burkholderia cepacia* (Bu.ce) and *Stenotrophomonas maltophilia* (St.ma).

**Figure 11. B.** Comparison of the sequences flanking the regions encoding 16S and 23S RNA from *Pseudomonas aeruginosa* (Ps.ae) (SEQ ID NO 76), *Burkholderia cepacia* (Bu.ce) (SEQ ID NO 77). CNS: consensus.

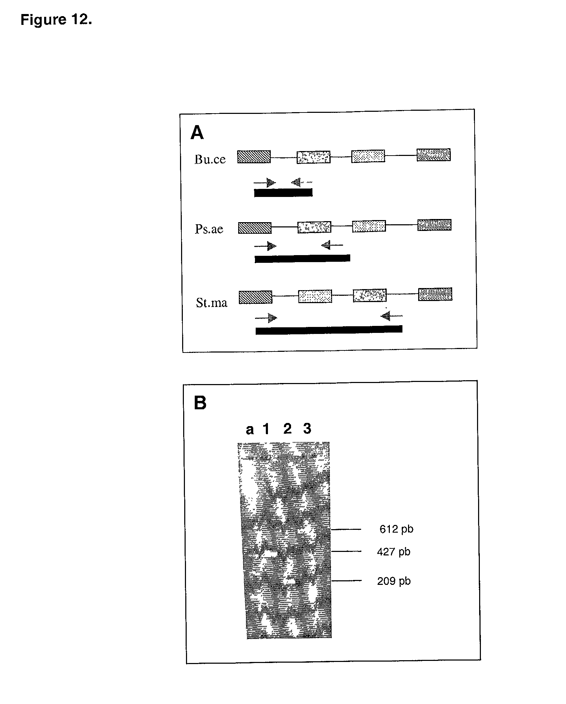

**Figure 12.** Discrimination by multiplex PCR.
The DNA from *Ps. aeruginosa* (1), *Bu. cepacia* (2) or *St. maltophilia* (3) was amplified using a conserved primer and a set of specific primers. **A.** Multiplex PCR theoretical scheme. **B**. Agarose Gel allowing the visualization of the amplified fragments of different sizes. In a, molecular weight marker (scale 123 bp).

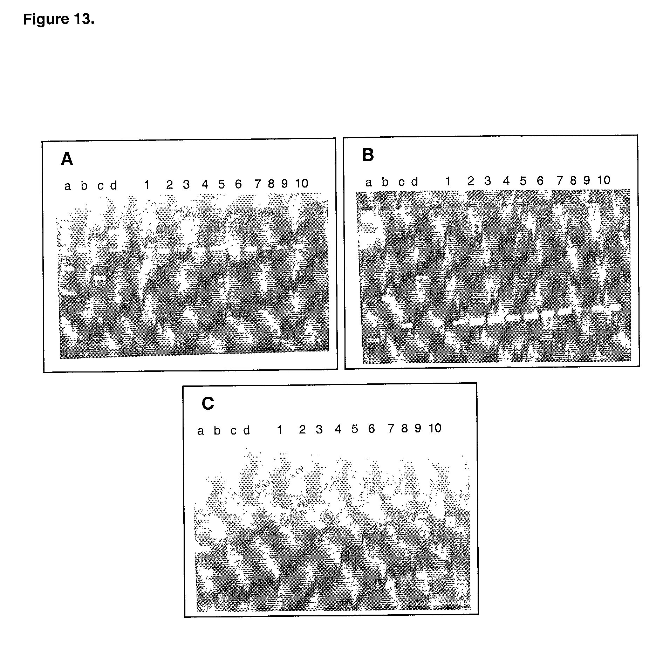

**Figure 13.** Multiplex PCR sensibility validation.
The DNA from clinical strains (1 to 10) and from *Ps. aeruginosa* **(A),** *Bu. cepacia* **(B)** or *St. maltophilia* **(C)** were amplified using PsMulti, Paer, Bcep and Sma1 primers. The amplified fragments (20 µl) are visualized after agarose gel (2 %) migration.
In a, molecular weight marker (scale 123 bp). In b, c and d are control fragments amplified from *Ps. aeruginosa, Bu. cepacia* and *St. maltophilia* respectively.

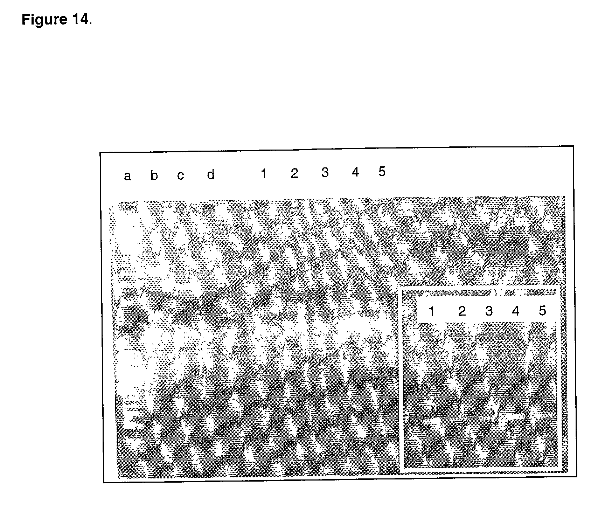

**Figure 14.** Multiplex PCR specificity validation.
In a, molecular weight marker (scale 123 bp). In b, c and d are control fragments amplified from *Ps. aeruginosa, Bu. cepacia* and *St. maltophilia* respectively.
The DNA from clinical strains of *Ps. putida* (1), *Ps. fluorescens* (2) or *Ps. alcaligenes* (3), *Ps. pseudoalcaligenes* (4) and *Ps. stuzeri* (5) were amplified using PsMulti, Paer, Bcep and Sma1 primers. The absence of amplification products (20 µl) was visualized after agarose gel (2 %) migration.
**Inset:** The DNA from clinical strains of *Ps. putida* (1), *Ps. fluorescens* (2) or *Ps. alcaligenes* (3), *Ps. pseudoalcaligenes* (4) and *Ps. stuzeri (5)* was amplified using ProUni1 and ProUni2 primers (universal primers amplifying part of the 16S RNA gene). The amplification products (20 µl) were visualized after agarose gel (2 %) migration.

**Figure 15.** Reverse hybridization for the discrimination between *Ps. aeruginosa, Bu. cepacia* and *St. maltophilia*
2 and 5 µl of probes corresponding to the three species where sequentially immobilized on Hybond N+ nylon membrane (from top to bottow, 2 µl and 5 µl of the probe specific for *Ps. aeruginosa,* 2 µl and 5 µl of the probe corresponding to *Bu. cepacia* and 2 µl and 5 µl of probe specific for *St. maltophilia).* Biotinylated amplicons were produced from the genomic DNA of each species and were hybridized with a membrane containing the different probes. The hybrids were colorimetrically detected.

**Figure 16.** Visualization of the second *rrn* operon from *Ps. putida*
**A.** Amplification products (20 µl) from DNA of *Ps. aeruginosa (1), Bu. cepacia (2), St. maltophilia (3), Ps. putida (4), Ps. alcaligenes* (5), *Ps. pseudoalcaligenes (6), Ps. fluorescens* (7) using PsSeq1 and PsSeq2 primers, visualized on agarose gel (2 %).
**B.** The DNA was transferred from the gel A to a nylon membrane, and hybridized with a biotiylated probe (DNA from *Ps. aeruginosa* amplified with PsSeq1 and psSep2b primers). The hybrids were revealed by colorimetry (streptavidine, DAB).
PM = molecular weight marker

**Figure 17.** Alignment of the two *rrn* operon sequences from *Ps. putida.* First (top) sequence is SEQ ID NO 78, second (bottom) sequence is SEQ ID NO 79.
The sequence of the shortest operon is boxed, the double-underlined sequence correspond to the missing region of the shortest operon.

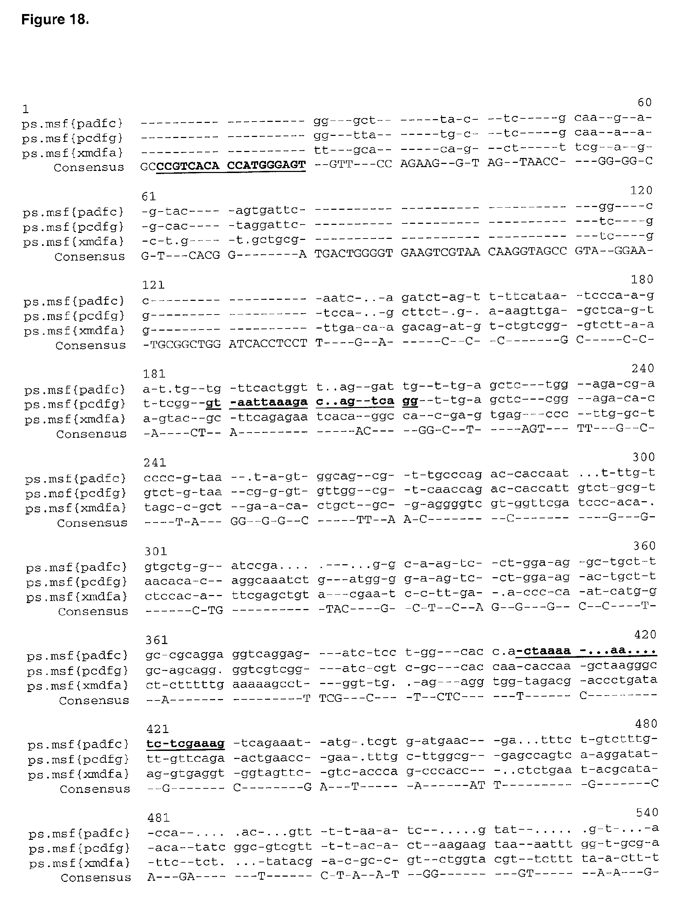

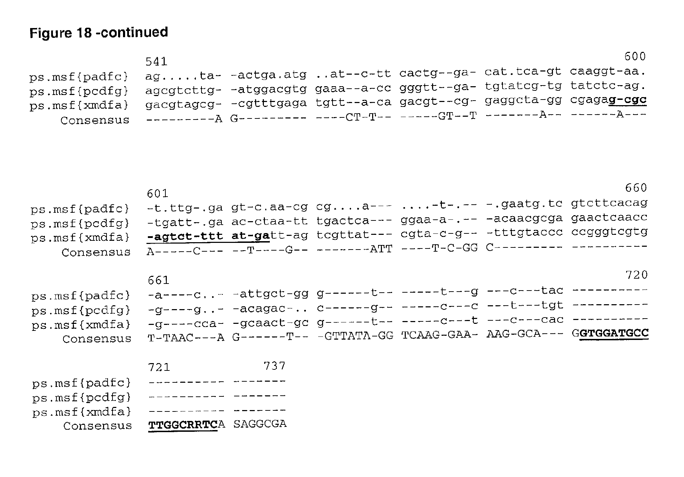

**Figure 18.** Alignment and consensus sequence between *Pseudomonas aeruginosa* (ps.msf{padfc}), *Burkholderia cepacia* (ps.msf{pcdfg} and *Stenotrophomonas maltophilia* (ps.msf{xmdfa}). The underlined sequences correspond to the regions where the primers for amplification were chosen.

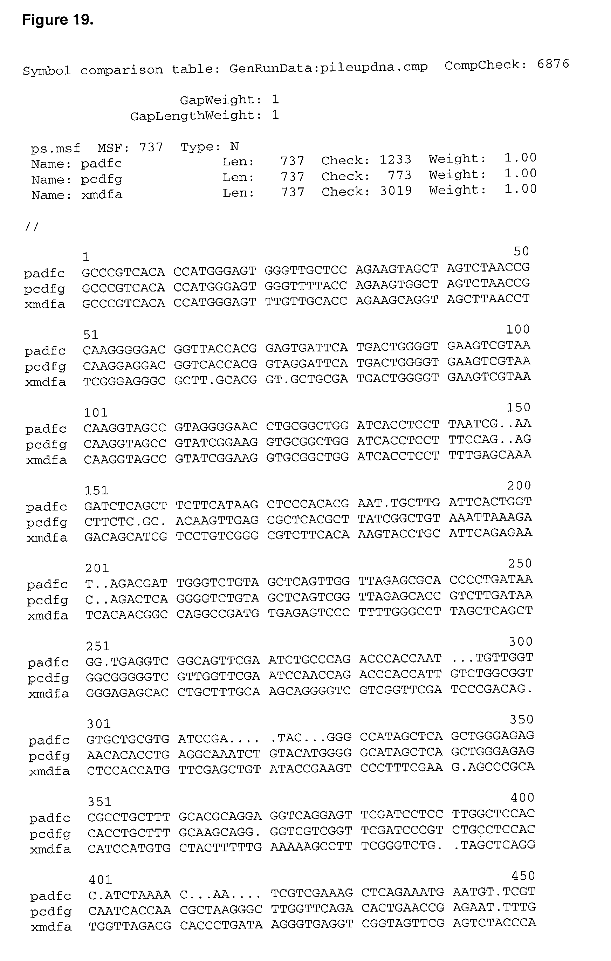

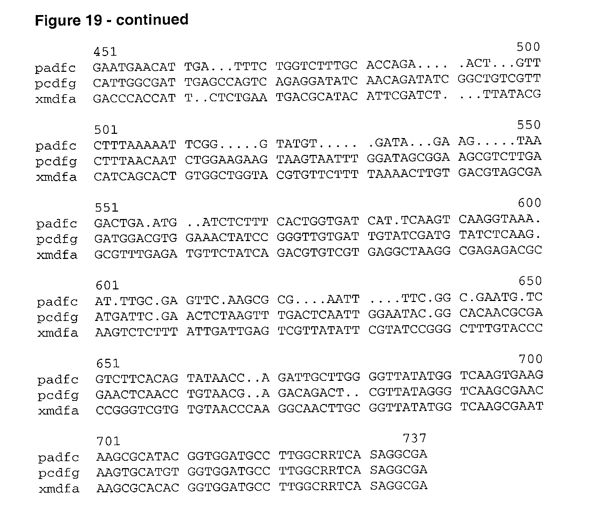

**Figure 19.** Alignment without consensus sequence between *Pseudomonas aeruginosa* (ps.msf{padfc}) (SEQ ID NO 80), *Burkholderia cepacia* (ps.msf{pcdfg} (SEQ ID NO 81) and *Stenotrophomonas maltophilia* (ps.msf{xmdfa}) (SEQ ID NO 82).

### EXAMPLES

### Example 1: Differential diagnosis of Mycobacterial species

### 1. Preparation of mycobacterial DNA.

a) *short protocol* (mycobacteria obtained from the Pasteur Institute) :
   DNA has been obtained by boiling the samples in order to put the mycobacterial DNA into solution.
b) *long protocol* (mycobacteria obtained from the Institute of Tropical Medicine):
   Mycobacteria (10 mg [wet weight]) were suspended in 200 µl of lysis solution (0.1 M NaOH, 1 M NaCI, and 5% sodium dodecyl sulfate [SDS]) and heated (100°C) for 20 min. The suspension was then cooled, neutralized with 3 volumes of 0.1 M Tris-HCI (pH 7.4) buffer, and centrifuged (5,000 × g, 5 min). Supernatants were extracted with phenolchloroform, and DNA was precipitated with ethanol, collected by centrifugation, dissolved in 50 µl of H₂O, and stored at 20°C.

### 2. PCR amplifications.

For amplification, an aliquot (10 µl) of the DNA samples was added to 90 µl of PCR mixture consisting of 10 mM Tris-HCI (pH 8.8), 1.5 mM MgCl₂, 50 mM KCI, 0.1% Triton X-100, 0.25 mM (each) deoxynucleoside triphosphates, 10 pmol of each primer (Tables 1 and 2) and 0.625 U of DyNAzyme DNA polymerase (Finnzymes Inc., Espoo, Finland). After an initial denaturation step (3 min at 96°C), 30 cycles of amplification were performed as follows: denaturation at 96°C for 30 s, annealing at 58°C for 45 s, and DNA extension at 72°C for 30 s, with an increment of 1 s per cycle for the denaturation and extension segments. A final extension was performed at 72°C for 15 min. Amplifications were carried out in a DNA 2400 thermocycler (Perkin-Elmer Applied Biosystems, Foster City, Calif.). PCR products were checked by loading 5 µl on a 2% (wt/vol) agarose gel. Electrophoresis is performed in 0.1 M Tris HCI (pH 8.6), 80 mM boric acid, 1 mM EDTA buffer containing 0.5 µg of ethidium bromide per ml. DNA fragments are visualized on a UV transilluminator at 300 nm. PCR products were cloned using the TOPO XL PCR cloning kit (Invitrogen, Carlsbad, Calif.), according to the manufacturer's protocol. The clones were further sequenced with the Taq Dye Deoxy Terminator Cycle sequencing kit and an ABI 377 DNA sequencer (Perkin-Elmer Applied Biosystems).

### 3. DNA sequence analysis.

Nucleotide sequence analyses were performed with the Genetics Computer Group software obtained from the University of Wisconsin, through the use of the Belgian EMBnet Node facility. Sequences were aligned by the Pileup program and paired comparisons were realised with the Bestfit program.

### 4. Reverse hybridization analysis.

Mycobacterial species-specific capture probes were produced by amplification of the us-P34 cloned-DNA from the reference strains with primers U1 and U4. Amplified DNA fragments were sequentially transferred onto nylon membranes (Hybond-N+; Amersham, Little Chalfont, United Kingdom) according to the Southern blot method. After 2 h of prehybridization, membranes were hybridized with us-p34 digoxigenin-labeled target probes, obtained by PCR amplification of reference or clinical mycobacterial genomic DNA with primers U1 and U4 (Table 1), in the presence of DIG-11-dUTP. Hybridization of heat denatured target probes (5 min at 95 °C) was performed at 50 °C for 4 h in 2x SSC (1x SSC is 0.15M NaCI and 0.015 M sodium citrate), 1% blocking reagent, 0.1% SDS, 0.1% N-Lauroylsarcosine, 5 mg/ml of salmon sperm DNA and 5% formamide. Filters were then washed twice with 2× SSC (1× SSC is 0.15 M NaCI plus 0.015 M sodium citrate)-0.1% SDS at 37°C for 5 min, and twice with 0.2× SSC-0.1% SDS at 50°C for 5 min. Hybridized digoxigenin-labeled DNA fragments were detected through alkaline-phosphatase-labeled anti-DIG Fab fragments. Colorimetric detection was performed with Nitro Blue Tetrazolium Chlorure and 5,3-bromo-4-indolylphosphate (BCIP) (Boerhinger Mannheim) according to the manufacturer's instructions.

### 5. Analysis on "Mycobacteria Biochips".

### Design of the biochips.

Mycobacterium micro-arrays have been developed by AAT (Namur, Belgium). This array is a glass slide bearing on its surface different mycobacterial species-specific capture nucleotide sequences (Table 3). Fixation, positive and negative hybridization controls capture probes are also included in the array and each capture probe of the array is composed of four replicates.

### Production of biotinylated amplicons.

Amplification of the mycobacterial us-p34 sequences is performed in a final volume of 50 µl containing 2.5 mM MgCl₂, 75 mM Tris-HCl, pH 9.0, 50 mM KCI, 20 mM (NH₄)₂SO₄, 0.5 µM of primers U4 and U5, 200 µM of dATP, 200 µM of dCTP, 200 µM of dGTP, 150 µM of dTTP, 50 µM of biotin-16-dUTP, 0.5 U of uracil-DNA-Glycosylase (Boehringer Mannheim, Germany), 1.25 U of *Taq* DNA polymerase (Biotools, Madrid, Spain) and 10 µl of DNA template. The reagents are first incubated at 94°C for 5 min and then cycled 40 times in a DNA 9600 thermocycler (Perkin Elmer, Foster City, CA, USA) using the following temperatures and cycle times: 94°C for 30 s, 49°C for 45 s, 72°C for 30 s. A final extension step of 10 min at 72°C is performed. PCR products are directly used or stored at -20°C.

### Hybridization and colorimetric detection.

The procedure for detecting the PCR products is carried out according to the manufacturer's instructions (AAT, Namur, Belgium) as follows: 5 µl of PCR product and 5 µl of the positive control, provided in the kit, are denatured with fresh NaOH 0.05N for 5 min at room temperature. This solution is then mixed with 35 µl of hybridization solution and loaded on the array framed by a hybridization chamber (Biozym, Landgraaf, The Netherlands). The chamber is closed with a plastic coverslip and hybridization is carried out for 30 min at 53°C. Slides are washed four times 1 min with washing buffer. The glass samples are then incubated 45 min at room temperature with 800 µl of streptavidin-conjugate 1000 times diluted in blocking buffer. After 5 washes, the slides are incubated 3 times 10 min with 800 µl of revelation mixture, then rinsed with water, dried and imaged using a colorimetric microarray reader (AAT, Namur, Belgium). While a quick visual inspection already yields most of the images relevant informations, the image obtained is analysed by an algorythm allowing the quantification of the spots intensities values and by a pattern recognition algorithm.

### 6. Cloning and sequencing of the upstream-P34 region of mycobacterial species.

Comparison of homologous p34 genes and its regulatory sequences (upstream p34 or us-p34) from *M. tuberculosis* (GenBank accession no. Z79700) and *M. avium* subsp. *paratuberculosis* (GenBank accession no. X68102) showed the presence of deletions in the *M. tuberculosis* region upstream of the p34 protein start codon (Figures 1 and 2). To confirm and extend these findings, the us-p34 sequence of *M. bovis* BCG and *M. avium* D4 were amplified and sequenced. Multiple sequences alignment of this region revealed interspecies polymorphisms specific for both tuberculous (*M. tuberculosis* and *M. bovis* Bacile de Calmette et Guérin [BCG]) and non-tuberculous (*M. avium* sp and *M. avium* subsp. *paratuberculosis*) mycobacteria, i.e. the us-p34 fragment was 79 bases shorter in the tuberculous species. Conversely, us-p34 appeared to be highly conserved within each group: differentiation between M. *tuberculosis* and *M. bovis* relied on a single T to C transition at position 41 of us-p34, and a single C to G transversion at position 264 of us-p34 in *M. avium* ss and *M. avium* subsp. *paratuberculosis* (Figure 1).

Based on sequence homology between the *M. tuberculosis* / *M. bovis* and *M. avium* / *M. avium* subsp. *paratuberculosis,* several oligonucleotides, U1, U2, U3, U4 and U9 (Tables 1 and 2), matching conserved sequences of the polymorphic us-p34 region, were designed to amplify the corresponding region of others reference bacteria from the mycobacterial genus, *M. szulgai, M. africanum, M. gordonae, M. gastri, M. kansasii, M. marinum, M. ulcerans, M. intracellulare, M. scrofulaceum, M. xenopi, M. malmoense and M. simiae* (Table 3). These fragments have been cloned and sequenced (Figure 3).

### 7. Development of a consensus PCR amplication of mycobacterial species.

Based on the mycobacterial sequences of us-p34, a consensus PCR assay was developed. Primers U1 and U4 have allowed to amplify the corresponding fragments, ranging from 163 bp and 298 bp in size, for *M. szulgai* (163 bp), *M. non chromogenicum* (169 bp), *M. tuberculosis, M. bovis, M. africanum* (178 pb), *M. gordonae* (182 bp), *M. gastri* (223 bp), *M. kansasii* (225 bp), *M. marinum,* and *M. ulcerans* (236 pb), *M. intracellulare, M. avium, M. paratuberculosis* (256 bp), *M. scrofulaceum* (259 bp), *M. xenopi* (265 bp), *M. leprae* (269 bp), *M. malmoense* (290 bp) and *M. simiae* (298 bp) (Figure 4; Table 4). Sequences alignments indicate both size and nucleotide sequence polymorphisms, as illustrated by some pair-wised alignments of all mycobacterial sequences with *M. tuberculosis* us-p34 region.

### 8. Validation of the consensus mycU1-mycU4 PCR on clinical isolates.

The reliability of this consensus amplification strategy was tested on a range of clinical mycobacterial isolates (Table 4). The strategy is characterized by a good sensitivity for most mycobacteria. The lower sensitivity displayed for some species is probably related rather to the DNA sample than to a lack of sensitivity of the strategy. As a matter of fact, *M. marinum-M. ulcerans* amplifications show a better sensitivity for "long protocol" DNA preparations (20/20) than for the "short protocol" ones (4/10). The quality of these "short protocol" DNA preparations have to be checked before running further conclusions. This could indeed influence further identification of potentially specific amplicons in other mycobacterial species (such as, for example, *M. phlei, M. flavescens, M. nonchromogenicum, M. chelonae)*

### 9. Development of species-specific PCR amplifications.

Based on alignment of all upstream p34 sequences between U1 and U4 (which contain most inter-species differences) or at the broadest between U9 and U2 (which also may contain specific inter-strain differences), primers which will selectively amplify one strain (two new primers or a combination of one new primer and one primer presented in Tables 1 and 2) are designed. This strategy enables the differentiation of the mycobacterial species by PCR.

### 10. Reverse hybridization strategy.

Based on homologous and non-homologous hybridization of amplicons from the same species and amplicons from two different species, respectively (Figure 5), a reverse hybridization assay was set up. In a first step, species-specific probes corresponding to each mycobacteria species, were produced by amplification of reference strains us-p34 cloned DNA with the two consensus primers U1 and U4 and were immobilized on nylon strips. DNA of species to be identified were amplified in the presence of the same primers, biotinylated at their 5' ends, hybridized with the immobilized probes and colorimetrically assayed. An assay, allowing a correct discrimination between *M. tuberculosis, M. avium, M. szulgai, M. xenopi, M. simiae and M. malmoense* is presented in Figure 6.

### 11. Development of a "Mycobacteria Biochips".

The polymorphic us-p34 sequences can also be applied on low-density microarray technology in order to develop simultaneous species-specific detection of mycobacteria. In a preliminary study, mycobacteria microarrays, bearing different mycobacterial species-specific capture probes, have been developed by AAT (Namur, Belgium). These probes were hybridized with the us-p34 biotinylated amplicons from the different mycobacterial species. Illustration of a specific detection of *M. gordonae versus M. tuberculosis, M. gastri, M.kansasii, M. intracellulare, M. leprae, M. avium, M. marinum, M. simiae, M. xenopi, M. malmoense, M. szulgai* and *M. scrofulaceum* specific capture probes is presented in Figure 7.

### Example 2: Differential diagnosis of Pseudomonas species.

Alignment of the sequences of the studied species are shown in Figures 11B, 17, 18 and 19.

The codes used for the alignments are the following:
ps.msf(padfc) = Pseudomonas aeruginosa
ps.msf(pcdfg) = Burkholderia cepacia
ps.msf(xmdfa) = Stenotrophoromonas maltophilia

The parameters used for the alignments in Figures 18 and 19 are: Plurality: 3.00 Threshold: 1 AveWeight: 1.00 AveMatch: 1.00 AvMisMatch: 0.00.

From the alignments, universal and species-specific probes and primers were designed by the present inventors and are represented in Table 6.

« Psmulti(PsSeq1) » and « PsSeq2 » are universal primers for Pseudomonas (hybridizing effectively to a consensus region determined by the alignment of the three sequences). The three other primers « Bcep », « Paer » and « Sma1 » are species-specific (c.f. Figure 18 and Figure 12A).

### Multiplex PCR:

The multiplex PCR amplifies distinctly *Pseudomonas aeruginosa, Burkholderia cepacia* and *Stenotrophomonas maltophilia.*

The amplification of the conserved region on the 16S rRNA gene was used as a control in most of the experiments. These sequences are represented in Table 7 and are known in the art.

The length of the amplified fragments (c.f. Figure 12) was as follows:

| | |
|---|---|
| PsSeq1-PsSeq2 | 726 bp |
| Bu. cepacia | 209 bp |
| Ps. aeruginosa | 427 bp |
| St. Maltophilia | 612 bp |
| ProUn1-ProUn2 | 211 bp |

### REFERENCES

1. Portaels f. Epidemiology of mycobacterial diseases. Clinics in Dermatology 1995 ;13 :207-222.
2. Raviglione MC, Snider DE, Kochi A. Global epidemiology of tuberculosis : morbidity and mortality of a wordlwide epidemic. JAMA 1995 ;273 :220-26.
3. Cobelens FG, van Deutekom H, Draayer-Jansen IW, et al. Risk of infetion with Myvccobacterium tuberculosis in travellers to areas of high tuberculosis endemicity=. Lancet 2000 ;356 :461-465.
4. Pablos-Mendez A, raviglione MC, Laszlo A, et al ; Global surveillance for antituberculosis-drug resistance, 1994-1997. New Engl J Med 1998;338 :1641-49.
5. Tsang AY and Farber ER. The primary isolation of Mycobacterium ulcerans. Am. J. Clin. Pathol. 1973 ; 59 : 688-692.
6. Portaels F, Fonteyne PA, De Beenhouwer H, de Rijk P, Guédénon A, Hayman J, Meyers WM. Variability in 3' end of 16S rRNA sequence of Mycobacterium ulcerans is related to geographic origin of isolates. J. Clin Microbiology 1996 ;34 :962-965.
7. O'Callaghan EM, Tanner MS, Boulnois GJ. Developpement of a PCR. probe test for identifying *P.aeruginosa and P.(B) cepacia.* Clin Pathol 1994 ; 47:222-226.
8. Karpati F and Jonasson J. Polymerase chain reaction for the detection of Pseudomonas aeruginosa, Stenotrophomonas maltophilia and Burkholderia cepacia in sputum of patients with cystic fibrosis. Molecular and Cellular probes, 1996, 10 :397-403.

### Table 1. Oligonucleotide primers

These consensus primers have been successfully used to amplify a wide panel of reference and clinical mycobacterial strains (see Table 4)

U1: 5'-GAGTAGGTCATGGCTCCTCC-3' (antisense) (SEQ ID NO 1)

U6: 5'-GTGCGCATATAGCGGTCGTC-3' (sense) (SEQ ID NO 2)

U4: 5'-CATGCAGCGAATTAGAACGT-3' (sense) (SEQ ID NO 3)

### Table 2. Oligonucleotide primers

U2: 5'-AACTTGACGAACTCGCCG-3' (sense) (SEQ ID NO 4)

U3: 5'-AGGTATTCGCGCAGCATG-3' (sense) (SEQ ID NO 5)

U5 : 5'-GTASGTCATRRSTYCTCC-3' (antisense) (SEQ ID NO 6)

U9: 5'-GGTGAACATTGGGCCGAA-3' (antisense) (SEQ ID NO 7)

S= G or C; R= G or A ; Y= T or C

U5 is an example of a degenerated primer that in combination with U4, as a consensus or universal primer, was used to amplify DNA fragments of various mycobacterial species. These mycobacterial species could subsequently be identified by reverse hybridization on a biochips

It is obvious that the above-mentioned nucleic acids can also be used as probes in other experiments.

### Table 3. Oligonucleotide probes and primers

### (a) Example of oligonucleotides used as capture probes that can specifically hybridize Mycobacteria strains on a biochips after DNA amplification using U4 and U5 consensus primers

For some species, several potential capture probes have been designed. Further testing may reveal advantages of some with respect to the others.

Avium 1 :	5'-CGGTCGTCTCCGAAGCCCGCG-3'(SEQ ID NO 8)

Gastrii 1 :		5'-GATCGGCAGCGGTGCCGGGG-3'(SEQ ID NO 9)

Gastrii 2 : 5'-GTATCGCGGGCGGCAAGGT-3'(SEQ ID NO 10)

Gastrii 3 : 5'-TCTGCCGATCGGCAGCGGTGCCGG-3'(SEQ ID NO 11)

Gastrii 4 :                    5'-GCCGGGGCCGGTATTCGCGGGCGG-3'(SEQ ID NO 12)

Gordonae 1 :	5'-GACGGGCACTAGTTGTCAGAGG-3'(SEQ ID NO 13)

Intracellulare 1:	5'-GGGCCGCCGGGGGCCTCGCCG-3'(SEQ ID NO 14)

Intracellulare 2 :	5'-GCCTCGCCGCCCAAGACAGTG-3'(SEQ ID NO 15)

Leprae 1: 5'-GATTTCGGCGTCCATCGGTGGT-3'(SEQ ID NO 16)

Kansasii 1: 5'-GATCGTCGGCAGTGGTGACGG-3'(SEQ ID NO 17)

Kansasii 2 : 5'-TCGTCGGCAGTGGTGAC-3'(SEQ ID NO 18)

Kansasii 3 : 5'-ATCCGCCGATCGTCGGCAGTGGTGACG-3'(SEQ ID NO 19)

Malmoense 1 :          5'-GACCCACAACACTGGTCGGCG-3'(SEQ ID NO 20)

Marinum 1 : 5'-CGGAGGTGATGGCGCTGGTCG-3'(SEQ ID NO 21)

Scrofulaceum 1 :	5'-CGGCGGCACGGATCGGCGTC-3'(SEQ ID NO 22)

Simiae 1: 5'-ATCGCTCCTGGTCGCGCCTA-3' (SEQ ID NO 23)

Szulgai 1 : 5'-CCCGGCGCGACCAGCAGAACG-3'(SEQ ID NO 24)

Tuberculosis 1:	5'-GCCGTCCAGTCGTTAATGTCGC-3'(SEQ ID NO 25)

Xenopi 1: 5'-CGGTAGAAGCTGCGATGACACG-3'(SEQ ID NO 26)

It is obvious that the above-mentioned nucleic acids can also be used as primers in other experiments.

### (b) Example of oligonucleotides used as capture probes that can specifically hybridize Mycobacteria strains on a biochips after DNA amplification using U1 and U4 consensus primers

For some species, several potential capture probes have been designed. Further testing may reveal advantages of some with respect to the others.

Avium 2 : 5'-GCGCGGTCGTCTCCGAAGCCC-3'(SEQ ID NO 27)

Avium 3 : 5'-CCGCTCGGCACTAAAAGGCAGTGGAAGC-3'(SEQ ID NO 28)

Avium 4 : 5'-GAAGCCCGCGGGCAAGCCAAT-3'(SEQ ID NO 29)

Gastrii 1 :          5'-GATCGGCAGCGGTGCCGG-3'(SEQ ID NO 30)

Gastrii 5 : 5'-GCGGTGCCGGGGCCGGTA-3'(SEQ ID NO 31)

Gastrii 6 : 5'-CGGTATCGCGGGCGGCAAGGT-3'(SEQ ID NO 32)
Gordonae 2 : 5'-GGCGACGGGCACTAGTTGTCAGAGGTG-3'(SEQ ID NO 33)
Intracellulare 3 : 5'-CCGCCGGGGGCCTCGC-3'(SEQ ID NO 34)
Intracellulare 4 : 5'-TCGCCGCCCAAGACAGTGGCG-3' (SEQ ID NO 35)
Kansasii 4 : 5'-ATCCGCCGATCGTCGGCAGTGGT-3'(SEQ ID NO 36)
Kansasii 5 : 5'-GATCGTCGGCAGTGGTGACGGGG-3'(SEQ ID NO 37)
Kansasii 6 : 5'-GGGCCGGTATCACGGGGGCAA-3'(SEQ ID NO 38)
Leprae 2: 5'-GATTTCGGCGTCCATCGGTGGTAG-3'(SEQ ID NO 39)
Malmoense 2 : 5'-AACGCAAGATCTCGAAGGTGTTTTCAAAGGCG-3'(SEQ ID NO
40)
Malmoense 3 : 5'-GACCCACAACACTGGTCGGCGCC-3'(SEQ ID NO 41)
Marinum 2 : 5'-GCCAATCGGCTCGGCGGGA-3'(SEQ ID NO 42)
Marinum 3 : 5'-ATCGACGGAGGTGATGGCGCTG-3'(SEQ ID NO 43)
Simiae 2 : 5'-CGATCGCTCCTGGTCGCGCCT-3'(SEQ ID NO 44)
Simiae 3 : 5'-CCGGCGCACCCGCTCGAAC-3'(SEQ ID NO 45)
Szulgai 2 : 5'-CTGCGATGAGCAAGCGGCCCG-3'(SEQ ID NO 46)

Gastrii 6 :	5'-CGGTATCGCGGGCGGCAAGGT-3'(SEQ ID NO 32)

Gordonae 2 : 5'-GGCGACGGGCACTAGTTGTCAGAGGTG-3'(SEQ ID NO 33)
Intracellulare 3 : 5'-CCGCCGGGGGCCTCGC-3'(SEQ ID NO 34)
Intracellulare 4 : 5'-TCGCCGCCCAAGACAGTGGCG-3' (SEQ ID NO 35)
Kansasii 4 : 5'-ATCCGCCGATCGTCGGCAGTGGT-3'(SEQ ID NO 36)
Kansasii 5 : 5'-GATCGTCGGCAGTGGTGACGGGG-3'(SEQ ID NO 37)
Kansasii 6 : 5'-GGGCCGGTATCACGGGGGCAA-3'(SEQ ID NO 38)
Leprae 2: 5'-GATTTCGGCGTCCATCGGTGGTAG-3'(SEQ ID NO 39)
Malmoense 2 : 5'-AACGCAAGATCTCGAAGGTGTTTTCAAAGGCG-3'(SEQ ID NO
40)
Malmoense 3 : 5'-GACCCACAACACTGGTCGGCGCC-3'(SEQ ID NO 41)
Marinum 2 : 5'-GCCAATCGGCTCGGCGGGA-3'(SEQ ID NO 42)
Marinum 3 : 5'-ATCGACGGAGGTGATGGCGCTG-3'(SEQ ID NO 43)
Simiae 2 : 5'-CGATCGCTCCTGGTCGCGCCT-3'(SEQ ID NO 44)
Simiae 3 : 5'-CCGGCGCACCCGCTCGAAC-3'(SEQ ID NO 45)
Szulgai 2 : 5'-CTGCGATGAGCAAGCGGCCCG-3'(SEQ ID NO 46)

Gordonae 2 :          5'-GGCGACGGGCACTAGTTGTCAGAGGTG-3'(SEQ ID NO 33)

Intracellulare 3 :	5'-CCGCCGGGGGCCTCGC-3'(SEQ ID NO 34)
Intracellulare 4 :	5'-TCGCCGCCCAAGACAGTGGCG-3' (SEQ ID NO 35)
Kansasii 4 :	5'-ATCCGCCGATCGTCGGCAGTGGT-3'(SEQ ID NO 36)
Kansasii 5 :	5'-GATCGTCGGCAGTGGTGACGGGG-3'(SEQ ID NO 37)
Kansasii 6 :	5'-GGGCCGGTATCACGGGGGCAA-3'(SEQ ID NO 38)
Leprae 2:	5'-GATTTCGGCGTCCATCGGTGGTAG-3'(SEQ ID NO 39)
Malmoense 2 :	5'-AACGCAAGATCTCGAAGGTGTTTTCAAAGGCG-3'(SEQ ID NO
40)
Malmoense 3 :	5'-GACCCACAACACTGGTCGGCGCC-3'(SEQ ID NO 41)
Marinum 2 :	5'-GCCAATCGGCTCGGCGGGA-3'(SEQ ID NO 42)
Marinum 3 :	5'-ATCGACGGAGGTGATGGCGCTG-3'(SEQ ID NO 43)
Simiae 2 :	5'-CGATCGCTCCTGGTCGCGCCT-3'(SEQ ID NO 44)
Simiae 3 :	5'-CCGGCGCACCCGCTCGAAC-3'(SEQ ID NO 45)
Szulgai 2 :	5'-CTGCGATGAGCAAGCGGCCCG-3'(SEQ ID NO 46)

Intracellulare 3 :	5'-CCGCCGGGGGCCTCGC-3'(SEQ ID NO 34)

Intracellulare 4 :	5'-TCGCCGCCCAAGACAGTGGCG-3' (SEQ ID NO 35)
Kansasii 4 :	5'-ATCCGCCGATCGTCGGCAGTGGT-3'(SEQ ID NO 36)
Kansasii 5 :	5'-GATCGTCGGCAGTGGTGACGGGG-3'(SEQ ID NO 37)
Kansasii 6 :	5'-GGGCCGGTATCACGGGGGCAA-3'(SEQ ID NO 38)
Leprae 2:	5'-GATTTCGGCGTCCATCGGTGGTAG-3'(SEQ ID NO 39)
Malmoense 2 :	5'-AACGCAAGATCTCGAAGGTGTTTTCAAAGGCG-3'(SEQ ID NO
40)
Malmoense 3 :	5'-GACCCACAACACTGGTCGGCGCC-3'(SEQ ID NO 41)
Marinum 2 :	5'-GCCAATCGGCTCGGCGGGA-3'(SEQ ID NO 42)
Marinum 3 :	5'-ATCGACGGAGGTGATGGCGCTG-3'(SEQ ID NO 43)
Simiae 2 :	5'-CGATCGCTCCTGGTCGCGCCT-3'(SEQ ID NO 44)
Simiae 3 :	5'-CCGGCGCACCCGCTCGAAC-3'(SEQ ID NO 45)
Szulgai 2 :	5'-CTGCGATGAGCAAGCGGCCCG-3'(SEQ ID NO 46)

Intracellulare 4 :      5'-TCGCCGCCCAAGACAGTGGCG-3' (SEQ ID NO 35)

Kansasii 4 :	5'-ATCCGCCGATCGTCGGCAGTGGT-3'(SEQ ID NO 36)
Kansasii 5 :	5'-GATCGTCGGCAGTGGTGACGGGG-3'(SEQ ID NO 37)
Kansasii 6 :	5'-GGGCCGGTATCACGGGGGCAA-3'(SEQ ID NO 38)
Leprae 2:	5'-GATTTCGGCGTCCATCGGTGGTAG-3'(SEQ ID NO 39)
Malmoense 2 :	5'-AACGCAAGATCTCGAAGGTGTTTTCAAAGGCG-3'(SEQ ID NO
40)
Malmoense 3 :	5'-GACCCACAACACTGGTCGGCGCC-3'(SEQ ID NO 41)
Marinum 2 :	5'-GCCAATCGGCTCGGCGGGA-3'(SEQ ID NO 42)
Marinum 3 :	5'-ATCGACGGAGGTGATGGCGCTG-3'(SEQ ID NO 43)
Simiae 2 :	5'-CGATCGCTCCTGGTCGCGCCT-3'(SEQ ID NO 44)
Simiae 3 :	5'-CCGGCGCACCCGCTCGAAC-3'(SEQ ID NO 45)
Szulgai 2 :	5'-CTGCGATGAGCAAGCGGCCCG-3'(SEQ ID NO 46)

Kansasii 4 : 5'-ATCCGCCGATCGTCGGCAGTGGT-3'(SEQ ID NO 36)

Kansasii 5 :	5'-GATCGTCGGCAGTGGTGACGGGG-3'(SEQ ID NO 37)
Kansasii 6 :	5'-GGGCCGGTATCACGGGGGCAA-3'(SEQ ID NO 38)
Leprae 2:	5'-GATTTCGGCGTCCATCGGTGGTAG-3'(SEQ ID NO 39)
Malmoense 2 :	5'-AACGCAAGATCTCGAAGGTGTTTTCAAAGGCG-3'(SEQ ID NO
40)
Malmoense 3 :	5'-GACCCACAACACTGGTCGGCGCC-3'(SEQ ID NO 41)
Marinum 2 :	5'-GCCAATCGGCTCGGCGGGA-3'(SEQ ID NO 42)
Marinum 3 :	5'-ATCGACGGAGGTGATGGCGCTG-3'(SEQ ID NO 43)
Simiae 2 :	5'-CGATCGCTCCTGGTCGCGCCT-3'(SEQ ID NO 44)
Simiae 3 :	5'-CCGGCGCACCCGCTCGAAC-3'(SEQ ID NO 45)
Szulgai 2 :	5'-CTGCGATGAGCAAGCGGCCCG-3'(SEQ ID NO 46)

Kansasii 5 :          5'-GATCGTCGGCAGTGGTGACGGGG-3'(SEQ ID NO 37)

Kansasii 6 :	5'-GGGCCGGTATCACGGGGGCAA-3'(SEQ ID NO 38)

Leprae 2:	5'-GATTTCGGCGTCCATCGGTGGTAG-3'(SEQ ID NO 39)
Malmoense 2 :	5'-AACGCAAGATCTCGAAGGTGTTTCAAAGGCG-3'(SEQ ID NO
40)
Malmoense 3 :	5'-GACCCACAACACTGGTCGGCGCC-3'(SEQ ID NO 41)
Marinum 2 :	5'-GCCAATCGGCTCGGCGGGA-3'(SEQ ID NO 42)
Marinum 3 :	5'-ATCGACGGAGGTGATGGCGCTG-3'(SEQ ID NO 43)
Simiae 2 :	5'-CGATCGCTCCTGGTCGCGCCT-3'(SEQ ID NO 44)
Simiae 3 :	5'-CCGGCGCACCCGCTCGAAC-3'(SEQ ID NO 45)
Szulgai 2 :	5'-CTGCGATGAGCAAGCGGCCCG-3'(SEQ ID NO 46)

Leprae 2:		5'-GATTTCGGCGTCCATCGGTGGTAG-3'(SEQ ID NO 39)

Malmoense 2 :        5'-AACGCAAGATCTCGAAGGTGTTTTCAAAGGCG-3'(SEQ ID NO
40)
Malmoense 3 :        5'-GACCCACAACACTGGTCGGCGCC-3'(SEQ ID NO 41)
Marinum 2 :          5'-GCCAATCGGCTCGGCGGGA-3'(SEQ ID NO 42)
Marinum 3 :          5'-ATCGACGGAGGTGATGGCGCTG-3'(SEQ ID NO 43)
Simiae 2 :           5'-CGATCGCTCCTGGTCGCGCCT-3'(SEQ ID NO 44)
Simiae 3 :           5'-CCGGCGCACCCGCTCGAAC-3'(SEQ ID NO 45)
Szulgai 2 :          5'-CTGCGATGAGCAAGCGGCCCG-3'(SEQ ID NO 46)

Malmoense 2 :        5'-AACGCAAGATCTCGAAGGTGTTTTCAAAGGCG-3'(SEQ ID NO
40)

Malmoense 3 :        5'-GACCCACAACACTGGTCGGCGCC-3'(SEQ ID NO 41)

Marinum 2 : 5'-GCCAATCGGCTCGGCGGGA-3'(SEQ ID NO 42)
Marinum 3 : 5'-ATCGACGGAGGTGATGGCGCTG-3'(SEQ ID NO 43)
Simiae 2 : 5'-CGATCGCTCCTGGTCGCGCCT-3'(SEQ ID NO 44)
Simiae 3 : 5'-CCGGCGCACCCGCTCGAAC-3'(SEQ ID NO 45)
Szulgai 2 : 5'-CTGCGATGAGCAAGCGGCCCG-3'(SEQ ID NO 46)

Marinum 2 : 5'-GCCAATCGGCTCGGCGGGA-3'(SEQ ID NO 42)

Marinum 3 : 5'-ATCGACGGAGGTGATGGCGCTG-3'(SEQ ID NO 43)

Simiae 2 : 5'-CGATCGCTCCTGGTCGCGCCT-3'(SEQ ID NO 44)
Simiae 3 : 5'-CCGGCGCACCCGCTCGAAC-3'(SEQ ID NO 45)
Szulgai 2 : 5'-CTGCGATGAGCAAGCGGCCCG-3'(SEQ ID NO 46)

Simiae 2 : 5'-CGATCGCTCCTGGTCGCGCCT-3'(SEQ ID NO 44)

Simiae 3 : 5'-CCGGCGCACCCGCTCGAAC-3'(SEQ ID NO 45)
Szulgai 2 : 5'-CTGCGATGAGCAAGCGGCCCG-3'(SEQ ID NO 46)

Simiae 3 : 5'-CCGGCGCACCCGCTCGAAC-3'(SEQ ID NO 45)

Szulgai 2 : 5'-CTGCGATGAGCAAGCGGCCCG-3'(SEQ ID NO 46)

Szulgai 3 :          5'-GCGGCCCGGTCGGCCG-3'(SEQ ID NO 47)
Tuberculosis 2 :     5'-CGGCCGTCCAGTCGTTAATGTCGC-3' (SEQ ID NO 48)
Xenopi 2 :           5'-CGGTAGAAGCTGCGATGACACGCCA-3'(SEQ ID NO 49)

It is obvious that the above-mentioned nucleic acids can also be used as primers in other experiments.

### Table 3 (c) Example of oligonucleotides used as species specific primers

The specific primer pair includes an universal (consensus) antisense primer (for instance U1, U5 or U9) and one of the following primers. This can be used for amplifying mycobacterial DNA in a multiplex diagnostic strategy.

Theoretical annealing temperature (Tm) of each primer as well as the expected size of the amplicon are given.

U1 5'-GAGTAGGTCATGGCTCCTCC -3' Tm 63,7 (see
table 1)

Avium 5 :	5'- GCAAGCCAATGGCGA -3' (SEQ ID NO 50)	Tm 64 ,8	166
bp

bp
Intracellulare 5 : 5'- CTCGCCGCCCAAGA -3'(SEQ ID NO 51) Tm 65,1 172
bp

Tuberculosis 3 :  5'- CCGTCCAGTCGTTAATGTC -3'(SEQ ID NO 52)   Tm 61,6          134
bp

Simiae 4 : 5'- ACGATCGCTCCTGGTCG -3'(SEQ ID NO 53) Tm 66,6 219
bp

Malmoense 4 :    5'- AAGATCTCGAAGGTGTTTTCAA -3'(SEQ ID NO 54)   Tm 62,5   119
bp

Mycobacterium avium can be further distinguished from Mycobacterium avium subspecies paratuberculosis by co-amplification with F57 gene specific primers.

A specific primer pair for amplification of at least part of the F57 gene of Mycobacterium avium subspecies paratuberculosis is:

F57a 5'-GGTCGCGTCATTCAGAATC-3'(SEQ ID NO 55) Tm 64,4 439
bp

F57b 5'-TCTCAGACAGTGGCAGGTG-3'(SEQ ID NO 56) Tm 64,1

It is obvious that the above-mentioned nucleic acids can also be used as probes in other experiments.

**Table 4.**

| **Validation of the consensus U1-U4 amplification on clinical mycobacterial strains.** | | |
|---|---|---|
| Mycobacteria | size (bp) | Nbr of + amplif. /Nbr of samples |
| ***Tuberculosiss Group (TUB)*** | | |
| M. tuberculosis | 177 | 15/15 |
| M. bovis | 177 | 9/10 |
| M. africanum | 177 | 5/5 |

| ***Non Tuberculosis Group (NTB)*** | | |
|---|---|---|
| M. avium Complex (MAC) | | |
| M. avium | 256 | 16/17 |
| M. paratuberculosis | 256 | 21/21 |
| M. intracellulare | 256 | 9/9 |
| M. scrofulaceum | 259 | 4/5 |

| MOTT (Myc. Other Then Tub - MOTT) | | |
|---|---|---|
| M. szulgai | 163 | 9/9 |
| M. kansasii | 225 | 10/11 |
| M. gastri | 223 | 2/2 |
| M. xenopi | 265 | 12/12 |
| M. ulcerans | 211 | 20/20 |
| M. leprae | 269 | 1/1 |
| M. non chromogenicum | 169 | 2/2 |
| M. simiae | 298 | 6/11 |
| M. malmoense | 290 | 4/7 |
| M. gordonae | 182 | 4/10 |
| M. marinum | 236 | 5/5 |

**Table 5.**

| **Classification of Pseudomonas** | |
|---|---|
| Group I | *Pseudomonas fluorescens* (*P. aeruginosa; P. fluorescens; P. putida)* *Pseudomonas stutzeri* *Pseudomonas pseudoalcaligenes; Ps. alcaligenes* |
| Group II | *Burkholderia cepacia; B. mallei; B. pseudomallei; B. pickettii; B. gladioli* |
| Group III | *Comanomas testosteroni; C. acidovrans* |
| Group IV | *Brevendimonas diminuta; B. vesicularis* |
| Group V | *Stenotrophomonas maltophilia* |

SEQUENCE LISTING

<110>  Université Catholique de Louvain

<120>  Identification of nucleotide sequences specific for mycobacterial and pseudo
monas
species, development of differential diagnosis strategies for mycobacterial and pse
udomonas
species

<130>  UCL-021-EP

<150>  US 60/269,848
<151>  2001-02-21

<150>  US 60/292,509
<151>  2001-05-23

<150>  EP 01870030.2
<151>  2001-02-19

<160>  89

<170>  PatentIn version 3.1

<210>  1
<211>  20
<212>  DNA
<213>  Mycobacterium sp.

<400>  1
gagtaggtca tggctcctcc                                                    20


<210>  2
<211>  20
<212>  DNA
<213>  Mycobacterium sp.

<400>  2
catgcagcga attagaacgt                                                    20


<210>  3
<211>  20
<212>  DNA
<213>  Mycobacterium sp.

<400>  3
catgcagcga attagaacgt                                                    20


<210>  4
<211>  18
<212>  DNA
<213>  Mycobacterium sp.

<400>  4
aacttgacga actcgccg                                                      18


<210>  5
<211>  18

<212>  DNA
<213>  Mycobacterium sp.

<400>  5
aggtattcgc gcagcatg                                                    18


<210>  6
<211>  18
<212>  DNA
<213>  Mycobacterium sp.

<400>  6
gtasgtcatr rstyctcc                                                    18


<210>  7
<211>  18
<212>  DNA
<213>  Mycobacterium sp.

<400>  7
ggtgaacatt gggccgaa                                                    18


<210>  8
<211>  21
<212>  DNA
<213>  Mycobacterium avium

<400>  8
cggtcgtctc cgaagcccgc g                                                21


<210>  9
<211>  20
<212>  DNA
<213>  Mycobacterium gastri

<400>  9
gatcggcagc ggtgccgggg                                                  20


<210>  10
<211>  19
<212>  DNA
<213>  Mycobacterium gastri

<400>  10
gtatcgcggg cggcaaggt                                                   19


<210>  11
<211>  24
<212>  DNA
<213>  Mycobacterium gastri

<400>  11
tctgccgatc ggcagcggtg ccgg                                             24


<210>  12
<211>  24

<212>  DNA
<213>  Mycobacterium gastri

<400>  12
gccggggccg gtattcgcgg gcgg                                              24


<210>  13
<211>  22
<212>  DNA
<213>  Mycobacterium gordonae

<400>  13
gacgggcact agttgtcaga gg                                                22


<210>  14
<211>  21
<212>  DNA
<213>  Mycobacterium intracellulare

<400>  14
gggccgccgg gggcctcgcc g                                                 21


<210>  15
<211>  21
<212>  DNA
<213>  Mycobacterium intracellulare

<400>  15
gcctcgccgc ccaagacagt g                                                 21


<210>  16
<211>  22
<212>  DNA
<213>  Mycobacterium leprae

<400>  16
gatttcggcg tccatcggtg gt                                                22


<210>  17
<211>  21
<212>  DNA
<213>  Mycobacterium kansasii

<400>  17
gatcgtcggc agtggtgacg g                                                 21


<210>  18
<211>  17
<212>  DNA
<213>  Mycobacterium kansasii

<400>  18
tcgtcggcag tggtgac                                                      17


<210>  19
<211>  27

<212>  DNA
<213>  Mycobacterium kansasii

<400>  19
atccgccgat cgtcggcagt ggtgacg                                          27


<210>  20
<211>  21
<212>  DNA
<213>  Mycobacterium malmoense

<400>  20
gacccacaac actggtcggc g                                                21


<210>  21
<211>  21
<212>  DNA
<213>  Mycobacterium marinum

<400>  21
cggaggtgat ggcgctggtc g                                                21


<210>  22
<211>  20
<212>  DNA
<213>  Mycobacterium scrofulaceum

<400>  22
cggcggcacg gatcggcgtc                                                  20


<210>  23
<211>  20
<212>  DNA
<213>  Mycobacterium simiae

<400>  23
atcgctcctg gtcgcgccta                                                  20


<210>  24
<211>  21
<212>  DNA
<213>  Mycobacterium szulgai

<400>  24
cccggcgcga ccagcagaac g                                                21


<210>  25
<211>  22
<212>  DNA
<213>  Mycobacterium tuberculosis

<400>  25
gccgtccagt cgttaatgtc gc                                               22


<210>  26
<211>  22

<212>  DNA
<213>  Mycobacterium xenopi

<400>  26
cggtagaagc tgcgatgaca cg                                              22


<210>  27
<211>  21
<212>  DNA
<213>  Mycobacterium avium

<400>  27
gcgcggtcgt ctccgaagcc c                                               21


<210>  28
<211>  28
<212>  DNA
<213>  Mycobacterium avium

<400>  28
ccgctcggca ctaaaaggca gtggaagc                                        28


<210>  29
<211>  21
<212>  DNA
<213>  Mycobacterium avium

<400>  29
gaagcccgcg ggcaagccaa t                                               21


<210>  30
<211>  18
<212>  DNA
<213>  Mycobacterium gastri

<400>  30
gatcggcagc ggtgccgg                                                   18


<210>  31
<211>  18
<212>  DNA
<213>  Mycobacterium gastri

<400>  31
gcggtgccgg ggccggta                                                   18


<210>  32
<211>  21
<212>  DNA
<213>  Mycobacterium gastri

<400>  32
cggtatcgcg ggcggcaagg t                                               21


<210>  33
<211>  27

<212>  DNA
<213>  Mycobacterium gordonae

<400>  33
ggcgacgggc actagttgtc agaggtg                                          27


<210>  34
<211>  16
<212>  DNA
<213>  Mycobacterium intracellulare

<400>  34
ccgccggggg cctcgc                                                      16


<210>  35
<211>  21
<212>  DNA
<213>  Mycobacterium intracellulare

<400>  35
tcgccgccca agacagtggc g                                                21


<210>  36
<211>  23
<212>  DNA
<213>  Mycobacterium kansasii

<400>  36
atccgccgat cgtcggcagt ggt                                              23


<210>  37
<211>  23
<212>  DNA
<213>  Mycobacterium kansasii

<400>  37
gatcgtcggc agtggtgacg ggg                                              23


<210>  38
<211>  21
<212>  DNA
<213>  Mycobacterium kansasii

<400>  38
gggccggtat cacgggggca a                                                21


<210>  39
<211>  24
<212>  DNA
<213>  Mycobacterium leprae

<400>  39
gatttcggcg tccatcggtg gtag                                             24


<210>  40
<211>  32

<212>  DNA
<213>  Mycobacterium malmoense

<400>  40
aacgcaagat ctcgaaggtg ttttcaaagg cg                                    32


<210>  41
<211>  23
<212>  DNA
<213>  Mycobacterium malmoense

<400>  41
gacccacaac actggtcggc gcc                                              23


<210>  42
<211>  19
<212>  DNA
<213>  Mycobacterium marinum

<400>  42
gccaatcggc tcggcggga                                                   19


<210>  43
<211>  22
<212>  DNA
<213>  Mycobacterium marinum

<400>  43
atcgacggag gtgatggcgc tg                                               22


<210>  44
<211>  21
<212>  DNA
<213>  Mycobacterium simiae

<400>  44
cgatcgctcc tggtcgcgcc t                                                21


<210>  45
<211>  19
<212>  DNA
<213>  Mycobacterium simiae

<400>  45
ccggcgcacc cgctcgaac                                                   19


<210>  46
<211>  21
<212>  DNA
<213>  Mycobacterium szulgai

<400>  46
ctgcgatgag caagcggccc g                                                21


<210>  47
<211>  16

<212>  DNA
<213>  Mycobacterium szulgai

<400>  47
gcggcccggt cggccg                                                    16


<210>  48
<211>  24
<212>  DNA
<213>  Mycobacterium tuberculosis

<400>  48
cggccgtcca gtcgttaatg tcgc                                           24


<210>  49
<211>  25
<212>  DNA
<213>  Mycobacterium xenopi

<400>  49
cggtagaagc tgcgatgaca cgcca                                          25


<210>  50
<211>  15
<212>  DNA
<213>  Mycobacterium avium

<400>  50
gcaagccaat ggcga                                                     15


<210>  51
<211>  14
<212>  DNA
<213>  Mycobacterium intracellulare

<400>  51
ctcgccgccc aaga                                                      14


<210>  52
<211>  19
<212>  DNA
<213>  Mycobacterium tuberculosis

<400>  52
ccgtccagtc gttaatgtc                                                 19


<210>  53
<211>  17
<212>  DNA
<213>  Mycobacterium simiae

<400>  53
acgatcgctc ctggtcg                                                   17


<210>  54
<211>  22

<212>  DNA
<213>  Mycobacterium malmoense

<400>  54
aagatctcga aggtgttttc aa                                              22


<210>  55
<211>  19
<212>  DNA
<213>  Mycobacterium avium subspecies paratuberculosis

<400>  55
ggtcgcgtca ttcagaatc                                                  19


<210>  56
<211>  19
<212>  DNA
<213>  Mycobacterium avium subspecies paratuberculosis

<400>  56
tctcagacag tggcaggtg                                                  19


<210>  57
<211>  216
<212>  DNA
<213>  Mycobacterium intracellulare

<400>  57
gttctacctg tgctgagcaa gctccggtga taccgaccgt ctcgccggag ggccgccggg    60

ggcctcgccg cccaagacag tggcggcgcc accggttccc gcacgtgcgc tagcgtgggt   120

gatcgaccgc gtcgcaatgc ggtgacgcgc ctgcaagcac agcgtcgcat cgccaccgcg   180

gcgcccgctc ggcacttaaa ggcactggta gcaaca                             216


<210>  58
<211>  881
<212>  DNA
<213>  Mycobacterium avium

<400>  58
tcgtagctgg cttcctcgtc ggtccacagc gcccgcatcg cttccaggta ttcgcgcagc    60

atggtgcggc gccggcccgc cggcacgccg tggtcggcga gttcgtcggt gttccagccg   120

aacccgacgc cgaggctgac ccggccgccg gacagatggt caagggtggc aatacttttc   180

gccagcgtga tcgggtcgtg ttcgaccggc agggccaccg cggtggacag ccgcacccgc   240

gaggtgacgg cacaggccgc gcccagactg acccacgggt ccagggtgcg catgtagcgg   300

tcgtcgggca gcgacgcgtc gccggtggtc gggtgcgcgg cctcccgctt gatcgggata   360

tgcgtgtgtt ccggcacgta gaaggtcgca aacccgtggt cgtcggcaag cttcgcggcc   420

gcagccggag agatgccacg gtcgctggtg aaaagcacaa gcccgtaatc catgcagtga   480

attagaacgt gttctacctc tgcggggcaa gctgtcgtga tacggaccgt ctcgccgcgc   540

ggtcgtctcc gaagcccgcg ggcaagccaa tggcgacggc accggccgtc gcacgtgcgc 600

tagcgtgggt gatcgaccgt gtcgctcgcg cagtgacgcg cctgcaagca ccgcgtcgca 660

tcgcaaccgt ggcgcccgct cggcactaaa aggcagtgga agcaacagga ggagccatga 720

cctactctcc cggcagcccc ggatatccac cggcgcagtc tggcggcacc tatgcaggcg 780

ccacaccatc tttcgccaaa gacgacgacg gcaagagcaa actcccgctc tacctcaaca 840

tcgccgtggt cgccctgggt ttcgcggcct acctgctgaa t 881

<210> 59
<211> 642
<212> DNA
<213> Mycobacterium gastri

<400> 59
gtgcgccggc gccccggcgg cacgccatgg tcggcgagtt cgtgcgcccg gcggcacgcc 60

atggtcggcg agttcgtcgg tgttccagcc gaatccgacg ccgacgctga cccggccccc 120

ggatagtggt ccagcgtggc aatgcttttg gccagcgtga tcgggtcatg ctccaccgca 180

gcgcaaccgc ggttgacagc ctgactcggg aggtgaccgc tgaagccgca cccaagctca 240

cccacgggtc cagggtgcgc atatagcggt cgtccggcag cgacgcgtca cccgtcgtgg 300

gatgggcggc ttcccgtttg accgggatat gcgtgtgttc gggcacgtag agagtgcgaa 360

agccatggtc gtcggccagt ttcgcggctg ccgccgggga gatcccacgg tcgctggtga 420

aaaggacaag cccgtaatcc atgaacagaa ttagaacgtg ttctacctcc gccgggcaag 480

cggctcatct gccgatcggc agcggtgccg gggccggtat cgcgggcggc aaggtcgcca 540

cggcgtgagt acccggccgt gcgctagcgt gggtcatcga attgtgtcgc agggagcaat 600

cgtcgcattg cagcaggcgt agcgacggca ccggaggtaa ca 642

<210> 60
<211> 745
<212> DNA
<213> Mycobacterium gordonae

<400> 60
gtgcgacgac ggccggccag cacgttatgg tcggcgagct cgtcggtgtt ccagccgaac 60

ccgacgccga ggctaactcg cccgccggac aggtgatcca gcgtggcgat gcttttcgcc 120

aaggtgatcg ggtcatgctc gaccggcaac gcgactgccg tcgacagccg cacccgcgac 180

gtcacagcac acgccgcgcc caggctcacc cagggatcca gggtgcgcat ataacggtcg 240

tcgggcagcg tctcgtctcc ggtggtggga tgagccgcct cgcgtttgat cgggatatgc 300

gtgtgttcgg gtacgtagaa ggtgtgaaaa ccatgtgtgt cggcaagttt cgctgctgcc 360

gcaggggaaa taccgcgatc gctggtgaac agaacgaggc tgtagtccat gccccaattt 420

agaacgtgtt ctacttttgg ccgcagccga ccccctgcgg cgacgggcac tagttgtcag 480

aggtgcgcta gcgtggttga tcgaatgcgt cgcaggccgt accgcgtcgt gccgaagcag 540

aggggccgtg acggcaccgg aagcaacagg aggacttatg acctacccgc ccggtagtcc 600

cggatatcca tccgcccagc agtcggccgg caactacggc agctccgctc ccgccgccgg 660

ccagtccgag ccgggtgaaa gcaagctggg actgtacctg gccatcgcgg tggcggccct 720

gggcctactg gcgtacctct tcagc 745

<210> 61
<211> 785
<212> DNA
<213> Mycobacterium kansasii

<400> 61
gtgcgccggc gcgccggcgg cacgccatgg tcagcgagtt cgtcggtgtt ccagccgaat 60

ccgacgccga cgctgacccg ccccccggat aggtggtcca gcgtggcaat gcttttggcc 120

agcgtgatcg ggtcatgctc gaccggcaac gcaaccgctg ttgacagtcg gacccggaag 180

gtgaccgctg aagccgcgcc caaactcacc cacgggtcca gcgtgcgcat atagcggtcg 240

tccggcagcg acgcgtcacc cgtcgtggga tggcggcctc ccgtttgacc gggatgtgcg 300

tgtgttcggg cacgtagaaa gtgcgaaagc catggtcgtc ggccagtttc gcggctgccg 360

cgggagaaat gccacggtcg ctggtgaaaa ggacaagccc gtaatccatg aacagaatta 420

gaacgtgttc tacctcagcc gggcaagcgg ctcatccgcc gatcgtcggc agtggtgacg 480

gggccggtat cacgggggca aggtcgccac ggcgcgagta ccaggccgtg cgctagcgtg 540

ggtcatcgaa tcgtgtcgca gggagcaatc gtcgcattgc agcaggcgta gcgacggcac 600

tggaggtaac aggaggagcc atgacctact caccaggtag tcccggatat ccgcccgcgc 660

aatcggccgg ctcctacgga gccgccacac cgtctttcgc caaggccgac gacggtgtca 720

gcaagcttcc gatgtacctg agcatggcgg ttgccgcgct cgggctgctg gcgtatctgg 780

ccagc 785

<210> 62
<211> 691
<212> DNA
<213> Mycobacterium malmoense

<400> 62
tcgtaggccg cttcctcctg ggtccacagc gcccgcattg cctcgatgta ttcacgcagc 60

atggtgcgac ggcgcccggc cggcacgccg tggtcggcga gctcgtcggt gttccagcca 120

aacccaacgc cgaggctgac ccggccgccg gacaggtggt ccaaggtggc aatacttttc 180

gccagcgtga tcgggtcgtg ctcgacgggc agcgccaccg cggtagacag ccgcacccgc 240

gacgtcacgg cgcacgccgc gcccaggctc acccacgggt ctagcgtgcg catatagcgg 300

tcgtccggca agcgacgcgc cacccgtcgt cggatgggcc gcctcgcgct tgaccgggat 360

atgggtgtgt tccggcacgt agaacgtctg gaagccgtgg tcgtcggcaa gtttggcggc 420

tgccgccggg gagatgccgc ggtcgctggt gaaaagtaca agcccgtaat ccatggacag 480

aattagaacg tgttctaccg gcggtgggca agccgctgcg ccgccgagga tctcgactcg 540

gacccacaac actggtcggc gccgggcgcg ccgacaggtc ggtcggcccg gcacgggcgg 600

ccgaacgtgc gctagcgtgg gtgatcgatc gcgtcgcaac gcaagatctc atgcggcgtc 660

gctgagggtc ttgaaggcac tggaagcaat a 691

<210> 63
<211> 698
<212> DNA
<213> Mycobacterium simiae

<400> 63
tcgtattggg cttcttcctg cgtccacagc gcccgcatgg cttccaggta ctcgcgcagc 60

atggtccgcc ggcgcgccgg cggcacgttg tggtcggcca gttcgtcggt gttccaaccg 120

aacccgacgc ccacactgac ccgtccgccg gacagatggt ccagggtggc gatgcttttc 180

gccagcgtga tcgggtcgtg ctcgacgggc agcgcgaccg cggtggacag tcgcacccgc 240

gaggtgaccg cgcacgccgc gcccagactg acccacgggt ccagcgtgcg catgtagcgg 300

tcgtcgggca gcgattcgtc gcccgtcgtg ggatgggccg cctcgcgctt gatcgggatg 360

tgagtgtgtt ctggcacgta gaacgttgtg aagccatggt cgtcggcgag tttggccgcg 420

gccgccgggg cgatgccccg atcactggtg aaaagcacga gcccgtaatc catgcacaga 480

attagaacgt gttctacctc tgtggagcaa gcggcccccg ctacgtcgac ccgcagacgg 540

gccgctgaga cgatcgctcc tggtcgcgcc taggggccgg tcgctcccgc gcacccgctc 600

gaacgtgcgc tagcgtggtt gatcggtcgc gcgtaacgca aacgcgggca agcagtgacg 660

tcgcgcccga cgaggtcttg aaggcactgg aagcaaca 698

<210> 64
<211> 712
<212> DNA
<213> Mycobacterium szulgai

<400> 64
gtgcggcggc gcccggccgg gacgccgtga tcagcgagct cgtcggtatt ccagccgaag 60

ccgacgccga ggctgacccg gctgccggac agatgatcca gcgtggcaat gcttttggcc 120

agcgtgatcg gatcatgctc gaccggcagc gccaccgcgg tggacaaccg gacccgagac 180

gtcaccgcgg ccgcagcacc caaactcacc cacgggtcca gcgtgcgcat gtagcggtca 240

tcgggcagcg acgcgtcact cgtagtggga tgggcagcct cccgcttgat cgggatgtgg 300

gtgtgttcag gcacgtagaa cgtctgaaaa ccgtggtcgt cggccagctt tgcggccgcc 360

gccggggcaa tgccgcgatc gctggtgaaa agtacaagcc cgtaatccat gcaccgaatt 420

agaacgtgtt ctacctgcga tgagcaagcg gcccggtcgg ccgacgagca ggtcggcccg 480

gcgcgaccag cagaacgtgc gctagcgtgg ttgatcgagt cgcgcaccgg aaagcaaccg 540

gaagtaatca ggaggagcca tgacctactc gaccggcagc cccggatatc cgcctgcgca 600

gcagcccggg gggtcgtacg gcggcgccac tcctggtgac gctcagagca agcttccgct 660

gtacctcagc atggcggtgg ccgccctcgg cctggccgcg tatctcgcca gc 712

<210> 65
<211> 802
<212> DNA
<213> Mycobacterium tuberculosis

<400> 65
tcatagcagg cctcctcttg ggtccacaac gcccgcatcg cctcgaggta ttcgcgcagc 60

atggtgcggc ggcgtccggg tggcacacca tgatcgacga gctcgtcggt gttccagccg 120

aacccgaccc cgacgctgac ccggccgtgc gacaaatgat ccagcgtcgc aatgcttttc 180

gccagcgtga tcggatcatg ctcgaccggc agcgccaccg cggtggcaag ccggatccgc 240

gacgtcaccg ccgatgctgc tcccaggctc acccacgggt ccaacgtgcg catatagcgg 300

tcgtccggca gcgaagcgtc acccgtcgtc ggatgggccg cctggcgctt gaccgggatg 360

tgggtgtgtt cgggcacgta aaacgtgcga aacccgtggc tttcagcaag tctggcggcc 420

gcggccgggg tgatgccgcg gtcgctggtg aacagcacaa gtccgtagtg catgcaccga 480

attagaacgt gttccacctg cgccgggcaa gcggccgtcc agtcgttaat gtcgcgagcg 540

ccggtcgctc cggcagcggc acccgaacgt gcgctagcgt ggttgatcga atcgcgtcgc 600

cgggagcaca gcgtcgcact gcaccagtgg aggagccatg acctactcgc cgggtaaccc 660

cggatacccg caagcgcagc ccgcaggctc ctacggaggc gtcacaccct cgttcgccca 720

cgccgatgag ggtgcgagca agctaccgat gtacctgaac atcgcggtgg cagtgctcgg 780

cctggctgcg tacttcgcca gc 802

<210> 66
<211> 628
<212> DNA
<213> Mycobacterium bovis

<400> 66
tcatagcagg cctcctcttg ggtccacaac gcccgcatcg cctcgaggta ttcgcgcagc 60

atggtgcggc ggcgtccggg tggcacacca tgatcgacga gctcgtcggt gttccagccg 120

aacccgaccc cgacgctgac ccggccgtgc gacaaatgat ccagcgtcgc aatgcttttc 180

gccagcgtga tcggatcatg ctcgaccggc agcgccaccg cggtggcaag ccggatccgc 240

gacgtcaccg ccgatgctgc tcccaggctc acccacgggt ccaacgtgcg catatagcgg 300

tcgtccggca gcgaagcgtc acccgccgtc ggatgggccg cctggcgctt gaccgggatg 360

tgggtgtgtt cgggcacgta aaacgtgcga aacccgtggc tttcagcaag tctggcggcc 420

gcggccgggg tgatgccgcg gtcgctggtg aacagcacaa gtccgtagtg catgcaccga 480

attagaacgt gttccacctg cgccgggcaa gcggccgtcc agtcgttaat gtcgcgagcg 540

ccggtcgctc cggcagcggc acccgaacgt gcgctagcgt ggttgatcga atcgcgtcgc 600

cgggagcaca gcgtcgcact gcaccagt 628

<210> 67
<211> 400
<212> DNA
<213> Mycobacterium xenopi

<400> 67
gttcacccac cgcgagcaag cggcgccggt agaagctgcg atgacacgcc agtcgccgcg 60

agacccccgc cgccaggtgc gctagcgtgg atggtcgaat cgcgtcgcaa cgcctgccct 120

gacaagtcac ggcgttaatg gagcggtcca cgcagcgtcg cgcggaagcg gcgccctggg 180

gatacagcgt cgcaacacag tggcgcccca acggcactga tgcacaggag aagccatgac 240

gtactcgccc ggtagccccg gatatccacc cgcgcagtcc cccggttcct acggcggctc 300

cccacagtcg ttcgccaaat ccgatgacgg cgccagcaag ctgcagctgt atctgaccgt 360

cgcggtggtg gcgctcggcc tggcggccta cctggcgagt 400

<210> 68
<211> 707
<212> DNA
<213> Mycobacterium paratuberculosis

<400> 68
tcgtagctgg cttcctcgtc ggtccacagc gcccgcatcg cttccaggta ttcgcgcagc 60

atggtgcggc gccggcccgc cggcacgccg tggtcggcga gttcgtcggt gttccagccg 120

aacccgacgc cgaggctgac ccggccgccg gacagatggt caagggtggc aatacttttc 180

gccagcgtga tcgggtcgtg ttcgaccggc agggccaccg cggtggacag ccgcacccgc 240

gaggtgacgg cacaggccgc gcccagactg acccacgggt ccagggtgcg catgtagcgg 300

tcgtcgggca gcgacgcgtc gccggtggtc gggtgcgcgg cctcccgctt gatcgggata 360

tgcgtgtgtt ccggcacgta gaaggtcgca aacccgtggt cgtcggcaag cttcgcggcc 420

gcagccggag agatgccacg gtcgctggtg aaaagcacaa gcccgtaatc catgcagtga 480

attagaacgt gttctacctc tgcggggcaa gctgtcgtga tacggaccgt ctcgccgcgc 540

ggtcgtctgc gaagcccgcg ggcaagccaa tggcgacggc accggccgtc gcacgtgcgc 600

tagcgtgggt gatcgaccgt gtcgctcgcg cagtgacgcg cctgcaagca ccgcgtcgca 660

tcgcaaccgt ggcgcccgct cggcactaaa aggcagtgga agcaaca 707

<210> 69
<211> 686
<212> DNA
<213> Mycobacterium marinum

<400> 69
tcgtaggcgg cttcctcctg cgtccacagt cgcccgcatc gcctcgaggt attcacgcaa 60

catcgtgcgg cgccgtccgg gtggaacgcc atggtcggcg agttcgtcgg tgttccaacc 120

gaaccccacg ccgaggctga cccgtccgcc ggacagatga tccagcgtgg caatgctctt 180

ggccagggtg atcgggtcat gctcgacggg cagcgccacc gcagtcgaca gccgtacccg 240

cgaggtcacc gccgatgccg cgcccaaact cacccagggg tccagcgtgc gcatataacg 300

atcgtcggga agcgaggaat cgcccgtcgt tggatgagcg gcttctcgct tgattgggat 360

atgggtgtgc tcaggcacgt agaaggtgtg aaagccgtgg tcgtcagcga gtctcgccgc 420

cgccgccgga gcgatgccgc ggtcgctggt gaaaagcaca agcccatagt ccataacaga 480

attagaacgt gttctacctc ggccgggcaa gcgccccccg cgccaatcgg ctcggcggga 540

tcgacggagg tgatggcgct ggtcgagcgg gggcaggtcg ccgcggcgcg agcaccggaa 600

cgtgcgctag cgtggttgtt cgaatcgcgt cgcagggacc aagcgtcgca atgcagcagc 660

ggcgccgcga cggcgcgcaa gtaaca 686

<210> 70
<211> 685
<212> DNA
<213> Mycobacterium ulcerans

<400> 70
tcgtaggcgg cttcctcctg cgtccacagc gcccgcatcg cctcgaggta ttcacgcaac 60

atcgtgcggc gccgtccggg tggaacgcca tggtcggcga gttcgtcggt gttccaaccg 120

aaccccacgc cgaggctgac ccgtccgccg gacagatgat ccagcgtggc aatgctcttg 180

gccagggtga tcgggtcatg ctcgacgggc agcgccaccg cagtcgacag ccgtacccgc 240

gaggtcaccg ccgatgccgc gcccaaactc acccaggggt ccagcgtgcg catataacga 300

tcgtcgggaa gcgaggaatc gcccgtcgtt ggatgagcgg cttctcgctt gattgggata 360

tgggtgtgct caggcacata gaaggtgtga aagccgtggt cgtcagcgag tctcgccgcc 420

gccgccggag cgatgccgcg gtcgctggtg aaaagcacaa gcccatagtc cataacagaa 480

ttagaacgtg ttctacctcg gccgggcaag cgccccccgc gccaatcggc ttggcgggat 540

cgacggaggt gatggcgctg gtcgagcggg ggcaggtcgc cgcggcgcga gcaccggaac 600

gtgcgctagc gtggttgttc gaatcgcgtc gcagggacca agcgtcgcaa tgcagcagcg 660

gcgccgcgac ggcgcgcaag taaca                                          685

<210>  71
<211>  729
<212>  DNA
<213>  Mycobacterium leprae

<400>  71
tcatataacg gcttcattct tgtgtccata atgcctgcat tgcttcgagg cattcgtaca      60

ccatggtgcg gcgccgcccg gatggcacat cgtgatcggt gagctcgttg gtcttccaac     120

cgaacccgac gccgaagttc actcactcgc cggacaaatt atccaggttg acaatacttt     180

tcgcaagtgt gattgggtca tgttagacgg gcagcgccac caccatgaac agtcgtagcc     240

tgccgatata acccgcatgt cgcgcccaaa cttacccatg agtcataggt acgcatcgca     300

tatagctgtc gtcactggac agtgatactc atccgtaacc aggtagtggg gtctgagtgg     360

caatggcata tgggtgtgtt cgggcacata gaacttgcgg aagccgtggc tctccgcaag     420

cttgactgct gccgcggggg tgatgccgcg gtcgttggtt aaaagcgcaa tcccgtagcc     480

cataccaaga atttagagcg tgttccacct gcgacggcca agcggtcgtg ccgacgattt     540

cggcgtccat cggtggtagg cgagctgaca cgcaggtcgt gccggcgcgg tcgccctaac     600

gtgcgctagc gttgatgatc gaatgcgccg caacgtaagc gctgccaatt tgggcgttta     660

tccaacggtg cgcatgggag cacagcgttg cactgcagca gtggcgccgt gacggcactg     720

gaaataaca                                                             729

<210>  72
<211>  129
<212>  DNA
<213>  Mycobacterium nonchromogenicum

<400>  72
gttcctgttc ggcgggcaac gggggggtcc ttgtcgcgca gtgttgaccc accgactcgg      60

cccgcaagtg cgctagcgtg gatggtcgaa gcgcgccgca ccgcccacca gcgccctgcc     120

acaagcaca                                                             129

<210>  73
<211>  219
<212>  DNA
<213>  Mycobacterium scrofulaceum

<400>  73
gttctacctc cggtgagcaa gctgccgccg cggcggcacg gatcggcgtc caagccggtc      60

gcgacggcac gcccgtcccg aagtgcgcta gcgtggttga tcgatcgcgt cgcaacgcaa     120

ccgccgggca cggcattcgt ggaacggcgc gcccgcacgc acagcgccgc gacgcaactg     180

tggcgcccgc aaaggcactt cacggcactg gaagcaaca                            219

<210> 74
<211> 116
<212> DNA
<213> Mycobacterium triplex

<400> 74
gttctacctt ggtcggcaag cggcgcggga acggccccgg caccggctcc ccgacgtgcg 60

ctagcgtggt tgttcgaatc gcgtcgcaac gcaagcgcgg cgagcctgga aaaaca 116


<210> 75
<211> 568
<212> DNA
<213> Mycobacterium paratuberculosis

<400> 75
gatctcagac agtggcaggt ggcggctccg aagctggcgt cagctattgg tgtaccgaat 60

gttgttgtca ccgagccggt cccaggtgtg ttcgagttgc agctgagaat tgtcgatccg 120

cttagttcgc cgcttgaatg gtcgtctgtg ccagccgccc actcgtggtc tctgagtttg 180

ggtatcgatg aaatgggcgt ctaccagtcg ctcccgttgg cgaacgtatc gggcgttgta 240

gtgggaggcg taccagggtc ggggaaaacc gcgtggctga cgagtgctct ggggtcgttc 300

ggtgcgtcag cggcggtcca gttcgctgtc atcgacggga agggtggtca ggacttggaa 360

tgcctgcgtg ctcgtagctg ccgattcatg aatgacgatc tggagctgcc tgagattgca 420

gcgattctga atgacgcgac cggtctagtc cgtgatcgaa ttagacaggg caacaacata 480

ttcggatcgt ccaacttttg ggatcgcggc ccgacgccgc aggttccgct ggtgttcgtg 540

gtgattgacg gctatcgggg ccgagatc 568


<210> 76
<211> 715
<212> DNA
<213> Pseudomonas aeruginosa

<400> 76
gcccgtcaca ccatgggagt gggttttacc agaagtggct agtctaaccg caaggaggac 60

ggtcaccacg gtaggattca tgactggggt gaagtcgtaa caaggtagcc gtatcggaag 120

gtgcggctgg atcacctcct ttccagagct tctcgcacaa gttgagcgct cacgcttatc 180

ggctgtaaat taaagacaga ctcaggggtc tgtagctcag tcggttagag caccgtcttg 240

ataaggcggg ggtcgttggt tcgaatccaa ccagacccac cattgtctgt cggtaacaca 300

cctgaggcaa atctgtacat gggggcatag ctcagctggg agagcacctg ctttgcaagc 360

aggggtcgtc ggttcgatcc cgtctgcctc caccaatcac caacgctaag ggcttggttc 420

agacactgaa ccgagaattt tgcattggcg attgagccag tcagaggata tcaacagata 480

tcggctgtcg ttctttaaca atctggaaga agtaagtaat ttggatagcg gaagcgtctt 540

gagatggacg tggaaactat ccgggttgtg attgtatcga tgtatctcaa gatgattcga 600

actctaagtt tgactcaatt ggaatacggc acaacgcgag aactcaacct gtaacgagac . 660

agactcgtta tagggtcaag cgaacaagtg catgtggtgg atgccttggc rrtca 715

<210> 77
<211> 653
<212> DNA
<213> Burkholderia cepacia

<400> 77
   gcccgtcaca ccatgggagt gggttgctcc agaagtagct agtctaaccg caagggggac 60
   ggttaccacg gagtgattca tgactggggt gaagtcgtaa caaggtagcc gtaggggaac 120
   ctgcggctgg atcacctcct taatcgaaga tctcagcttc ttcataagct cccacacgaa 180
   ttgcttgatt cactggttag acgattgggt ctgtagctca gttggttaga gcgcacccct 240
   gataaggtga ggtcggcagt tcgaatctgc ccagacccac caattgttgg tgtgctgcgt 300
   gatccgatac gggccatagc tcagctggga gagcgcctgc tttgcacgca ggaggtcagg 360
   agttcgatcc tccttggctc caccatctaa aacaatcgtc gaaagctcag aaatgaatgt 420
   tcgtgaatga acattgattt ctggtctttg caccagaact gttctttaaa aattcgggta 480
   tgtgatagaa gtaagactga atgatctctt tcactggtga tcattcaagt caaggtaaaa 540
   tttgcgagtt caagcgcgaa ttttcggcga atgtcgtctt cacagtataa ccagattgct 600
   tggggttata tggtcaagtg aagaagcgca tacggtggat gccttggcrr tca 653

<210> 78
<211> 600
<212> DNA
<213> Pseudomonas putida

<400> 78
gggttccccg aagtagctag tctaaccttc gggaggacgg ttaccacggt gtgattcatg 60

actggggtga agtcgtaaca aggtagccgt aggggaacct gcggctggat cacctcctta 120

atcgacgaca tcagcctgct gatgagctcc cacacgaatt gcttgattca ttgtcgaaga 180

cgatcaagac cctatatagg tctgtagctc agttggttag agcgcacccc tgataagggt 240

gaggtcggca gttcaaatct gcccagacct accaatatgc ggggccatag ctcagctggg 300

agagcgcctg ccttgcacgc aggaggtcag cggttcgatc ccgcttggct ccaccactcg 360

ctttacttga tcagaactta gaaatgaaca ttcgttgatg aatgttgatt tctgactttt 420

gtcagatcgt tctttaaaaa ttcggatatg tgatagaaat agactgaaca ccagtttcac 480

tgctggtgga tcaggctaag gtaaaatttg tgagttctgc tcgaaagagc aacgtgcgaa 540

ttttcggcga atgtcgtctt cacagtataa ccagattgct tggggttata tggtcaagtg 600

<210>  79
<211>  446
<212>  DNA
<213>  Pseudomonas putida

<400>  79
ggttcaccag aagtagctag tctaaccttc gggaggacgg ttaccacggt gtgattcatg      60

actggggtga agtcgtaaca aggtagccgt aggggaacct gcggctggat cacctcctta     120

atcgacgaca tcagcctgct gatgagctcc cacacgaatt gcttgattct ttgtaaaaga     180

cgatcaaggc cttgtgcagg cctcgcgttg ttcctgatca gaacttggaa atgagcattc     240

gcttcgaatg ttgatttctg gcttttgtca gatcgttctt taaaaattcg gatatgtgat     300

agaaatagac tgaacaccag tttcactgct ggtggatcag gctaaggtaa aatttgtgag     360

ttctgctcga aagagcaacg tgcgaatttt cggcgaatgt cgtcttcaca gtataaccag     420

attgcttggg gttatatggt caagtg                                          446


<210>  80
<211>  660
<212>  DNA
<213>  Pseudomonas aeruginosa

<400>  80
gcccgtcaca ccatgggagt gggttgctcc agaagtagct agtctaaccg caagggggac      60

ggttaccacg gagtgattca tgactggggt gaagtcgtaa caaggtagcc gtaggggaac     120

ctgcggctgg atcacctcct taatcgaaga tctcagcttc ttcataagct cccacacgaa     180

ttgcttgatt cactggttag acgattgggt ctgtagctca gttggttaga gcgcacccct     240

gataaggtga ggtcggcagt tcgaatctgc ccagacccac caattgttgg tgtgctgcgt     300

gatccgatac gggccatagc tcagctggga gagcgcctgc tttgcacgca ggaggtcagg     360

agttcgatcc tccttggctc caccatctaa aacaatcgtc gaaagctcag aaatgaatgt     420

tcgtgaatga acattgattt ctggtctttg caccagaact gttctttaaa aattcgggta     480

tgtgatagaa gtaagactga atgatctctt tcactggtga tcattcaagt caaggtaaaa     540

tttgcgagtt caagcgcgaa ttttcggcga atgtcgtctt cacagtataa ccagattgct     600

tggggttata tggtcaagtg aagaagcgca tacggtggat gccttggcrr tcasaggcga     660


<210>  81
<211>  722
<212>  DNA
<213>  Burkholderia cepacia

<400>  81
gcccgtcaca ccatgggagt gggttttacc agaagtggct agtctaaccg caaggaggac      60

ggtcaccacg gtaggattca tgactggggt gaagtcgtaa caaggtagcc gtatcggaag     120

gtgcggctgg atcacctcct ttccagagct tctcgcacaa gttgagcgct cacgcttatc 180

ggctgtaaat taaagacaga ctcaggggtc tgtagctcag tcggttagag caccgtcttg 240

ataaggcggg ggtcgttggt tcgaatccaa ccagacccac cattgtctgg cggtaacaca 300

cctgaggcaa atctgtacat gggggcatag ctcagctggg agagcacctg ctttgcaagc 360

aggggtcgtc ggttcgatcc cgtctgcctc caccaatcac caacgctaag ggcttggttc 420

agacactgaa ccgagaattt tgcattggcg attgagccag tcagaggata tcaacagata 480

tcggctgtcg ttctttaaca atctggaaga agtaagtaat ttggatagcg gaagcgtctt 540

gagatggacg tggaaactat ccgggttgtg attgtatcga tgtatctcaa gatgattcga 600

actctaagtt tgactcaatt ggaatacggc acaacgcgag aactcaacct gtaacgagac 660

agactcgtta tagggtcaag cgaacaagtg catgtggtgg atgccttggc rrtcasaggc 720

ga 722

<210> 82
<211> 725
<212> DNA
<213> Stenotrophomonas maltophilia

<400> 82
gcccgtcaca ccatgggagt ttgttgcacc agaagcaggt agcttaacct tcgggagggc 60

gcttgcacgg tgctgcgatg actggggtga agtcgtaaca aggtagccgt atcggaaggt 120

gcggctggat cacctccttt tgagcaaaga cagcatcgtc ctgtcgggcg tcttcacaaa 180

gtacctgcat tcagagaatc acaacggcca ggccgatgtg agagtccctt ttgggcctta 240

gctcagctgg gagagcacct gctttgcaag caggggtcgt cggttcgatc ccgacagctc 300

caccatgttc gagctgtata ccgaagtccc tttcgaagag cccgcacatc catgtgctac 360

tttttgaaaa agcctttcgg gtctgtagct caggtggtta gacgcaccct gataagggtg 420

aggtcggtag ttcgagtcta cccagaccca ccattctctg aatgacgcat acattcgatc 480

tttatacgca tcagcactgt ggctggtacg tgttctttta aaacttgtga cgtagcgagc 540

gtttgagatg ttctatcaga cgtgtcgtga ggctaaggcg agagacgcaa gtctctttat 600

tgattgagtc gttatattcg tatccgggct ttgtaccccc gggtcgtgtg taacccaagg 660

caacttgcgg ttatatggtc aagcgaataa gcgcacacgg tggatgcctt ggcrrtcasa 720

ggcga 725

<210> 83
<211> 18
<212> DNA
<213> Pseudomonas sp.

<400> 83
acgtcacacc atgggagt 18

<210>  84
<211>  23
<212>  DNA
<213>  Burkholderia cepacia

<400>  84
ccctgagtct gtctttaatt tac                                                23

<210>  85
<211>  20
<212>  DNA
<213>  Pseudomonas aeruginosa

<400>  85
ctttcgacga ttgttttagt                                                    20

<210>  86
<211>  21
<212>  DNA
<213>  Stenotrophomonas maltophilia

<400>  86
tcaataaaag agacttgcgt c                                                  21

<210>  87
<211>  18
<212>  DNA
<213>  Pseudomonas sp.

<400>  87
gattgccaag gcatccac                                                      18

<210>  88
<211>  18
<212>  DNA
<213>  Pseudomonas sp.

<400>  88
gaggaaggtg gggatgac                                                      18

<210>  89
<211>  18
<212>  DNA
<213>  Pseudomonas sp.

<400>  89
tgggaacgta ttcaccgt                                                      18

Table 6. Species specific and universal probes

Name | Sequence | | T°m
PsMulti
(PsSeq1) | 5' ACGTCACACCATGGGAGT 3' (SEQ ID NO 83) | Sense | 62.8°C
Bcep | 5' CCCTGAGTCTGTCTTTAATTTAC 3' (SEQ ID NO 84) | Antisense | 58.3°C
Paer | 5' CTTTCGACGATTGTTTTAGT 3' (SEQ ID NO 85) | Antisense | 56.8°C
Smal | 5' TCAATAAAAGAGACTTGCGTC 3' (SEQ ID NO 86) | Antisense | 57.9°C
PsSeq2 | 5' GATTGCCAAGGCATCCAC 3' (SEQ ID NO 87) | Antisense | 65.4°C

Table 7. Control probes

ProUni1 | 5' GAGGAAGGTGGGGATGAC 3' (SEQ ID NO 88) | Sense | 63.1°C
ProUni2 | 5' TGGGAACGTATTCACCGT 3' (SEQ ID NO 89) | Antisense | 62.5°C

## Claims

1. Method for detecting at least one Mycobacterium strain in a sample, comprising:
(i) providing at least one non-tuberculosis Mycobacterium species-specific upstream p34 gene region (us-p34) nucleotide probe,
(ii) reacting said us-p34 nucleotide probe with said sample under conditions that allow for the selective formation of nucleotide duplexes between said us-p34 nucleotide probe and a corresponding non-tuberculosis Mycobacterium nucleic acid target present in said sample, and,
(iii) detecting any nucleotide duplexes containing said us-p34 nucleotide probe.

2. Method according to claim 1 wherein said non-tuberculosis Mycobacterium species-specific us-p34 nucleotide probe specifically hybridizes with at least part of a nucleic acid represented in any of SEQ ID NOs 57, 59 to 64, 67 and 69 to 74, or the complement thereof, or the corresponding sequences wherein T has been replaced by U.

3. Method according to claim 1 wherein said Mycobacterium species-specific us-p34 nucleotide probe is selected from the group of nucleic acids having a sequence as represented in any of SEQ ID NOs 8 to 24, 26 to 47, 49 to 51, 53, 54, 57, 59 to 64, 67, 69 to 74, or the complement thereof, or the corresponding sequences wherein T has been replaced by U.

4. Method for the differential detection of Mycobacteria in a sample, comprising:
(i) providing at least two distinct non-tuberculosis Mycobacterium species-specific us-p34 nucleotide probes,
(ii) reacting said us-p34 nucleotide probes with said sample under conditions that allow for the selective formation of nucleotide duplexes between said us-p34 specific nucleotide probe and a non-tuberculosis Mycobacterium nucleic acid present in said sample,
(iii) detecting any nucleotide duplexes containing said us-p34 nucleotide probe, and,
(iv) inferring from the nucleotide duplex formed, the presence and the identification of a specific Mycobacterium strain.

5. Method according to claim 4 wherein said non-tuberculosis Mycobacterium species-specific us-p34 nucleotide probes are selected from the group of nucleic acids having a sequence as represented in any of SEQ ID NOs 8 to 24, 26 to 47, 49 to 51, 53 and 54 (Tables 3 (a) to (c)) and SEQ ID NOs 57, 59 to 64, 67, 69 to 74, or the the complement thereof, or the corresponding sequences wherein T has been replaced by U.

6. Method for detecting at least one Mycobacterium strain in a sample, comprising:
(i) providing a suitable primer pair containing at least one sense or antisense Mycobacterium species-specific us-p34 primer,
(v) reacting said us-p34 primer pair with said sample under conditions that allow for the selective amplification of an us-p34 sequence in a non-tuberculosis Mycobacterium nucleic acid present in said sample, and,
(vi) detecting the amplified product of step (ii), and,
(vii) inferring from the amplification product the presence and the identification of at least one specific Mycobacterium strain.

7. Method according to claim 6 wherein said sense or antisense non-tuberculosis Mycobacterium species-specific us-p34 primer is selected from the group of nucleic acids having a sequence as represented in any of SEQ ID NOs 8 to 54.

8. Method for the differential detection of mycobacteria in a sample, comprising:
(i) providing at least one suitable us-p34 primer pair comprising a sense and anti-sense Mycobacterium species-specific us-p34 primer,
(ii) reacting said us-p34 primer pair with said sample under conditions that allow for the selective amplification of us-p34 sequences of at least one Mycobacterium nucleic acid present in said sample,
(iii) detecting the amplified product of step (ii), and,
(iv) inferring from the amplified product formed, the presence and the identification of at least one (specific) Mycobacterium.

9. Method according to claim 8 wherein said us-p34 primer is selected from the group of nucleic acds having a sequence as represented in any of SEQ ID NOs 4 to 54.

10. Method for the differential detection of mycobacteria in a sample, comprising:
(i) providing a suitable us-p34 primer pair containing a first sense or anti-sense Mycobacterium us-p34 primer selected from the group of nucleic acids having a sequence as represented in any of SEQ ID NOs 1 to 3 and a second Mycobacterium us-p34 primer selected from the group of nucleic acids having a sequence as represented in any of SEQ ID NOs 8 to 54,
(ii) reacting said us-p34 primer pair with said sample under conditions that allow for the selective amplification of us-p34 sequences of at least one Mycobacterium nucleic acid present in said sample,
(iii) detecting the amplified product of step (ii), and,
(iv) inferring from the amplified product formed, the presence and the identification of at least one (specific) Mycobacterium.

11. Method for the detection of MAC complex Mycobacterium species in a sample, comprising:
(i) providing at least one us-p34 probe selected from the group of nucleic acids having a sequence as represented in any of SEQ ID NOs 8, 14, 15, 22, 27, 28, 29, 34, 35, 50, 51, 57, and 73,
(ii) reacting said us-p34 probe with said sample under conditions that allow for the selective formation of nucleotide duplexes between said us-p34 nucleotide probe and a MAC complex Mycobacterium nucleic acid target in said sample, and,
(iii) detecting any nucleotide duplexes containing said us-p34 nucleotide probe.

12. Method for the detection of MOTT Mycobacterium species in a sample, comprising:
(i) providing at least one us-p34 probe selected from the group of nucleic acids having a sequence as represented in any of SEQ ID NOs 9 to 13, 16 to 21, 23, 24, 26, 30 to 33, 36 to 47, 49, 53, 54, 59 to 64, 67, 69 to 72 and 74,
(ii) reacting said us-p34 primer probe said sample under conditions that allow for the selective formation of nucleotide duplexes between said us-p34 nucleotide probe and a MOTT Mycobacterium nucleic acid target in said sample, and,
(iii) detecting any nucleotide duplexes containing said us-p34 nucleotide probe.

13. Method for detecting new us-p34 sequences in a sample, comprising:
(i) providing at least one suitable primer pair comprising a sense and anti-sense us-p34 primer selected from the nucleic acids having a sequence as represented in any of SEQ ID NOs 4 to 7,
(ii) reacting said us-p34 primer pair with said sample under conditions that allow for the amplification of an us-p34 sequence in a Mycobacterium nucleic acid target in said sample, and,
(iii) determining the sequence of the amplification product obtained in (ii).

14. Method for detecting new us-p34 sequences in a sample, comprising:
(i) providing at least one suitable primer pair comprising a sense or anti-sense us-p34 primer selected from the sequences represented in SEQ ID NOs 1 to 3 and a sense or anti-sense us-p34 primer selected from the sequences represented in SEQ ID NOs 4 to 7,
(ii) reacting said us-p34 primer pair with said sample under conditions that allow for the amplification of an us-p34 sequence in a Mycobacterium nucleic acid target in said sample, and,
(iii) determining the sequence of the amplification product obtained in (ii).

15. Method for the differential detection of mycobacteria in a sample, comprising:
(i) providing at least one suitable primer pair containing a sense and anti-sense us-p34 primer selected from the group of nucleic acids having a sequence as represented in any of SEQ ID NOs 1 to 7,
(ii) reacting said us-p34 primer pair with said sample under conditions that allow for the amplification of an us-p34 sequence in a Mycobacterium nucleic acid target in said sample,
(iii) selectively hybridizing the amplification products obtained in (ii) with at least one Mycobacterium species-specific us-p34 nucleotide probe selected from the group of nucleic acids having a sequence as represented in any of SEQ ID NOs 8 to 74,
(iv) detecting any nucleotide duplexes containing said Mycobacterium species-specific us-p34 nucleotide probe, and,
(v) inferring from the nucleotide duplex formed, the presence of a specific non-tuberculosis Mycobacterium species.

16. A Mycobacterium species-specific us-p34 nucleotide probe or primer comprising at least 8 contiguous nucleotides from one of the nucleic acid sequences represented in SEQ ID NOs 57 to 74, or the complement thereof, or the corresponding sequences wherein T has been replaced by U.

17. The Mycobacterium species-specific us-p34 nucleotide probe or primer of claim 16 having a sequence selected from the group of sequences as represented in any of SEQ ID NOs 8 to 74.

18. A Mycobacterium us-p34 nucleotide primer having a sequence selected from the group of sequences as represented in any of SEQ ID NOs 4 to 7.

19. A nucleic acid comprising a sequence selected from the group of sequences represented in any of SEQ ID NOs 8 to 54, 57, 59 to 64, 67, and 69 to 74.

20. A composition comprising at least one nucleotide probe, primer or nucleic acid according to any of claims 16 to 18.

21. A diagnostic kit comprising a probe, primer or nucleic acid according to any of claims 16 to 18 or a composition according to claim 19.

22. A method for producing a bochip or a micro-array comprising attaching at least one Mycobacterium species-specific probe according to claim 16 or 17.

23. A diagnostic kit for detecting two or more nucleic acids chosen from the group of nucleic acids represented in any of SEQ ID NOs 8 to 54, 57, 59 to 64, 67, and 69 to 74.

24. A solid support for the detection of mycobacteria comprising fixed to said support at least two probes selected from the group of nucleic acids as represented in any of SEQ ID NOs 8 to 74.

25. A solid support according to claim 24 for use in a method of any of claims 1 to 5, 10, 11 or 15.

26. A micro-array for performing a method according to any of claims 1 to 5, 10, 11 or 15 comprising a solid support, said solid support having immobilized thereon a set of different capture probes, said set of capture probes being optionally sub-divided in sub-sets of capture probes, wherein optionally each of said capture probes or each of said sub-set of capture probes is immobilized within a predefined region on said solid support.

27. A micro-array according to claim 26 comprising at least one Mycobacterium species-specific capture probe of claim 16 or 17.

28. A micro-array which allows detection of one or more nucleic acids chosen from the group of nucleic acids represented in any of SEQ ID NOs 57 to 74.
